# EUROPÄISCHE PATENTANMELDUNG

(11) **EP 2 033 515 A1**
(43) Veröffentlichungstag der Anmeldung: **11.03.2009**
(21) Anmeldenummer: 07115754.9
(22) Anmeldetag: 05.09.2007
(51) Int. Cl.: A01N 37/18, A01N 47/28, C07C 317/28, C07D 213/00

(54) **Verfahren zur verbesserten Nutzung des Produktionspotentials transgener Pflanzen**

(71) Anmelder: Bayer CropScience AG, 40789 Monheim (DE)
(72) Erfinder: Fischer, Rüdiger, Dr., 50259 Pulheim (DE); Hungenberg, Heike, 40764 Langenfeld (DE); Andersch, Wolfram, Dr., 51469 Bergisch Gladbach (DE); Jeschke, Peter, Dr., 51467 Bergisch Gladbach (DE); Velten, Robert, Dr., 40764 Langenfeld (DE)

(57) **Zusammenfassung**

Die Erfindung betrifft ein Verfahren zur Verbesserung der Nutzung des Produktionspotentials einer transgenen Pflanze, in dem die Pflanze mit einer wirksamen Menge mindestens einer optisch aktiven Phthalsäurediamidverbindung behandelt wird.

## Beschreibung

Die Erfindung betrifft ein Verfahren zur Verbesserung der Nutzung des Produktionspotentials transgener Pflanzen.

Der Anteil transgener Pflanzen in der Landwirtschaft ist in den letzten Jahren deutlich gestiegen, wenn auch regionale Unterschiede derzeit noch erkennbar sind. So hat sich beispielsweise der Anteil an transgenem Mais in den USA seit 2001 von 26% auf 52% verdoppelt, während transgener Mais in Deutschland bisher kaum eine praktische Rolle spielt. In anderen europäischen Ländern, beispielsweise in Spanien, liegt der Anteil an transgenem Mais aber bereits bei etwa 12%.

Transgene Pflanzen werden vor allem eingesetzt, um das Produktionspotential der jeweiligen Pflanzensorte bei möglichst geringem Einsatz von Produktionsmitteln möglichst günstig zu nutzen. Die genetische Veränderung der Pflanzen zielt dazu vor allem darauf ab, in den Pflanzen eine Resistenz gegen bestimmte Schädlinge oder Schadorganismen oder aber auch Herbizide sowie gegen abiotischen Stress (beispielsweise Dürre, Hitze oder erhöhte Salzgehalte) zu erzeugen. Ebenso kann eine Pflanze genetisch modifiziert werden, um bestimmte Qualitäts- oder Produktmerkmale, wie z.B. den Gehalt an ausgewählten Vitaminen oder Ölen, zu erhöhen oder bestimmte Fasereigenschaften zu verbessern.

Eine Herbizidresistenz bzw. -toleranz kann beispielsweise durch den Einbau von Genen in die Nutzpflanze zur Expression von Enzymen zur Detoxifikation bestimmter Herbizide erreicht werden, die somit selbst in Gegenwart dieser Herbizide zur Bekämpfung von Unkräutern und Ungräsern möglichst ungehindert wachsen können. Als Beispiele seien Baumwoll-Sorten bzw. Mais-Sorten genannt, die den herbiziden Wirkstoff Glyphosate (Roundup®), (Roundup Ready®, Monsanto) oder die Herbizide Glufosinate oder Oxynil tolerieren.

In jüngerer Zeit wurden zudem Nutzpflanzen entwickelt, die zwei oder mehrere genetische Veränderungen enthalten ("stacked transgenic plants" oder mehrfach-transgene Kulturen). So hat beispielsweise die Firma Monsanto mehrfach-transgene Maissorten entwickelt, die gegen den Maiszünsler (*Ostrinia nubilalis*) und den Maiswurzelbohrer (*Diabrotica virgifera*) resistent sind. Ebenso sind Mais- oder Baumwollkulturen bekannt, die sowohl gegen den Maiswurzelbohrer bzw. den Baumwollkapselwurm resistent sind als auch das Herbizid Roundup® tolerieren.

Es hat sich nunmehr gezeigt, dass sich die Nutzung des Produktionspotentials transgener Nutzpflanzen nochmals verbessern läßt, wenn die Pflanzen mit einem oder mehreren Phthalsäurediamidverbindungen behandelt werden. Dabei schließt der Begriff "Behandlung" alle Maßnahmen ein, die zu einem Kontakt zwischen diesen Wirkstoffen und mindestens einem Pflanzenteil führen. Unter "Pflanzenteilen" sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Phthalsäurediamidverbindungen und ihre insektizide Wirkung sind aus dem Stand der Technik bekannt. So beschreibt beispielsweise die EP-A-1 006 107 verschiedene Verbindungen aus der Klasse der Phthalsäurediamide und ihre insektizide Wirkung. Die insektizide Wirkung verschiedener optisch aktiver Phthalsäurediamidverbindungen ist in EP-A-1 782 689 beschrieben.

Aus diesen Dokumenten sind dem Fachmann Verfahren zur Herstellung, zur Verwendung und zur Wirkung von Phthalsäurediamidverbindungen ohne weiteres geläufig.

Die erfindungsgemäß einsetzbaren optisch aktiven Phthalsäurediamidverbindungen weisen die allgemeine Formel (I) wie folgt auf:
Optisch aktive Phthalsäurediamidverbindung der Formel (I) 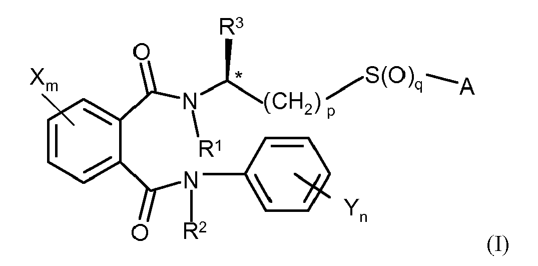 wobei
   - R¹ und R²: xunabhängig voneinander für Wasserstoff, (C₁-C₆)-Alkyl, substituiertes (C₁-C₆)-Alkyl, das mit mindestens einem Substituenten, ausgewählt unter Halogen, Cyano, (C₁-C₆)-Alkoxy, (C₁-C₆)-Alkylthio, Mono-(C₁-C₆)-alkylamino und Di-(C₁-C₆)-alkylamino in welcher die Alkylgruppen gleich oder verschieden sind, substituiert ist, oder (C₁-C₆)-Alkoxycarbonyl stehen;
   - R³: für (C₁-C₆)-Alkyl steht;
   - A: für Wasserstoff, (C₁-C₆)-Alkyl, Halo(C₁-C₆)-alkyl, (C₃-C₆)-Alkenyl, Halo(C₃-C₆)-alkenyl, (C₃-C₆)-Alkinyl, Halo(C₃-C₆)-alkinyl, (C₁-C₆)-Alkoxy(C₁-C₆)-alkyl, Halo(C₁-C₆)-alkoxy(C₁- C₆)-alkyl, (C₁-C₆)-Alkylthio(C₁-C₆)-alkyl, Halo(C₁-C₆)-alkylthio(C₁-C₆)-alkyl, (C₁-C₆)- Alkylsulfinyl(C₁-C₆)-alkyl, Halo(C₁-C₆)alkylsulfinyl(C₁-C₆)-alkyl, (C₁-C₆)-Alkylsulfonyl(C₁- C₆)-alkyl, Halo(C₁-C₆)alkylsulfonyl(C₁-C₆)-alkyl, Tri-(C₁-C₆)-alkylsilyl, (C₁-C₆)- Alkylcarbonyl, Mono-(C₁-C₆)-alkylcarbamid, Di-(C₁-C₆)-alkylcarbamid in welcher die Alkylgruppen gleich oder verschieden sind, Phenoxy(C₁-C₆)-alkyl oder substituiertes Phenoxy(C₁-C₆)-alkyl steht, das mit einem oder mehreren, gleichen oder verschiedenen Substituenten substituiert ist, welche ausgewählt sind unter Halogen, (C₁-C₆)-Alkyl, Halo(C₁-C₆)-alkyl, (C₁-C₆)-alkoxy, Halo(C₁-C₆)-alkoxy, (C₁-C₆)-Alkylthio, Halo(C₁-C₆)- alkylthio, (C₁-C₆)-Alkylsulfinyl, Halo(C₁-C₆)-alkylsulfinyl, (C₁-C₆)-Alkylsulfonyl, Halo(C₁- C₆)-alkylsulfonyl, Mono-(C₁-C₆)-alkylamino, Di-(C₁-C₆)-alkylamino, in welcher die Alkylgruppen gleich oder verschieden sind, Mono-(halo(C₁-C₆)-alkyl)amino und Di- (halo(C₁-C₆)-alkyl)amino, in welcher die Alkylgruppen gleich oder verschieden sind, für Phenyl(C₁-C₆)-alkyl oder substituiertes Phenyl(C₁-C₆)-alkyl steht, dessen Phenylring mit einem oder mehreren, gleichen oder verschiedenen Substituenten substituiert ist, welche ausgewählt sind unter Halogen, (C₁-C₆)-Alkyl, Halo(C₁-C₆)-alkyl, (C₁-C₆)-Alkoxy, Halo (C₁-C₆)-alkoxy, (C₁-C₆)-Alkylthio, Halo(C₁-C₆)-alkylthio, (C₁-C₆)-Alkylsulfinyl, Halo(C₁- C₆)-alkylsulfinyl, (C₁-C₆)-Alkylsulfonyl, Halo(C₁-C₆)-alkylsulfonyl, Mono-(C₁-C₆)- alkylamino, Di-(C₁-C₆)-alkylamino in welcher die Alkylgruppen gleich oder verschieden sind, Mono-(halo(C₁-C₆)-alkyl)amino und Di-(halo(C₁-C₆)-alkyl)amino in welcher die Alkylgruppen gleich oder verschieden sind;
   - p: für 0, 1, 2, 3 oder 4 steht;
   - q: für 0, 1 oder 2 steht;
   - X: unabhängig voneinander für Halogen, Cyano, Amino, Nitro, (C₁-C₆)-Alkyl, Halo(C₁-C₆)- alkyl, (C₂-C₆)-Alkenyl, Halo(C₂-C₆)-alkenyl, (C₂-C₆)-Alkinyl, Halo(C₂-C₆)-alkinyl, (C₁-C₆)- Alkoxy, Halo(C₁-C₆)-alkoxy, (C₁-C₆)-Alkylthio, Halo(C₁-C₆)-alkylthio, (C₁-C₆)- Alkylsulfinyl, Halo(C₁-C₆)-alkylsulfinyl, (C₁-C₆)-Alkylsulfonyl, (C₁-C₆)-Alkylsulfonyloxy, Halo(C₁-C₆)-alkylsulfonyl, Mono-(C₁-C₆)-alkylamino oder Di-(C₁-C₆)-alkylamino in welcher die Alkylgruppen gleich oder verschieden sind, steht;
   - m: für 0, 1, 2, 3 oder 4 steht;
   - Y: unabhängig voneinander für Wasserstoff, Halogen, Cyano, Amino, Nitro, (C₁-C₆)-Alkyl, Halo(C₁-C₆)-alkyl, (C₂-C₆)-Alkenyl, Halo(C₂-C₆)-alkenyl, (C₂-C₆)-Alkinyl, Halo(C₂-C₆)- alkinyl, (C₃-C₆)-Cycloalkyl, Halo(C₃-C₆)-cycloalkyl, (C₁-C₆)-Alkoxy, Halo(C₁-C₆)-alkoxy, (C₁-C₆)-Alkylthio, Halo(C₁-C₆)-alkylthio, (C₁-C₆)-Alkylsulfinyl, Halo(C₁-C₆)-alkylsulfinyl, (C₁-C₆)-Alkylsulfonyl, Halo(C₁-C₆)-alkylsulfonyl, Mono-(C₁-C₆)-alkylamino, Di-(C₁-C₆)- alkylamino oder Tri-(C₁-C₆)-alkylsilyl in welchen die Alkylgruppen gleich oder verschieden sind, Phenyl oder substituiertes Phenyl steht, das mit einem oder mehreren, gleichen oder verschiedenen Substituenten, ausgewählt unter Halogen, (C₁-C₆)-Alkyl, Halo(C₁-C₆)-alkyl, (C₁-C₆)-Alkoxy group, Halo(C₁-C₆)-alkoxy, (C₁-C₆)-Alkylthio, Halo(C₁-C₆)-alkylthio, (C₁- C₆)-Alkylsulfinyl, Halo(C₁-C₆)-alkylsulfinyl, (C₁-C₆)-Alkylsulfonyl, Halo(C₁-C₆)- alkylsulfonyl, Mono-(C₁-C₆)-alkylamino, Di-(C₁-C₆)-alkylamino in welcher die Alkylgruppen gleich oder verschieden sind, Mono-(halo(C₁-C₆)-alkyl)amino, und Di(halo (C₁-C₆)-alkyl)amino in welcher die Alkylgruppen gleich oder verschieden sind, substitutiert ist, für heterocyclische Verbindungen oder substituierte heterocyclische Verbindungen steht, die mit einem oder mehreren, gleichen oder verschiedenen Substituenten, welche ausgewählt sind unter Halogen, (C₁-C₆)-Alkyl, Halo(C₁-C₆)-alkyl, (C₁-C₆)-Alkoxy, Halo(C₁-C₆)-alkoxy, (C₁-C₆)-Alkylthio, Halo(C₁-C₆)-alkylthio, (C₁-C₆)-Alkylsulfinyl, Halo(C₁-C₆)-alkylsulfinyl, (C₁-C₆)-Alkylsulfonyl, Halo(C₁-C₆)-alkylsulfonyl, Mono-(C₁-C₆)-alkylamino, Di-(C₁-C₆)- alkylamino in welcher die Alkylgruppen gleich oder verschieden sind, Mono-(halo(C₁-C₆)- alkyl)amino, und Di-(halo(C₁-C₆)-alkyl)amino in welcher die Alkylgruppen gleich oder verschieden sind, substituiert sind; und
   - n: für 1, 2, 3, 4 oder 5 steht.

In einer bevorzugten Ausführungsform der Erfindung wird mindestens eine insektizid wirksame optisch aktive Phthalsäurediamidverbindung zur Behandlung transgener Nutzpflanzen verwendet. Der Begriff "insektizid wirksam" oder "insektizid" umfaßt im Sinne der Erfindung insektizide, akarizide, molluskizide, nematizide, ovizide Wirkungen, sowie eine abschreckende (repellent), verhaltensändernde (behavior modifier) oder sterilisierende (sterilant) Wirkung auf Schädlinge.

Bevorzugte insektizide Wirkstoffe sind Verbindungen der Formel (I), in welcher
- R¹ und R²: unabhängig voneinander für Wasserstoff oder (C₁-C₆)-Alkyl stehen;
- R³: für Methyl oder Ethyl steht;
- A: für (C₁-C₆)-Alkyl, (C₃-C₆)-Alkenyl, (C₃-C₆)-Alkinyl, (C₁-C₆)-Alkoxy(C₁-C₆)-alkyl, (C₁-C₆)- Alkylthio(C₁-C₆)-alkyl, (C₁-C₆)-Alkylsulfinyl(C₁-C₆)-alkyl, (C₁-C₆)-Alkylsulfonyl(C₁-C₆)- alkyl oder Mono-(C₁-C₆)-alkylcarbamid steht;
- p: für 0, 1, 2, 3 oder 4 steht;
- q: für 0, 1 oder 2 steht;
- X: unabhängig voneinander für Halogen, Nitro, Halo(C₁-C₆)-alkyl, Halo(C₁-C₆)-alkoxy, (C₁- C₆)-Alkylthio, (C₁-C₆)-Alkylsulfinyl, (C₁-C₆)-Alkylsulfonyl, (C₁-C₆)-Alkylsulfonyloxy, steht;
- m: für 0, 1 oder 2 steht;
- Y: unabhängig voneinander für Halogen, Cyano, (C₁-C₆)-Alkyl, Halo(C₁-C₆)-alkyl, (C₂-C₆)- Alkenyl, Halo(C₂-C₆)-alkenyl, (C₂-C₆)-Alkinyl, Halo(C₂-C₆)-alkinyl, (C₁-C₆)-Alkoxy, Halo(C₁-C₆)-alkoxy, (C₁-C₆)-Alkylthio, Halo(C₁-C₆)-alkylthio, (C₁-C₆)-Alkylsulfinyl, Halo(C₁-C₆)-alkylsulfinyl, (C₁-C₆)-Alkylsulfonyl, Halo(C₁-C₆)-alkylsulfonyl, Mono- (halo(C₁-C₆)-alkyl)amino oder Di-(halo(C₁-C₆)-alkyl)amino in welcher die Alkylgruppen gleich oder verschieden sind steht;
und
- n: für 0, 1, 2, 3 oder 4 steht.

Besonders bevorzugt sind Verbindungen der Formel (I), in welcher
- R¹ und R²: unabhängig voneinander für Wasserstoff oder (C₁-C₆)-Alkyl stehen;
- R³: für Methyl steht;
- A: unabhängig voneinander für (C₁-C₆)-Alkyl, (C₃-C₆)-Alkenyl, (C₃-C₆)-Alkinyl, (C₁-C₆)- Alkoxy(C₁-C₆)-alkyl, (C₁-C₆)-Alkylthio(C₁-C₆)-alkyl, (C₁-C₆)-Alkylsulfinyl(C₁-C₆)-alkyl oder (C₁-C₆)-Alkylsulfonyl(C₁-C₆)-alkyl steht;
- p: für 1 oder 2 steht;
- q: für 0, 1 oder 2 steht;
- X: unabhängig voneinander für Halogen, Nitro, Halo(C₁-C₆)-alkyl, Halo(C₁-C₆)-alkoxy, (C₁- C₆)-Alkylthio, (C₁-C₆)-Alkylsulfinyl, (C₁-C₆)-Alkylsulfonyl oder (C₁-C₆)-Alkylsulfonyloxy steht;
- m: für 1 oder 2 steht;
und
- Y: unabhängig voneinander für Halogen, Cyano, (C₁-C₆)-Alkyl, Halo(C₁-C₆)-alkyl, (C₂-C₆)- Alkenyl, Halo(C₂-C₆)-alkenyl, (C₂-C₆)-Alkinyl, Halo(C₂-C₆)-alkinyl, (C₁-C₆)-Alkoxy, Halo(C₁-C₆)-alkoxy, (C₁-C₆)-Alkylthio, Halo(C₁-C₆)-alkylthio, (C₁-C₆)-Alkylsulfinyl, Halo(C₁-C₆)-alkylsulfinyl, (C₁-C₆)-Alkylsulfonyl oder Halo(C₁-C₆)-alkylsulfonyl steht; und
- n: für 1, 2 oder 3 steht.

Insbesondere bevorzugt sind die folgenden Verbindungen: *(S)*-3-Iodo-N¹-{2-methyl-4-[1,2,2,2-tetrafluoro-1-(trifluoromethyl)ethyl]phenyl}-N²-(1-methyl-2-methylsulfonylethyl)phthalamid, und/oder *(S)*-3-Chloro-N'-{2-methyl-4-[1,2,2,2-tetrafluoro-1-(trifluoromethyl)ethyl]phenyl}-N²-(1-methyl-2-methylsulfonylethyl)phthalamid, und/oder *(S)*-3-Bromo-N¹-{2-methyl-4-[1,2,2,2-tetrafluoro-1-(trifluoromethyl)ethyl]phenyl}-N²-(1-methyl-2-methylsulfonylethyl)phthalamid, und/oder *(S)*-3-Iodo-N¹-{2-methyl-4-[1,2,2,2-tetrafluoro-1-(trifluoromethyl)ethyl]phenyl}-N²-(1-methyl-2-methylsulfinylethyl)phthalamid oder deren Salze.

Die Verbindungen der Formel (I) können als Isomerengemische in unterschiedlicher Zusammensetzung vorliegen.

Insbesondere bevorzugt sind Verbindungen der oben genannten Formel (I), in welcher die Reste die folgende Bedeutung haben:

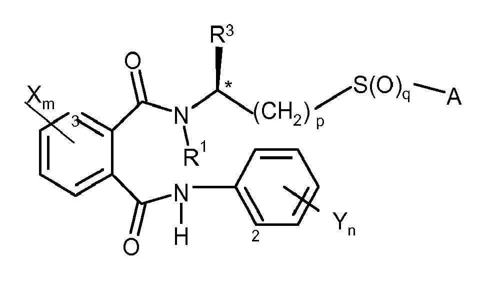

**Tabelle A**

| Nr. | Yₙ | Xₘ | R¹ | R³ | A | p | q | log p | Schmelz punkt |
|---|---|---|---|---|---|---|---|---|---|
| 1 | 2-Methyl 4-[1,2,2,2-tetrafluoro-1-(trifluoromethyl)ethyl] | 3-Iodo | H | Methyl | Methyl | 1 | 2 | 3,18^{[a]} 3,2^{[c]} | 149-152°C |
| 2 | 2-Methyl 4-[2,2,2-trifluoro-1-(trifluoromethyl)ethyl] | 3-Iodo | H | Methyl | Methyl | 1 | 0 | | |
| 3 | 2-Methyl 4-[2,2,2-trifluoro-1-(trifluoromethyl)ethyl] | 3-Iodo | H | Methyl | Methyl | 1 | 2 | | |
| 4 | 2-Methyl 4-[2,2,2-trifluoro-1-(trifluoromethyl)ethyl] | 3-Iodo | H | Methyl | Methyl | 1 | 1 | | |
| 5 | 2-Methyl 4-[1,2,2,2-tetrafluoro-1-(trifluoromethyl)ethyl] | 3-Chloro | H | Methyl | Methyl | 1 | 2 | | 168-170°C |
| 6 | 2-Methyl 4-[1,2,2,2-tetrafluoro-1-(trifluoromethyl)ethyl] | 3-Iodo | H | Methyl | Ethyl | 1 | 2 | | |
| 7 | 2-Methyl 4-[1,2,2,2-tetrafluoro-1-(trifluoromethyl)ethyl] | 3-Bromo | H | Methyl | Methyl | 1 | 2 | | |
| 8 | 2-Methyl 3-Fluoro 4-[1,2,2,2-tetrafluoro-1-(trifluoromethyl)ethyl] | 3-Iodo | H | Methyl | Methyl | 1 | 1 | 2,78^{[a]} | |
| 9 | 2-Methyl 3-Fluoro 4-[1,2,2,2-tetrafluoro-1-(trifluoromethyl)ethyl] | 3-Iodo | H | Methyl | Ethyl | 1 | 1 | 2,97^{[a]} | 210°C |
| 10 | 2-Methyl 3-Fluoro 4-Pentafluoroethyl | 3-Chloro | H | Methyl | Ethyl | 1 | 0 | 4,21^{[a]} | 116-120°C |
| 11 | 2-Methyl 3-Fluoro 4-Pentafluoroethyl | 3-Chloro | H | Methyl | Methyl | 1 | 0 | 3,94^{[b]} | 190-193°C |
| 12 | 2-Methyl 3-Chloro 4-Pentafluoroethyl | 3-Chloro | H | Methyl | Ethyl | 1 | 2 | 3,14^{[a]} | 91-92°C |
| 13 | 2-Methyl 3-Fluoro 4-Pentafluoroethyl | 3-Chloro | H | Methyl | Methyl | 1 | 2 | 2,82^{[a]} | |
| 14 | 2-Methyl 3-Fluoro 4-Pentafluoroethyl | 3-Chloro | H | Methyl | Ethyl | 1 | 2 | 3,01^{[a]} | |
| 15 | 2-Methyl 3-Fluoro 4-Pentafluoroethyl | 3-Bromo | H | Methyl | Methyl | 1 | 0 | 3,93^{[a]} | 150-155°C |
| 16 | 2-Methyl 3-Fluoro 4-Pentafluoroethyl | 3-Bromo | H | Methyl | Methyl | 1 | 2 | 2,86^{[a]} | 88-94°C |
| 17 | 2-Methyl 3-Fluoro 4-[1,2,2,2-tetrafluoro-1-(trifluoromethyl)ethyl] | 3-Bromo | H | Methyl | Methyl | 1 | 0 | 4,31^{[a]} | |
| 19 | 2-Methyl 3-Fluoro 4-[1,2,2,2-tetrafluoro-1-(trifluoromethyl)ethyl] | 3-Iodo | H | Methyl | Ethyl | 1 | 0 | 4,71^{[a]} | |
| 20 | 2-Methyl 3-Chloro 4-Pentafluoroethyl | 3-Iodo | H | Methyl | Ethyl | 1 | 2 | 3,28^{[a]} | |
| 21 | 2-Methyl 3-Fluoro 4-[1,2,2,2-tetrafluoro-1-(trifluoromethyl)ethyl] | 3-Iodo | H | Methyl | Ethyl | 1 | 2 | 3,47^{[a]} | |
| 22 | 2-Methyl 3-Chloro 4-Pentafluoroethyl | 3-Bromo | H | Methyl | Ethyl | 1 | 2 | 3,19^{[a]} | |
| 23 | 2-Methyl 3-Fluoro 4-Pentafluoroethyl | 3-Iodo | H | Methyl | Methyl | 1 | 0 | 4,04^{[a]} | |
| 24 | 2-Methyl 3-Fluoro 4-Pentafluoroethyl | 3-Iodo | H | Methyl | Methyl | 1 | 2 | 2,97^{[a]} | |
| 25 | 2-Methyl 3-Fluoro 4-[1,2,2,2-tetrafluoro-1-(trifluoromethyl)ethyl] | 3-Iodo | H | Methyl | Methyl | 1 | 0 | 4,41^{[a]} | |
| 26 | 2-Methyl 3-Chloro 4-Pentafluoroethyl | 3-Iodo | H | Methyl | Methyl | 1 | 0 | 4,2^{[a]} | |
| 27 | 2-Methyl 3-Fluoro 4-[1,2,2,2-tetrafluoro-1-(trifluoromethyl)ethyl] | 3-Iodo | H | Methyl | Methyl | 1 | 2 | 3,27^{[a]} | |
| 28 | 2-Methyl 3-Chloro 4-Pentafluoroethyl | 3-Iodo | H | Methyl | Methyl | 1 | 2 | 3,1^{[a]} | |
| 29 | 2-Bromo 4-[1-(Difluoromethyl)-1,2,2,2-tetrafluoroethyl] | 3-Bromo | H | Methyl | Methyl | 1 | 0 | 3,81^{[a]} | 138-142°C |
| 30 | 2-Bromo 4-[1-(Difluoromethyl)-1,2,2,2-tetrafluoroethyl] | 3-Bromo | H | Methyl | Ethyl | 1 | 2 | 3,06^{[a]} | 75-79°C |
| 31 | 2-Methyl 3-Fluoro 4-Pentafluoroethyl | 3-Iodo | H | Methyl | Methyl | 1 | 1 | 2,5^{[a]} | 71-74°C |
| 32 | 2-Methyl 4-[2-Bromo-1,2,2-trifluoro-1-(trifluoromethyl)ethyl] | 3-Iodo | H | Methyl | Ethyl | 1 | 0 | 4,68^{[a]} 471^{[c]} | 114°C |
| 33 | 2-Methyl 4-[1-(Difluoromethyl)-1,2,2,2-tetrafluoroethyl] | 3-Iodo | H | Methyl | Ethyl | 1 | 0 | 3,96^{[a]} 3,96^{[c]} | 105°C |
| 34 | 2-Chloro 4-[1-(Difluoromethyl)-1,2,2,2-tetrafluoroethyl] | 3-Iodo | H | Methyl | Ethyl | 1 | 0 | 4,2^{[a]} 4,2^{[c]} | 187°C |
| 35 | 2-Methyl 4-[1-(Difluoromethyl)-2,2,2-trifluoroethyl] | 3-Iodo | H | Methyl | Ethyl | 1 | 0 | 3,63^{[a]} 3,62^{[c]} | 105°C |
| 36 | 2-Methyl 4-[2-Bromo-1,2,2-trifluoro-1-(trifluoromethyl)ethyl] | 3-Iodo | H | Methyl | Ethyl | 1 | 2 | 3,49^{[a]} 3,47^{[c]} | 133°C |
| 37 | 2-Bromo 4-[1-(Difluoromethyl)-2,2,2-trifluoroethyl] | 3-Iodo | H | Methyl | Methyl | 1 | 0 | 3,51^{[a]} 3,54^{[c]} | 167°C |
| 38 | 2-Chloro 4-[1-(Difluoromethyl)-2,2,2-trifluoroethyl] | 3-Iodo | H | Methyl | Ethyl | 1 | 0 | 3,76^{[a]} 3,79^{[c]} | 165°C |
| 39 | 2-Bromo 4-[1-(Difluoromethyl)-2,2,2-trifluoroethyl] | 3-Iodo | H | Methyl | Ethyl | 1 | 0 | 3,78^{[a]} 3,8^{[c]} | 160°C |
| 40 | 2-Fluoro 4-[1-(Difluoromethyl)-2,2,2-trifluoroethyl] | 3-Iodo | H | Methyl | Ethyl | 1 | 0 | 3,57^{[a]} 3,57^{[c]} | 144°C |
| 41 | 2-Chloro 4-[1-(Difluoromethyl)-1,2,2,2-tetrafluoroethyl] | 3-Iodo | H | Methyl | Ethyl | 1 | 2 | 3,12^{[a]} 3,18^{[c]} | 132-135°C |
| 42 | 2-Chloro 4-[1-(Difluoromethyl)-2,2,2-trifluoroethyl] | 3-Iodo | H | Methyl | Methyl | 1 | 2 | 2,69^{[a]} 2,72^{[c]} | 116-120°C |
| 43 | 2-Bromo 4-[1-(Difluoromethyl)-2,2,2-trifluoroethyl] | 3-Iodo | H | Methyl | Methyl | 1 | 2 | 2,71^{[a]} 2,76^{[c]} | 198°C |
| 44 | 2-Fluoro 4-[1-(Difluoromethyl)-1,2,2,2-tetrafluoroethyl] | 3-Iodo | H | Methyl | Methyl | 1 | 2 | 2,72^{[a]} 2,76^{[c]} | 124-126°C |
| 45 | 2-Fluoro 4-[1-(Difluoromethyl)-2,2,2-trifluoroethyl] | 3-Iodo | H | Methyl | Methyl | 1 | 2 | 2,5^{[a]} 2,54^{[c]} | 144-147°C |
| 46 | 2-Bromo 4-[1-(Difluoromethyl)-2,2,2-trifluoroethyl] | 3-Iodo | H | Methyl | Ethyl | 1 | 2 | 2,87^{[a]} 2,92^{[c]} | 122°C |
| 47 | 2-Fluoro 4-[1-(Difluoromethyl)-1,2,2,2-tetrafluoroethyl] | 3-Iodo | H | Methyl | Ethyl | 1 | 2 | 2,89^{[a]} 2,94^{[c]} | 142-144°C |
| 48 | 2-Fluoro 4-[1-(Difluoromethyl)-2,2,2-trifluoroethyl] | 3-Iodo | H | Methyl | Ethyl | 1 | 2 | 2,65^{[a]} 2,69^{[c]} | 128°C |
| 49 | 2-Methyl 4-[1-(Difluoromethyl)-1,2,2,2-tetrafluoroethyl] | 3-Chloro | H | Methyl | Methyl | 1 | 2 | 2,61^{[a]} 2,64^{[c]} | 101-103°C |
| 50 | 2-Methyl 4-[1,2,2,2-tetrafluoro-1-(trifluoromethyl)ethyl] | 3-Chloro | H | Methyl | Methyl | 1 | 0 | | |
| 51 | 2-Methyl 3-Fluoro 4-[2,2,2-trifluoro-1-(trifluoromethyl)ethyl] | 3-Chloro | H | Methyl | Methyl | 1 | 1 | 2,5^{[a]} 2,41^{[c]} | 116-118°C |
| 52 | 2-Methyl 4-[2,2,2-trifluoro-1-(trifluoromethyl)ethyl] 5-Fluoro | 3-Chloro | H | Methyl | Methyl | 1 | 1 | 2,58^{[a]} 2,51^{[c]} | 130-135°C |
| 53 | 2-Methyl 4-[2,2,2-trifluoro-1-(trifluoromethyl)ethyl] 5-Fluoro | 3-Bromo | H | Methyl | Methyl | 1 | 1 | 2,58^{[a]} 2,51^{[c]} | 187°C |

Die Messung der logP Werte erfolgte gemäß EEC Directive 79/831 Annex V.A8 durch HPLC (High Performance Liquid Chromatography) an reverser Phase Säulen (C 18), mit nachfolgenden Methoden:
^{[a]} Die Bestimmung erfolgt bei pH 2.3 mit 0,1% wässriger Phosphorsäure und Acetonitril als Eluenten (linearer Gradient von 10 bis 95% Acetonitril).
^{[b]} Die Bestimmung erfolgt mit LC-MS bei pH 2,7 mit 0,1 % wässriger Ameisensäure und Acetonitril als Eluenten (linearer Gradient von 10% bis 95% Acetonitril).
^{[c]} Die Bestimmung erfolgt mit LC-MS bei pH 7,8 mit 0,001 M wässriger Ammoniumhydrogencarbonat-Lösung und Acetonitril als Eluenten (linearer Gradient von 10 % bis 95 % Acetonitril).

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen). Die lambda-maX-Werte wurden an Hand der UV-Spektren von 200 bis 400 nm in den Absorptionsmaxima ermittelt.

Erfindungsgemäß steht "Alkyl" für unverzweigte oder verzweigte aliphatische Kohlenwasserstoffe mit 1 bis 6, vorzugsweise 1 bis 4 Kohlenstoffatomen. Geeignete Alkylgruppen sind beispielsweise Methyl, Ethyl, *n*-Propyl, *i*-Propyl, *n*-, *iso-, sec- or* tert-Butyl, Pentyl oder Hexyl. Die Alkylgruppe kann unsubstituiert vorliegen oder ist mit mindestens einem der hier genannten Substituenten substituiert.

Erfindungsgemäß steht "Alkenyl" für unverzweigte oder verzweigte Kohlenwasserstoffe mit mindestens einer Doppelbindung. Die Doppelbindung der Alkenylgruppe kann unkonjugiert sein oder steht in Konjugation mit einer ungesättigten Bindung oder Gruppe. Alkenylgruppen mit 2 bis 6 bzw. 3 bis 6 Kohlenstoffatomen sind bevorzugt. Geeignete Alkenylgruppen sind beispielsweise Vinyl oder Allyl. Die Alkenylgruppe kann unsubstituiert vorliegen oder ist mit mindestens einem der hier genannten Substituenten substituiert.

Erfindungsgemäß steht "Alkinyl" für unverzweigte oder verzweigte Kohlenwasserstoffe mit mindestens einer Dreifachbindung. Die Dreifachbindung der Alkinylgruppe kann unkonjugiert sein oder steht in Konjugation mit einer ungesättigten Bindung oder Gruppe. Alkinylgruppen mit 2 bis 6 bzw. 3 bis 6 Kohlenstoffatomen sind bevorzugt. Geeignete Alkinylgruppen sind beispielsweise Ethinyl, Propinyl, Butinyl, Pentinyl, Hexinyl, Methylpropinyl, 4-Methyl-1-butinyl, 4-Propyl-2-pentinyl, and 4-Butyl-2-hexinyl. Die Alkinylgruppe kann unsubstituiert vorliegen oder ist mit mindestens einem der hier genannten Substituenten substituiert.

Erfindungsgemäß steht "Cycloalkyl" für cyclische Kohlenwasserstoffe mit 3 bis 6 Kohlenwasserstoffen. Geeignete Cycloalkylgruppen sind beispielsweise Cyclopropyl, Cyclobutyl, Cyclopentyl oder Cyclohexyl. Die Cycloalkylgruppe kann unsubstituiert vorliegen oder ist mit mindestens einem der hier genannten Substituenten substituiert.

Erfindungsgemäß steht "Alkoxy" für Alkoxygruppen mit 1 bis 6 Kohlenstoffatomen, vorzugsweise mit 1 bis 4 Kohlenstoffatomen. Geeignete Alkoxygruppen sind beispielsweise Methyloxy, Ethyloxy, *n*-Propyloxy, *i*-Propyloxy, *n-, iso-, sec-* oder tert-Butyloxy, Pentyloxy, oder Hexyloxy. Die Alkoxygruppe kann unsubstituiert vorliegen oder ist mit mindestens einem der hier genannten Substituenten substituiert.

Erfindungsgemäß steht "Alkylamino" für Alkylaminogruppem mit 1 bis 6 Kohlenstoffatomen, vorzugsweise mit 1 bis 4 Kohlenstoffatomen. Geeignete Alkylaminogruppen sind beispielsweise Methylamino, Ethylamino, *n*-Propylamino, *i*-Propylamino, *n-, iso-, sec- or* tert-Butylamino, Pentylamino oder Hexylamino. Die Alkylaminogruppe kann unsubstituiert vorliegen oder ist mit mindestens einem der hier genannten Substituenten substituiert.

Erfindungsgemäß steht "heterocyclische Verbindungen" für cyclische Kohlenwasserstoffe mit vorzugsweise 3 bis 14, besonders bevorzugt 3 bis 10 und ganz besonders bevorzugt 5 bis 6 Kohlenstoffatomen die mindestens ein Heteroatom, wie beispielsweise Stickstoff, Sauerstoff oder Schwefel enthalten und die nach gängigen Methoden herstellbar sind. Die heterocyclische Verbindungen können gesättigte und ungesättigte Bindungen oder Gruppen enthalten, die zusätzlich mit weiteren ungesättigten Bindungen oder Gruppen in Konjugation stehen. Geeignete heterocyclische Verbindungen sind beispielsweise Oxiran, Aziridin, Azetidin, Tetrahydrofuran, Dioxan, Tetrahydrofuran-2-on, Caprolactam; ungesättigte heterocyclische Verbindungen wie beispielsweise 2H-Pyrrol, 4H-Pyran, 1,4-Dihydropyridin; und Heteroaryle wie beispielsweise Pyrrol, Pyrrazol, Imidazol, Oxazol, Isoxazol, Thiazol, Oxathiazol, Triazol, Tetrazol, Pyridin, Pyridazin, Pyrimidin, Pyrazin, Purin, Pteridin, Chinolin, Isochinolin, Acridin und Phenazin. Die heterocyclischen Verbindungen können unsubstituiert vorliegen oder sind mit mindestens einem der hier genannten Substituenten substituiert.

Erfindungsgemäß steht "Halogen" für Fluor, Chlor, Brom oder Iod, bevorzugt für Fluor, Chlor oder Brom.

Erfindungsgemäß steht "Haloalkyl" für Alkylgruppen mit 1 bis 6, vorzugsweise 1 bis 4 Kohlenstoffatomen in denen mindestens ein Wasserstoffatom gegen ein Halogen ausgetauscht ist. Geeignete Haloalkylgruppen sind beispielsweise CH₂F, CHF₂, CF₃, CF₂Cl, CFCl₂, CF₂Br, CF₂CF₃, CFHCF₃, CH₂CF₃, CFClCF₃, CCl₂CF₃, CF₂CH₃, CF₂CH₂F, CF₂CHF₂, CF₂CF₂Cl, CF₂CF₂Br, CFHCH₃, CFHCH₂F, CFHCHF₂, CHFCF₃, CHFCF₂Cl, CHFCF₂Br, CH₂CF₃, CFClCF₃, CCl₂CF₃, CF₂CF₂CF₃, CH₂CF₂CF₃, CF₂CH₂CF₃, CF₂CF₂CH₃, CHFCF₂CF₃, CF₂CHFCF₃, CF₂CF₂CHF₂, CF₂CF₂CH₂F, CF₂CF₂CF₂Cl, CF₂CF₂CF₂Br, 1,2,2,2-Tetrafluoro-1-(trifluoromethyl)ethyl, 2,2,2-Trifluoro-1-(trifluoromethyl)ethyl, Pentafluoroethyl, 1-(Difluoromethyl)-1,2,2,2-tetrafluoroethyl, 2-Bromo-1,2,2-trifluoro-1-(trifluoromethyl)ethyl, 1-(Difluoromethyl)-2,2,2-trifluoroethyl. Die Haloalkylgruppe kann unsubstituiert vorliegen oder ist mit mindestens einem der hier genannten Substituenten substituiert.

Erfindungsgemäß steht "Trialkylsilyl" für Trialkylsilylgruppen mit jeweils 1 bis 6, vorzugsweise 1 bis 3 Kohlenstoffatomen pro Alkylgruppe. Eine geeignete Trialkylsilylgruppe ist beispielsweise Trimethylsilyl. Die Trialkylsilylgruppen können unsubstituiert vorliegen oder sind mit mindestens einem der hier genannten Substituenten substituiert.

Erfindungsgemäß steht "Aryl" für Arylgruppen mit 6 bis 10, bevorzugt 6 Kohlenstoffatomen. Geeignete Arylgruppen sind beispielsweise Phenyl or Naphtyl. Die Arylgruppe kann unsubstituiert vorliegen oder ist mit mindestens einem der hier genannten Substituenten substituiert.

Bevorzugt sind Mischungen aus zwei oder mehr, vorzugsweise zwei oder drei, besonders bevorzugt zwei, der insektizid wirksamen Verbindungen.

Gemäß dem erfindungsgemäßen Verfahren werden transgene Pflanzen, insbesondere Nutzpflanzen mit optisch aktiven Phthalsäurediamidverbindungen behandelt, um die landwirtschaftliche Produktivität zu steigern. Bei transgenen Pflanzen im Sinne der Erfindung handelt es sich um Pflanzen, die mindestens ein Gen oder Genfragment codieren, das sie nicht durch Befruchtung übernommen haben. Dieses Gen oder Genfragment kann von einer anderen Pflanze derselben Spezies, von Pflanzen unterschiedlicher Spezies, aber auch von Organismen aus dem Reich der Tiere oder Mikroorganismen (einschließlich Viren) stammen oder abgeleitet sein ("Fremdgen") und/oder ggf. bereits gegenüber der natürlich vorkommenden Sequenz Mutationen aufweisen. Auch die Verwendung synthetischer Gene ist erfindungsgemäß möglich und wird hier ebenso unter den Terminus "Fremdgen" subsumiert. Es ist auch möglich, dass eine transgene Pflanze zwei oder mehr Fremdgene unterschiedlicher Herkunft codiert.

Das "Fremdgen" im Sinne der Erfindung ist des weiteren dadurch gekennzeichnet, dass es eine Nukleinsäuresequenz umfasst, die in der transgenen Pflanze eine bestimmte biologische oder chemische Funktion bzw. Aktivität ausübt. In der Regel codieren diese Gene Biokatalysatoren, wie z.B. Enzyme oder Ribozyme, oder aber umfassen regulatorische Sequenzen, wie beispielsweise Promotoren oder Terminatoren zur Beeinflussung der Expression endogener Proteine. Dazu können sie aber auch regulatorische Proteine codieren wie beispielsweise Repressoren oder Induktoren. Das Fremdgen kann des weiteren ebenso der gezielten Lokalisation eines Genprodukts der transgenen Pflanze dienen und dazu beispielsweise eine Signalsequenz codieren. Auch Inhibitoren, wie beispielsweise antisense-RNA können durch das Fremdgen codiert werden.

Vielfältige Verfahren zur Herstellung transgener Pflanzen sowie Verfahren zur gezielten Mutagenese, zur Gentransformation und Klonierung sind dem Fachmann ohne weiteres bekannt, so beispielsweise aus: Willmitzer, 1993, Transgenic plants, In: Biotechnology, A Multivolume Comprehensive Treatise, Rehm et al. (eds.), Vol.2, 627-659, VCH Weinheim, Germany;

Ein Beispiel für eine komplexe genetische Manipulation einer Nutzpflanze ist die so genannte GURT-Technologie ("Genetic Use Restriction Technologies"), die der technischen Kontrolle der Vermehrung der jeweiligen transgenen Pflanzensorte dient. Dazu werden in der Regel zwei oder drei Fremdgene in die Nutzpflanze einkloniert, die in einem komplexen Wechselspiel nach der Gabe eines externen Stimulus eine Kaskade auslösen, die zum Absterben des sich anderenfalls entwickelnden Embryos führt. Der externe Stimulus (beispielsweise ein Wirkstoff oder ein anderer chemischer oder abiotischer Reiz) kann dazu beispielsweise mit einem Repressor interagieren, der damit nicht mehr die Expression einer Rekombinase unterdrückt, so dass die Rekombinase einen Inhibitor spalten kann und damit die Expression eines Toxins erlaubt, das das Absterben des Embryos bewirkt. Beispiele für diese Art der transgenen Pflanzen sind in US 5,723,765 oder US 5,808,034 offenbart.

Dem Fachmann sind demnach Verfahren zur Generierung transgener Pflanzen bekannt, die aufgrund der Integration regulatorischer Fremdgene und der damit ggf. vermittelten Überexpression, Suppression oder Inhibierung endogener Gene oder Gensequenzen oder der Existenz oder Expression von Fremdgenen oder deren Fragmente veränderte Eigenschaften aufweisen.

Wie bereits oben ausgeführt, ermöglicht das erfindungsgemäße Verfahren eine Verbesserung der Ausnutzung des Produktionspotentials transgener Pflanzen. Dies kann einerseits ggf. darauf zurückzuführen sein, dass die Aufwandmenge des erfindungsgemäß einsetzbaren Wirkstoffs reduziert werden kann; beispielsweise durch eine Verminderung der eingesetzten Dosis oder aber durch eine Reduktion der Anzahl der Applikationen. Andererseits kann ggf. der Ertrag der Nutzpflanzen quantitativ und/oder qualitativ gesteigert werden. Dies gilt vor allem bei einer transgen erzeugten Resistenz gegen biotischen oder abiotischen Streß. Werden beispielsweise insektizide optisch aktive Phthalsäurediamidverbindungen eingesetzt, kann die Dosierung des Insektizids u.U. auf eine subletale Dosis begrenzt werden, ohne damit die gewünschte Wirkung des Wirkstoffs auf die Schädlinge signifikant abzuschwächen.

Diese synergistischen Wirkungen können je nach Pflanzenarten bzw. Pflanzensorten, deren Standort und Wachstumsbedingungen (Böden, Klima, Vegetationsperiode, Ernährung) variieren und können vielfältig sein. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Diese Vorteile sind auf eine erfindungsgemäß erreichte synergistische Wirkung zwischen dem einsetzbaren optisch aktiven Phthalsäurediamidverbindungen und dem jeweiligen Wirkprinzip der genetischen Veränderung der transgenen Pflanze zurückzuführen. Mit dieser durch den Synergismus bedingten Reduzierung der Produktionsmittel bei gleichzeitiger Ertrags- oder Qualitätserhöhung sind erhebliche ökonomische und ökologische Vorteile verbunden.

Eine Liste mit dem Fachmann bekannten Beispielen transgener Pflanzen unter Nennung der jeweils betroffenen Struktur in der Pflanze bzw. dem durch die genetische Veränderung exprimierten Protein in der Pflanze ist in **Tabelle 1** zusammengestellt. Darin ist der betroffenen Struktur bzw. dem exprimierten Prinzip jeweils eine bestimmte Merkmalsausprägung im Sinne einer Toleranz gegenüber einem bestimmten Streßfaktor zugeordnet. Eine ähnliche Liste **(Tabelle 3)** stellt - in etwas geänderter Anordnung - ebenso Beispiele von Wirkprinzipien, damit induzierten Toleranzen und möglichen Nutzpflanzen zusammen. Weitere Beispiele von transgenen Pflanzen, die für die erfindungsgemäße Behandlung geeignet sind, sind in der **Tabelle 4** zusammengestellt.

In einer vorteilhaften Ausführungsform werden die optisch aktiven Phthalsäurediamidverbindungen zur Behandlung transgener Pflanzen enthaltend mindestens ein Gen oder Genfragment zur Codierung eines Bt-Toxins eingesetzt. Bei einem Bt-Toxin handelt es sich um ein ursprünglich aus dem Bodenbakterium *Bacillus thuringiensis* stammendes oder abgeleitetes Protein, das entweder der Gruppe der *crystal toxins (Cry)* oder der *cytolytic toxins (Cyt)* zugehörig ist. Sie werden in dem Bakterium ursprünglich als Protoxine gebildet und werden erst im alkalischen Milieu - beispielsweise im Verdauungstrakt bestimmter Fraßinsekten - zu ihrer aktiven Form metabolisiert. Dort bindet das dann aktive Toxin an bestimmte Kohlenwasserstoffstrukturen an Zelloberflächen und verursacht eine Porenbildung, die das osmotische Potential der Zelle zerstören und damit Zelllysis bewirken kann. Der Tod der Insekten ist die Folge. Bt-Toxine sind vor allem wirksam gegen bestimmte Schädlingsarten aus den Ordnungen der Lepidoptera (Schmetterlinge), Homoptera (Gleichflügler), Diptera (Zweiflügler) und Coleoptera (Käfer) in all ihren Entwicklungsstadien; also von der Eilarve über ihre juvenilen bis hin zu ihren adulten Formen.

Es ist seit langem bekannt, Gensequenzen codierend Bt-Toxine, Teile davon oder aber von Bt-Toxin abgeleitete Proteine oder Peptide mit Hilfe gentechnologischer Verfahren in landwirtschaftliche Nutzpflanzen einzuklonieren und somit transgene Pflanzen mit endogenen Resistenzen gegen Bt-Toxin empfindlichen Schädlinge zu erzeugen. Die mindestens ein Bt-Toxin oder daraus abgeleitete Proteine codierenden transgenen Pflanzen werden im Sinne der Erfindung als "Bt-Pflanzen" definiert.

Die "erste Generation" solcher Bt-Pflanzen enthielt in der Regel nur die Gene, die die Bildung eines bestimmten Toxins ermöglichten und somit nur gegen eine Gruppe von Schaderregern resistent machten. Beispiele für eine kommerziell erhältliche Mais-Sorte mit dem Gen zur Bildung des CrylAb Toxins ist "YieldGard®" von Monsanto, die gegen den Maiszünsler resistent ist. Die Resistenz gegen andere Schaderreger aus der Familie der Lepidopteren wird dagegen in der Bt-Baumwoll-Sorte (Bollgard®) durch Einklonieren der Gene zur Bildung des CrylAc Toxins erzeugt. Wiederum andere transgene Kulturpflanzen exprimieren Gene zur Bildung von Bt-Toxinen mit Wirkung gegen Schaderreger aus der Ordnung der Coleopteren. Als Beispiele dafür sei die Bt-Kartoffel-Sorte "NewLeaf®" (Monsanto) genannt, die das Cry3A Toxin bilden kann und somit gegen den Kartoffelkäfer ("Colorado Potatoe Beetle") resistent ist, bzw. die transgene Mais-Sorte "YieldGard®" (Monsanto), die das Cry 3Bb1 Toxin bilden und somit gegen verschiedene Arten des Maiswurzelbohrers geschützt ist.

In einer "zweiten Generation" wurden die oben bereits beschriebenen mehrfach-transgenen Pflanzen generiert, die mindestens zwei Fremdgene enthalten oder exprimieren.

Erfindungsgemäß bevorzugt sind transgene Pflanzen mit Bt-Toxinen aus der Gruppe der *Cry-*Familie (siehe z.B. Crickmore et al., 1998, Microbiol. Mol. Biol. Rev. 62 : 807-812), die insbesondere wirksam sind gegen Lepidopteren, Coleopteren oder Dipteren. Beispiele für Gene codierend die Proteine sind:
cry1Aa1, cry1Aa2, cry1Aa3, cry1Aa4, cry1Aa5, cry1Aa6, cry1Aa7, cry1Aa8, cry1Aa9, cry1Aa10, cry1Aa11 cry1Ab1, cry1Ab2, cry1Ab3, cry1Ab4, cry1Ab5, cry1Ab6, cry1Ab7, cry1Ab8, cry1Ab9, cry1Ab10, cry1Ab11, cry1Ab12, cry1Ab13, cry1Ab14, cry1Ac1, cry1Ac2, cry1Ac3, cry1Ac4, cry1Ac5, cry1Ac6, cry1Ac7, cry1Ac8, cry1Ac9, cry1Ac10, cry1Ac11, cry1Ac12, cry1Ac13, cry1Ad1, cry1Ad2, cry1Ae1, cry1Af1, cry1Ag1, cry1Ba1, cry1Ba2, cry1Bb1, cry1Bc1, cry1Bd1, cry1Be1, cry1Ca1, cry1Ca2, cry1Ca3, cry1Ca4, cry1Ca5, cry1Ca6, cry1Ca7, cry1Cb1, cry1Cb2, cry1Da1, cry1Da2, cry1Db1, cry1Ea1, cry1Ea2, cry1Ea3, cry1Ea4, cry1Ea5, cry1Ea6, cry1Eb1, cry1Fa1, cry1Fa2, cry1Fb1, cry1Fb2, cry1Fb3, cry1Fb4, cry1Ga1, cry1Ga2, cry1Gb1, cry1Gb2, cry1Ha1, cry1Hb1, cry1Ia1, cry1Ia2, cry1Ia3, cry1Ia4, cry1Ia5, cry1Ia6, cry1Ib1, cry1Ic1, cry1Id1, cry1Ie1, cry1I-like, cry1Ja1, cry1Jb1, cry1Jc1, cry1Ka1, cry1-like, cry2Aa1, cry2Aa2, cry2Aa3, cry2Aa4, cry2Aa5, cry2Aa6, cry2Aa7, cry2Aa8, cry2Aa9, cry2Ab1, cry2Ab2, cry2Ab3, cry2Ac1, cry2Ac2, cry2Ad1, cry3Aa1, cry3Aa2, cry3Aa3, cry3Aa4, cry3Aa5, cry3Aa6, cry3Aa7, cry3Ba1, cry3Ba2, cry3Bb1, cry3Bb2, cry3Bb3, cry3Ca1, cry4Aa1, cry4Aa2, cry4Ba1, cry4Ba2, cry4Ba3, cry4Ba4, cry5Aa1, cry5Ab1, cry5Ac1, cry5Ba1, cry6Aa1, cry6Ba1, cry7Aa1, cry7Ab1, cry7Ab2, cry8Aa1, cry8Ba1, cry8Ca1, cry9Aa1, cry9Aa2, cry9Ba1, cry9Ca1, cry9Da1, cry9Da2, cry9Ea1, cry9 like, cry10Aa1, cry10Aa2, cry11Aa1, cry11Aa2, cry11Ba1, cry11Bb1, cry12Aa1, cry13Aa1, cry14Aa1, cry15Aa1, cry16Aa1, cry17Aa1, cry18Aa1, cry18Ba1, cry18Ca1, cry19Aa1, cry19Ba1, cry20Aa1, cry21Aa1, cry21Aa2, cry22Aa1, cry23Aa1, cry24Aa1, cry25Aa1, cry26Aa1, cry27Aa1, cry28Aa1, cry28Aa2, cry29Aa1, cry30Aa1, cry31Aa1, cyt1Aa1, cyt1Aa2, cyt1Aa3, cyt1Aa4, cyt1Ab1, cyt1Ba1, cyt2Aa1, cyt2Ba1, cyt2Ba2, cyt2Ba3, cyt2Ba4, cyt2Ba5, cyt2Ba6, cyt2Ba7, cyt2Ba8, cyt2Bb1.

Besonders bevorzugt sind die Gene oder Genabschnitte der Subfamilien cry1, cry2, cry3, cry5 und cry9, insbesondere bevorzugt sind cry1Ab, cry1Ac, cry3A, cry3B und cry9C.

Weiterhin bevorzugt ist es, Pflanzen einzusetzen, die neben den Genen für ein oder mehrere Bt-Toxine ggf. auch Gene zur Expression beispielsweise eines Protease- bzw. Peptidase-Inhibitors (wie in WO-A 95/35031), von Herbizidresistenzen (z.B. gegen Glufosinate oder Glyphosate durch Expression des pat- oder bar-Gens) oder zur Ausbildung von Nematoden-, Pilz- oder Virusresistenzen (z.B. durch Expression einer Glucanase, Chitinase) enthalten oder exprimieren. Sie können aber auch in ihren metabolischen Eigenschaften genetisch modifiziert sein, so daß eine qualitative und/oder quantitative Änderung von Inhaltsstoffen (z.B. durch Modifikationen des Energie-, Kohlenhydrat-, Fettsäure- oder Stickstoffwechsels oder diese beeinflussende Metabolitflüsse) zeigen (siehe oben).

Eine Liste mit Beispielen von Wirkprinzipien, die durch eine genetische Modifikation in eine Nutzpflanze eingebracht werden können, und die sich für die erfindungsgemäße Behandlung allein oder in Kombination eignen, ist in **Tabelle 2** zusammengestellt. Diese Tabelle enthält unter der Angabe "AP" (aktives Prinzip) das jeweils betroffene Wirkprinzip und dem zugeordnet den damit zu kontrollierenden Schädling.

In einer besonders bevorzugten Variante wird das erfindungsgemäße Verfahren zur Behandlung transgener Gemüse-, Mais-, Soja-, Baumwoll-, Tabak-, Reis-, Kartoffel und Zuckerrübensorten eingesetzt. Dabei handelt es sich vorzugsweise um Bt-Pflanzen.

Bei den Gemüsepflanzen bzw. -sorten handelt es sich beispielsweise um folgende Nutzpflanzen:
○ Kartoffeln: vorzugsweise Stärkekartoffeln, Süßkartoffeln und Speisekartoffeln;
○ Wurzelgemüse: vorzugsweise Möhren (Karotten), Kohlrüben (Speiserüben, Stoppelrüben, Mairüben, Herbstrüben, Teltower Rübchen), Schwarzwurzeln, Topinambur, Wurzelpetersilie, Pastinake, Rettich und Meerrettich;
○ Knollengemüse: vorzugsweise Kohlrabi, Rote Bete (Rote Rübe), Sellerie (Knollensellerie), Radies und Radieschen;
○ Zwiebelgemüse: vorzugsweise Porree, Lauch und Zwiebeln (Steck- und Samenzwiebel);
○ Kohlgemüse: vorzugsweise Kopfkohl (Weißkohl, Rotkohl, Blattkohl, Wirsingkohl), Blumenkohl, Sprossenkohl, Brokkoli, Grünkohl, Markstammkohl, Meerkohl und Rosenkohl;
○ Fruchtgemüse: vorzugsweise Tomaten (Freiland- ,Strauch-, Fleisch-, Gewächshaus-, Cocktail-, Industrie- und Frischmarkt-Tomaten), Melonen, Eierfrucht, Auberginen, Paprika (Gemüse- und Gewürzpaprika, Spanischer Pfeffer), Peperoni, Kürbis, Zucchini und Gurken (Freiland-, Gewächshaus-, Schlangen- und Einlegegurken);
○ Gemüsehülsenfrüchte: vorzugsweise Buschbohnen (als Schwertbohnen, Perlbohnen, Flageoletbohnen, Wachsbohnen, Trockenkochbohnen mit grün- und gelbhülsigen Sorten), Stangenbohnen (als Schwertbohnen, Perlbohnen, Flageoletbohnen, Wachsbohnen mit grün-, blau- und gelbhülsigen Sorten), Dicke Bohnen (Ackerbohnen, Puffbohnen, Sorten mit weiß und schwarz gefleckten Blüten), Erbsen (Platterbsen, Kichererbsen, Markerbsen, Schalerbsen, Zuckererbsen, Palerbsen, Sorten mit hell- und dunkelgrünem Frischkorn) und Linsen;
○ Blatt- und Stielgemüse: vorzugsweise Chinakohl, Kopfsalat, Pflücksalat, Feldsalat, Eisbergsalat, Romanasalat, Eichblattsalat, Endivien, Radicchio, Lollo rosso-Salat, Ruccola-Salat, Chicoree, Spinat, Mangold (Blatt- und Stielmangold) und Petersilie;
○ Sonstige Gemüse: vorzugsweise Spargel, Rhabarber, Schnittlauch, Artischocken, Minzen, Sonnenblumen, Knollenfenchel, Dill, Gartenkresse, Senf, Mohn, Erdnuß, Sesam und Salatzichorien.

Bt-Gemüse einschließlich beispielhafter Methoden zu ihrer Herstellung sind ausführlich beschrieben beispielsweise in Barton et al., 1987, Plant Physiol. 85 : 1103-1109 ; Vaeck et al., 1987, Nature 328 : 33-37 ; Fischhoff et al., 1987, Bio/Technology 5 : 807-813. Bt-Gemüsepflanzen sind darüber hinaus bereits bekannt als kommerziell erhältliche Varianten, beispielsweise die Kartoffelsorte NewLeaf^{®} (Monsanto). Ebenso beschreibt die US 6,072,105 die Herstellung von Bt-Gemüse. Ebenso ist auch bereits Bt-Baumwolle dem Grunde nach bekannt, beispielsweise aus der US-A-5,322,938 Im Rahmen der vorliegenden Erfindung ist die Bt-Baumwolle mit den Handelsnamen NuCOTN33® und NuCOTN33B® besonders bevorzugt.

Auch die Verwendung und Herstellung von Bt-Mais ist bereits lange bekannt, beispielsweise aus Ishida, Y., Saito, H., Ohta, S., Hiei, Y., Komari, T., and Kumashiro, T. (1996). High efficiency transformation of maize (Zea mayz L.) mediated by Agrobacterium tumefaciens, Nature Biotechnology 4: 745-750. Auch die EP-B-0485506 beschreibt die Herstellung von Bt-Maispflanzen. Bt-Mais ist weiterhin in verschiedenen Variationen kommerziell erhältlich, beispielsweise unter folgenden Handelsnamen (Firma/Firmen jeweils in Klammern): KnockOut® (Novartis Seeds), NaturGard® (Mycogen Seeds), Yieldgard® (Novartis Seeds, Monsanto, Cargill, Golden Harvest, Pioneer, DeKalb u. a.), Bt-Xtra® (DeKalb) und StarLink® (Aventis CropScience, Garst u. a.). Im Sinne der vorliegenden Erfindung sind vor allem die folgenden Maissorten besonders bevorzugt: KnockOut®, NaturGard®, Yieldgard®, Bt-Xtra® und StarLink®.

Auch für Sojabohne sind Roundup®Ready Sorten oder Sorten mit einer Resistenz gegen das Herbizid Liberty Link® erhältlich und erfindungsgemäß behandelbar. Bei Reis sind eine Vielzahl von Linien des so genannten "Golden Rice" erhältlich, die sich gleichermaßen dadurch auszeichnen, dass sie durch eine transgene Modifikation einen erhöhten Gehalt an Provitamin A aufweisen. Auch hierbei handelt es sch um Beispiele für Pflanzen, die nach dem erfindungsgemäßen Verfahren mit den geschilderten Vorteilen behandelt werden können.

Das erfindungsgemäße Verfahren eignet sich zur Bekämpfung einer Vielzahl von Schadorganismen, die insbesondere in Gemüse, Mais bzw. Baumwolle auftreten, insbesondere Insekten und Spinnentieren, ganz besonders bevorzugt Insekten. Zu den erwähnten Schädlingen gehören:
○ Aus der Ordnung der Anoplura (Phthiraptera), z.B. Damalinia spp., Haematopinus spp., Linognathus spp., Pediculus spp., Trichodectes spp..
○ Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.
○ Aus der Klasse der Bivalva z.B. Dreissena spp..
○ Aus der Ordnung der Chilopoda z.B. Geophilus spp., Scutigera spp..
○ Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp..
○ Aus der Ordnung der Collembola z.B. Onychiurus armatus.
○ Aus der Ordnung der Dermaptera z.B. Forficula auricularia.
○ Aus der Ordnung der Diplopoda z.B. Blaniulus guttulatus.
○ Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp..
○ Aus der Klasse der Gastropoda z.B. Arion spp., Biomphalaria spp., Bulinus spp., Deroceras spp., Galba spp., Lymnaea spp., Oncomelania spp., Succinea spp..
○ Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.
○ Weiterhin lassen sich Protozoen, wie Eimeria, bekämpfen.
○ Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp..
○ Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii..
○ Aus der Ordnung der Hymenoptera z.B. Diprion spp., Hoplocampa spp., Lasius spp., Monomorium pharaonis, Vespa spp..
○ Aus der Ordnung der Isopoda z.B. Armadillidium vulgare, Oniscus asellus, Porcellio scaber.
○ Aus der Ordnung der Isoptera z.B. Reticulitermes spp., Odontotermes spp..
○ Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mame-stra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp..
○ Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.
○ Aus der Ordnung der Siphonaptera z.B. Ceratophyllus spp., Xenopsylla cheopis.
○ Aus der Ordnung der Symphyla z.B. Scutigerella immaculata.
○ Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp..
○ Aus der Ordnung der Thysanura z.B. Lepisma saccharina.
○ Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp..

Insbesondere ist das erfindungsgemäße Verfahren zur Behandlung von Bt-Gemüse, Bt-Mais, Bt-Baumwolle, Bt-Soja, Bt-Tabak sowie auch Bt-Reis, Bt-Zuckerrüben oder Bt-Kartoffeln geeignet zur Kontrolle von beißenden Insekten oder Blattläusen (Aphidina), Weißen Fliegen (Trialeurodes), Thrips (Thysanoptera), Spinnmilben (Arachnida), Schild- oder Schmierläusen (Coccoidae bzw. Pseudococcoidae).

Die erfindungsgemäß einsetzbaren Wirkstoffe können in üblichen Formulierungen eingesetzt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Spritzpulver sind in Wasser gleichmäßig dispergierbare Präparate, die neben dem Wirkstoff außer einem Verdünnungs- oder Inertstoff noch Netzmittel, z.B. polyoxethylierte Alkylphenole, polyoxethylierte Fettalkohole, Alkyl- oder Alkylphenol-sulfonate und Dispergiermittel, z.B. ligninsulfonsaures Natrium, 2,2'-dinaphthylmethan-6,6'-disulfonsaures Natrium enthalten.

Stäubemittel erhält man durch Vermahlen des Wirkstoffes mit fein verteilten festen Stoffen, z.B. Talkum, natürlichen Tonen wie Kaolin, Bentonit, Pyrophillit oder Diatomeenerde. Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen beispielsweise in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP-POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Diese einzelnen Formulierungstypen sind im Prinzip bekannt und beispielsweise beschrieben in: "Pesticides Formulations", 2nd Ed., Marcel Dekker N.Y.; Martens, 1979, "Spray Drying Handbook", 3rd Ed., G. Goodwin Ltd. London.

Der Fachmann kann aufgrund seiner allgemeinen Fachkenntnisse geeignete Formulierungshilfen auswählen (siehe dazu beispielsweise Watkins, "Handbook of Insecticide Dust Diluents and Carriers", 2nd Ed., Darland Books, Caldwell N.J.).

In einer bevorzugten Ausführungsform werden die Pflanzen oder Pflanzenteile mit einem Suspensionskonzentrat auf Ölbasis erfindungsgemäß behandelt. Ein vorteilhaftes Suspensionskonzentrat ist aus der WO 2005/084435 (EP 1 725 104 A2) bekannt. Es besteht aus mindestens einem bei Raumtemperatur festen agrochemischen Wirkstoff, mindestens einem "geschlossenen" Penetrationsförderer, mindestens einem Pflanzenöl oder Mineralöl, mindestens einem nicht-ionischen Tensid und/oder mindestens einem anionischen Tensid und gegebenenfalls einem oder mehreren Zusatzstoffen aus den Gruppen der Emulgiermittel, der schaumhemmenden Mittel, der Konservierungsmittel, der Antioxydantien, der Farbstoffe und/oder der inerten Füllmaterialien. Bevorzugte Ausführungsformen des Suspensionskonzentrats sind in der genannte WO 2005/084435 beschrieben. Beide Dokumente werden zu Zwecken der Offenbarung vollumfänglich in Bezug genommen.

In einer weiteren bevorzugten Ausführungsform werden die Pflanzen oder Pfanzenteile mit Mitteln enthaltend Ammonium- oder Phosphoniumsalzen und gegebenenfalls Penetrationsförderen erfindungsgemäß behandelt. Ein vorteilhaftes Mittel ist aus W02007/068350 bekannt. Es besteht aus mindestens einem Wirkstoff aus der Klasse der optisch aktiven Phthalsäurediamide und mindestens einem Ammonium- oder Phosphoniumsalz, gegebenenfalls Penetrationsförderern. Bevorzugte Ausführungsformen sind in W02007/068350 beschrieben. Dieses Dokument wird zu Zwecken der Offenbarung vollumfänglich in Bezug genommen.

Die Formulierungen enthalten im Allgemeinen zwischen 0,01 und 98 Gew.-% Wirkstoff, vorzugsweise zwischen 0,5 und 90 %. In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Mit den äußeren Bedingungen wie Temperatur, Feuchtigkeit u.a. kann auch die erforderliche Aufwandmenge variieren. Sie kann innerhalb weiter Grenzen schwanken, z.B. zwischen 0,1 g/ha und 5,0 kg/ha oder mehr Aktivsubstanz. Vorzugsweise liegt sie jedoch zwischen 0,1 g/ha und 1,0 kg/ha. Aufgrund der synergistischen Effekte zwischen Bt-Gemüse und Insektizid sind Aufwandmengen von 0,1 bis 500 g/ha, besonders bevorzugt.

Für Verbindungen der Formel (I) sind Aufwandmengen von 10 bis 500 g/ha bevorzugt, besonders bevorzugt sind 10 bis 200 g/ha.

In einer besonderen Ausführungsform des erfindungsgemäßen Verfahrens wird die Verbindung der Formel (I) in einer Aufwandmenge von 0,1 g/ha bis 5,0 kg/ha, bevorzugt von 0,1 bis 500 g/ha und besonders bevorzugt von 50 bis 500 g/ha und insbesondere bevorzugt von 50 bis 200 g/ha verwendet.

Die erfindungsgemäßen Wirkstoffe können in ihren handelsüblichen Formulierungen sowie in den aus diesen Formulierungen bereiteten Anwendungsformen in Mischungen mit anderen Wirkstoffen, wie Insektiziden, Lockstoffen, Sterilantien, Akariziden, Nematiziden, Fungiziden, wachstumsregulierenden Stoffen oder Herbiziden vorliegen.

Besonders günstige Mischpartner sind z.B. die folgenden:

### Fungizide:

Inhibitoren der Nucleinsäure Synthese
Benalaxyl, Benalaxyl-M, Bupirimat, Chiralaxyl, Clozylacon, Dimethirimol, Ethirimol, Furalaxyl, Hymexazol, Metalaxyl, Metalaxyl-M, Ofurace, Oxadixyl, Oxolinsäure
Inhibitoren der Mitose und Zellteilung
Benomyl, Carbendazim, Diethofencarb, Fuberidazole, Pencycuron, Thiabendazol, Thiophanat-methyl, Zoxamid
Inhibitoren der Atmungskette Komplex I
Diflumetorim
Inhibitoren der Atmungskette Komplex II
Boscalid, Carboxin, Fenfuram, Flutolanil, Furametpyr, Mepronil, Oxycarboxin, Penthiopyrad, Thifluzamid
Inhibitoren der Atmungskette Komplex III
Azoxystrobin, Cyazofamid, Dimoxystrobin, Enestrobin, Famoxadon, Fenamidon, Fluoxastrobin, Kresoximmethyl, Metominostrobin, Orysastrobin, Pyraclostrobin, Picoxystrobin, Trifloxystrobin
Entkoppler
Dinocap, Fluazinam
Inhibitoren der ATP Produktion
Fentinacetat, Fentinchlorid, Fentinhydroxid, Silthiofam
Inhibitoren der Aminosäure- und Proteinbiosynthese
Andoprim, Blasticidin-S, Cyprodinil, Kasugamycin, Kasugamycinhydrochlorid Hydrat, Mepanipyrim, Pyrimethanil
Inhibitoren der Signal-Transduktion
Fenpiclonil, Fludioxonil, Quinoxyfen
Inhibitoren der Fett- und Membran Synthese
Chlozolinat, Iprodion, Procymidon, Vinclozolin
Ampropylfos, Kalium-Ampropylfos, Edifenphos, Iprobenfos (IBP), Isoprothiolan, Pyrazophos
Tolclofos-methyl, Biphenyl
Iodocarb, Propamocarb, Propamocarb hydrochlorid
Inhibitoren der Ergosterol Biosynthese
Fenhexamid,
Azaconazol, Bitertanol, Bromuconazol, Cyproconazol, Diclobutrazol, Difenoconazol, Diniconazol, Diniconazol-M, Epoxiconazol, Etaconazol, Fenbuconazol, Fluquinconazol, Flusilazol, Flutriafol, Furconazol, Furconazol-cis, Hexaconazol, Imibenconazol, Ipconazol, Metconazol, Myclobutanil, Paclobutrazol, Penconazol, Propiconazol, Prothioconazol, Simeconazol, Tebuconazol, Tetraconazol, Triadimefon, Triadimenol, Triticonazol, Uniconazol, Voriconazol, Imazalil, Imazalilsulfat, Oxpoconazol, Fenarimol, Flurprimidol, Nuarimol, Pyrifenox, Triforin, Pefurazoat, Prochloraz, Triflumizol, Viniconazol,
Aldimorph, Dodemorph, Dodemorphacetat, Fenpropimorph, Tridemorph, Fenpropidin, Spiroxamin,
Naftifin, Pyributicarb, Terbinafin
Inhibitoren der Zellwand Synthese
Benthiavalicarb, Bialaphos, Dimethomorph, Flumorph, Iprovalicarb, Polyoxins, Polyoxorim, Validamycin A
Inhibitoren der Melanin Biosynthese
Capropamid, Diclocymet, Fenoxanil, Phtalid, Pyroquilon, Tricyclazol
Resistenzinduktion
Acibenzolar-S-methyl, Probenazol, Tiadinil
Multisite
Captafol, Captan, Chlorothalonil, Kupfersalze wie: Kupferhydroxid, Kupfernaphthenat, Kupferoxychlorid, Kupfersulfat, Kupferoxid, Oxin-Kupfer und Bordeaux Mischung, Dichlofluanid, Dithianon, Dodin, Dodin freie Base, Ferbam, Folpet, Fluorofolpet, Guazatin, Guazatinacetat, Iminoctadin, Iminoctadinalbesilat, Iminoctadintriacetat, Mankupfer, Mancozeb, Maneb, Metiram, Metiram Zink, Propineb, Schwefel und Schwefelpräparate enthaltend Calciumpolysulphid, Thiram, Tolylfluanid, Zineb, Ziram
Unbekannter Mechanismus
Amibromdol, Benthiazol, Bethoxazin, Capsimycin, Carvon, Chinomethionat, Chloropicrin, Cufraneb, Cyflufenamid, Cymoxanil, Dazomet, Debacarb, Diclomezine, Dichlorophen, Dicloran, Difenzoquat, Difenzoquat Methylsulphat, Diphenylamin, Ethaboxam, Ferimzon, flumetover, Flusulfamid, Fluopicolid, Fluoroimid, Hexachlorobenzol, 8-Hydroxychinolinsulfat, Irumamycin, Methasulphocarb, Metrafenon, Methyl Isothiocyanat, Mildiomycin, Natamycin, Nickel dimethyldithiocarbamat, Nitrothal-isopropyl, Octhilinon, Oxamocarb, Oxyfenthiin, Pentachlorophenol und Salze, 2-Phenylphenol und Salze, Piperalin, Propanosin -Natrium, Proquinazid, Pyrrolnitrin, Quintozen, Tecloftalam, Tecnazen, Triazoxid, Trichlamid, Zarilamid und 2,3,5,6-Tetrachlor-4-(methylsulfonyl)-pyridin, N-(4-Chlor-2-nitrophenyl)-N-ethyl-4-methyl-benzenesulfonamid, 2-Amino-4-methyl-N-phenyl-5-thiazolecarboxamid, 2-Chlor-N-(2,3-dihydro-1,1,3-trimethyl-1H-inden-4-yl)-3-pyridincarboxamid, 3-[5-(4-Chlorphenyl)-2,3-dimethylisoxazolidin-3-yl]pyridin, cis-1-(4-Chlorphenyl)-2-(1H-1,2,4-triazol-1-yl)-cycloheptanol, 2,4-Dihydro-5-methoxy-2-methyl-4-[[[[1-[3-(trifluoromethyl)-phenyl]-ethyliden]-amino]-oxy]-methyl]-phenyl]-3H-1,2,3-triazol-3-on (185336-79-2), Methyl 1-(2,3-dihydro-2,2-dimethyl-1H-inden-1-yl)-1H-imidazole-5-carboxylat, 3,4,5-Trichlor-2,6-pyridindicarbonitril, Methyl 2-[[[cyclopropyl[(4-methoxyphenyl) imino]methyl]thio]methyl]-.alpha.-(methoxymethylen)- benzacetat, 4-Chlor-alpha-propinyloxy-N-[2-[3-methoxy-4-(2-propinyloxy)phenyl]ethyl]-benzacetamide, (2S)-N-[2-[4-[[3-(4-chlorophenyl)-2-propinyl]oxy]-3-methoxyphenyl]ethyl]-3-methyl-2-[(methylsulfonyl)amino]-butanamid, 5-Chlor-7-(4-methylpiperidin-1-yl)-6-(2,4,6-trifluorophenyl)[1,2,4]triazolo[1,5-a]pyrimidin, 5-Chlor-6-(2,4,6-trifluorophenyl)-N-[(1R)-1,2,2-trimethylpropyl][1,2,4]triazolo[1,5-a]pyrimidin-7-amin, 5-Chlor-N-[(1R)-1,2-dimethylpropyl]-6-(2,4,6-trifluorophenyl) [1,2,4]triazolo[1,5-a]pyrimidin-7-amine, N-[1-(5-Brom-3-chloropyridin-2-yl)ethyl]-2,4-dichloronicotinamid, N-(5-Brom-3-chlorpyridin-2-yl)methyl-2,4-dichlornicotinamid, 2-Butoxy-6-iod-3-propyl-benzopyranon-4-on, N-{(Z)-[(cyclopropylmethoxy) imino][6-(difluormethoxy)-2,3-difluorphenyl]methyl}-2-benzacetamid, N-(3-Ethyl-3,5,5-trimethyl-cyclohexyl)-3-formylamino-2-hydroxy-benzamid, 2-[[[[1-[3(1Fluor-2-phenylethyl)oxy] phenyl] ethyliden]amino]oxy]methyl]-alpha-(methoxyimino)-N-methyl-alphaE-benzacetamid, N-{2-[3-Chlor-5-(trifluormethyl)pyridin-2-yl]ethyl}-2-(trifluoromethyl)benzamid, N-(3',4'-dichlor-5-fluorbiphenyl-2-yl)-3-(difluormethyl)-1-methyl-1H-pyrazol-4-carboxamid, N-(6-Methoxy-3-pyridinyl)-cyclopropan carboxamid, 1-[(4-Methoxyphenoxy)methyl]-2,2-dimethylpropyl-1H-imidazol-1- carbonsäure, O-[1-[(4-Methoxyphenoxy)methyl]-2,2-dimethylpropyl]-1H-imidazol- 1- carbothioic acid, 2-(2-{[6-(3-Chlor-2-methylphenoxy)-5-fluorpyrimidin-4-yl]oxy}phenyl)-2-(methoxyimino)-N-methylacetamid

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Acetylcholinesterase (AChE) Inhibitoren
Carbamate,
   zum Beispiel Alanycarb, Aldicarb, Aldoxycarb, Allyxycarb, Aminocarb, Bendiocarb, Benfuracarb, Bufencarb, Butacarb, Butocarboxim, Butoxycarboxim, Carbaryl, Carbofuran, Carbosulfan, Cloethocarb, Dimetilan, Ethiofencarb, Fenobucarb, Fenothiocarb, Formetanate, Furathiocarb, Isoprocarb, Metam-sodium, Methiocarb, Methomyl, Metolcarb, Oxamyl, Pirimicarb, Promecarb, Propoxur, Thiodicarb, Thiofanox, Trimethacarb, XMC, Xylylcarb, Triazamate
Organophosphate,
   zum Beispiel Acephate, Azamethiphos, Azinphos (-methyl, -ethyl), Bromophos-ethyl, Bromfenvinfos (-methyl), Butathiofos, Cadusafos, Carbophenothion, Chlorethoxyfos, Chlorfenvinphos, Chlormephos, Chlorpyrifos (-methyl/-ethyl), Coumaphos, Cyanofenphos, Cyanophos, Chlorfenvinphos, Demeton-S-methyl, Demeton-S-methylsulphon, Dialifos, Diazinon, Dichlofenthion, Dichlorvos/DDVP, Dicrotophos, Dimethoate, Dimethylvinphos, Dioxabenzofos, Disulfoton, EPN, Ethion, Ethoprophos, Etrimfos, Famphur, Fenamiphos, Fenitrothion, Fensulfothion, Fenthion, Flupyrazofos, Fonofos, Formothion, Fosmethilan, Fosthiazate, Heptenophos, Iodofenphos, Iprobenfos, Isazofos, Isofenphos, Isopropyl O-salicylate, Isoxathion, Malathion, Mecarbam, Methacrifos, Methamidophos, Methidathion, Mevinphos, Monocrotophos, Naled, Omethoate, Oxydemetonmethyl, Parathion (-methyl/-ethyl), Phenthoate, Phorate, Phosalone, Phosmet, Phosphamidon, Phosphocarb, Phoxim, Pirimiphos (-methyl/-ethyl), Profenofos, Propaphos, Propetamphos, Prothiofos, Prothoate, Pyraclofos, Pyridaphenthion, Pyridathion, Quinalphos, Sebufos, Sulfotep, Sulprofos, Tebupirimfos, Temephos, Terbufos, Tetrachlorvinphos, Thiometon, Triazophos, Triclorfon, Vamidothion
Natrium-Kanal-Modulatoren / Spannungsabhängige Natrium-Kanal-Blocker
Pyrethroide,
   zum Beispiel Acrinathrin, Allethrin (d-cis-trans, d-trans), Beta-Cyfluthrin, Bifenthrin, Bioallethrin, Bioallethrin-S-cyclopentyl-isomer, Bioethanomethrin, Biopermethrin, Bioresmethrin, Chlovaporthrin, Cis-Cypermethrin, Cis-Resmethrin, Cis-Permethrin, Clocythrin, Cycloprothrin, Cyfluthrin, Cyhalothrin, Cypermethrin (alpha-, beta-, theta-, zeta-), Cyphenothrin, Deltamethrin, Empenthrin (1R-isomer), Esfenvalerate, Etofenprox, Fenfluthrin, Fenpropathrin, Fenpyrithrin, Fenvalerate, Flubrocythrinate, Flucythrinate, Flufenprox, Flumethrin, Fluvalinate, Fubfenprox, Gamma-Cyhalothrin, Imiprothrin, Kadethrin, Lambda- Cyhalothrin, Metofluthrin, Permethrin (cis- , trans-), Phenothrin (1R-trans isomer), Prallethrin, Profluthrin, Protrifenbute, Pyresmethrin, Resmethrin, RU 15525, Silafluofen, Tau-Fluvalinate, Tefluthrin, Terallethrin, Tetramethrin (-1R-isomer), Tralomethrin, Transfluthrin, ZXI 8901, Pyrethrins (pyrethrum)
DDT
Oxadiazine,
   zum Beispiel Indoxacarb
Semicarbazon,
   zum Beispiel Metaflumizon (BAS3201)
Acetylcholin-Rezeptor-Agonisten/-Antagonisten
Chloronicotinyle,
   zum Beispiel Acetamiprid, Clothianidin, Dinotefuran, Imidacloprid, Nitenpyram, Nithiazine, Thiacloprid, Thiamethoxam, AKD 1022, Imidaclothiz
Nicotine, Bensultap, Cartap
Acetylcholin-Rezeptor-Modulatoren
Spinosyne,
   zum Beispiel Spinosad
GABA-gesteuerte Chlorid-Kanal-Antagonisten
Organochlorine,
   zum Beispiel Camphechlor, Chlordane, Endosulfan, Gamma-HCH, HCH, Heptachlor, Lindane, Methoxychlor
Fiprole,
   zum Beispiel Acetoprole, Ethiprole, Fipronil, Pyrafluprole, Pyriprole, Vaniliprole
Chlorid-Kanal-Aktivatoren
Mectine,
   zum Beispiel Abamectin, Emamectin, Emamectin-benzoate, Ivermectin, Lepimectin, Milbemycin Juvenilhormon-Mimetika,
   zum Beispiel Diofenolan, Epofenonane, Fenoxycarb, Hydroprene, Kinoprene, Methoprene, Pyriproxifen, Triprene
Ecdysonagonisten/disruptoren
Diacylhydrazine,
   zum Beispiel Chromafenozide, Halofenozide, Methoxyfenozide, Tebufenozide
Inhibitoren der Chitinbiosynthese
Benzoylharnstoffe,
   zum Beispiel Bistrifluron, Chlofluazuron, Diflubenzuron, Fluazuron, Flucycloxuron, Flufenoxuron, Hexaflumuron, Lufenuron, Novaluron, Noviflumuron, Penfluron, Teflubenzuron, Triflumuron
   Buprofezin
   Cyromazine
Inhibitoren der oxidativen Phosphorylierung, ATP-Disruptoren
   Diafenthiuron
   Organozinnverbindungen,
   zum Beispiel Azocyclotin, Cyhexatin, Fenbutatin-oxide
Entkoppler der oxidativen Phoshorylierung durch Unterbrechung des H-Protongradienten
Pyrrole,
   zum Beispiel Chlorfenapyr
Dinitrophenole,
   zum Beispiel Binapacyrl, Dinobuton, Dinocap, DNOC, Meptyldinocap
Seite-I-Elektronentransportinhibitoren
METI's,
   zum Beispiel Fenazaquin, Fenpyroximate, Pyrimidifen, Pyridaben, Tebufenpyrad, Tolfenpyrad
   Hydramethylnon
   Dicofol
Seite-II-Elektronentransportinhibitoren
Rotenone
Seite-III-Elektronentransportinhibitoren
Acequinocyl, Fluacrypyrim
Mikrobielle Disruptoren der Insektendarmmembran
Bacillus thuringiensis-Stämme
Inhibitoren der Fettsynthese
Tetronsäuren,
zum Beispiel Spirodiclofen, Spiromesifen,
Tetramsäuren,
zum Beispiel cis-3-(2,5-dimethylphenyl)-4-hydroxy-8-methoxy-1-azaspiro[4.5]dec-3-en-2-on
   Carboxamide,
   zum Beispiel Flonicamid
   Oktopaminerge Agonisten,
   zum Beispiel Amitraz
Inhibitoren der Magnesium-stimulierten ATPase,
   Propargite
   Nereistoxin-Analoge,
   zum Beispiel Thiocyclam hydrogen oxalate, Thiosultap-sodium
Agonisten des Ryanodin-Rezeptors,
   Benzoesäuredicarboxamide,
   zum Beispiel Flubendiamide
   Anthranilamide,
   zum Beispiel Rynaxypyr (3-Bromo-N-{4-chloro-2-methyl-6-[(methylamino)carbonyl]phenyl}-1-(3-chloropyridin-2-yl)-1H-pyrazole-5-carboxamide)
Biologika, Hormone oder Pheromone
Azadirachtin, Bacillus spec., Beauveria spec., Codlemone, Metarrhizium spec., Paecilomyces spec., Thuringiensin, Verticillium spec.
Wirkstoffe mit unbekannten oder nicht spezifischen Wirkmechanismen
Begasungsmittel,
   zum Beispiel Aluminium phosphide, Methyl bromide, Sulfuryl fluoride
Fraßhemmer,
   zum Beispiel Cryolite, Flonicamid, Pymetrozine
Milbenwachstumsinhibitoren,
   zum Beispiel Clofentezine, Etoxazole, Hexythiazox
Amidoflumet, Benclothiaz, Benzoximate, Bifenazate, Bromopropylate, Buprofezin, Chinomethionat, Chlordimeform, Chlorobenzilate, Chloropicrin, Clothiazoben, Cycloprene, Cyflumetofen, Dicyclanil, Fenoxacrim, Fentrifanil, Flubenzimine, Flufenerim, Flutenzin, Gossyplure, Hydramethylnone, Japonilure, Metoxadiazone, Petroleum, Piperonyl butoxide, Potassium oleate, Pyridalyl, Sulfluramid, Tetradifon, Tetrasul, Triarathene,Verbutin oder Lepimectin
Insektizide Verbindungen aus der Klasse der Butenolide
   zum Beispiel
4- {[(6-Brompyrid-3-yl)methyl](2-fluorethyl)amino}furan-2(5H)-on
4- {[(6-Fluorpyrid-3-yl)methyl](2,2-difluorethyl)amino}furan-2(5H)-on
4- {[(2-Chlor-1,3-thiazol-5-yl)methyl](2-fluorethyl)amino}furan-2(5H)-on
4- {[(6-Chlorpyrid-3-yl)methyl](2-fluorethyl)amino}furan-2(5H)-on
4- {[(6-Chlorpyrid-3-yl)methyl](2,2-difluorethyl)amino}furan-2(5H)-on
4- {[(6-Chlor-5-fluorpyrid-3-yl)methyl](methyl)amino}furan-2(5H)-on
4- {[(5,6-Dichlorpyrid-3-yl)methyl](2-fluorethyl)amino}furan-2(5H)-on
4- {[(6-Chlor-5-fluorpyrid-3-yl)methyl](cyclopropyl)amino}furan-2(5H)-on
4- {[(6-Chlorpyrid-3-yl)methyl](cyclopropyl)amino}furan-2(5H)-on
4- {[(6-Chlorpyrid-3-yl)methyl](methyl)amino}furan-2(5H)-on.

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Der Wirkstoffgehalt der aus den handelsüblichen Formulierungen bereiteten Anwendungsformen kann von 0,00000001 bis zu 95 Gew.-%, vorzugsweise zwischen 0,00001 und 1 Gew.-% Wirkstoff liegen.

**Tabelle 1: Pflanze: Mais**

| Betroffene Struktur bzw. Exprimiertes Prinzip | Merkmal der Pflanze / Toleranz gegenüber |
|---|---|
| Acetolactatsynthase (ALS) | Sulfonylharnstoffverbindungen, Imidazolinone |
| | Triazolpyrimidine, Pyrimidyloxybenzoate, |
| | Phthalide |
| Acetyl-CoA-Carboxylase (ACCase) | Aryloxyphenoxyalkancarbonsäure, |
| | Cyclohexandion |
| Hydroxyphenylpyruvat-Dioxygenase (HPPD) | Isooxazole wie Isoxaflutol oder Isoxachlortol, |
| | Trione wie Mesotrion oder Sulcotrion |
| Phosphinothricinacetyltransferase | Phosphinothricin |
| O-Methyltransferase | Geänderter Ligningehalt |
| Glutaminsynthetase | Glufosinat, Bialaphos |
| Adenylosuccinatlyase (ADSL) | Inhibitoren der IMP- und AMP- Synthese |
| Adenylosuccinatsynthase | Inhibitoren der Adenylosuccinatsynthese |
| Anthranilatsynthase | Inhibitoren der Tryptophansynthese und des |
| | -abbaus |
| Nitrilase | 3,5-Dihalogen-4-hydroxy-benzonitrile wie |
| | Bromoxynil und Loxinyl |
| 5-Enolpyruvyl-3-phosphoshikimat- | Glyphosat oder Sulfosat |
| Synthase (EPSPS) | |
| Glyphosatoxidoreductase | Glyphosat oder Sulfosat |
| Protoporphyrinogenoxidase (PROTOX) | Diphenylether, cyclisches Imid, |
| | Phenylpyrazol, Pyridinderivat, |
| | Phenopylat, Oxadiazol usw. |
| Cytochrom P450 z.B. P450 SU1 | Xenobiotika und Herbizide wie |
| | Sulfonylharnstoff |
| Dimboa-Biosynthese (Bx1-Gen) | Helminthosporium turcicum, |
| | Rhopalosiphum maydis, Diplodia |
| | maydis, Ostrinia nubilalis, lepidoptera sp. |
| CMIII (kleiner grundlegender Peptidbaustein | Pflanzenpathogene z.B. Fusarium, Alternaria, |
| aus dem Maiskorn) | Sclerotina |
| Com- SAFP (Zeamatin) | Pflanzenpathogene, z.B. Fusarium, |
| | Alternaria, Sclerotina, Rhizoctonia, |
| | Chaetomium, Phycomycen |
| Hm1-Gen | Cochliobulus |
| Chitinasen | Pflanzenpathogene |
| Glucanasen | Pflanzenpathogene |
| Hüllproteine | Viren wie das Maisverzwergungsvirus |
| | (MDMV) |
| Toxine von Bacillus thuringiensis, VIP 3, | Lepidoptera, Coleoptera, Diptera, |
| Bacillus-cereus-Toxin, Photorabdus und | Nematoden, z.B. Ostrinia nubilalis, |
| Toxine von Xenorhabdus | Heliothis zea, Heerwürmer z.B. |
| | Spodoptera frugiperda, Maiswurzelbohrer, |
| | Sesamia sp., Aprotis ipsilon, Asiatischer Mais- |
| | zünsler, Rüsselkäfer |
| 3- Hydroxysteroidoxidase | Lepidoptera, Coleoptera, Diptera, Nematoden, |
| | z.B. Ostrinia nubilalis, Heliothis zea, |
| | Heerwürmer z.B. Spodoptera frugiperda, |
| | Maiswurzelbohrer, Sesamia sp., Aprotis ipsilon, |
| | Asiatischer Maiszünsler, Rüsselkäfer |
| Peroxidase | Lepidoptera, Coleoptera, Diptera, Nematoden, |
| | z.B. Ostrinia nubilalis, Heliothis zea, |
| | Heerwürmer z.B. Spodoptera frugiperda, |
| | Maiswurzelbohrer, Sesamia sp., Aprotis ipsilon, |
| | Asiatischer Maiszünsler, Rüsselkäfer |
| Aminopeptidase-Inhibitoren z.B. Leucin | Lepidoptera, Coleoptera, Diptera, |
| Aminopeptidase-Inhibitoren (LAPI) | Nematoden, z.B. Ostrinia nubilalis, |
| | Heliothis zea, Heerwürmer z.B. Spodoptera |
| | frugiperda, Maiswurzelbohrer, Sesamia sp., |
| | Aprotis ipsilon, Asiatischer Maiszünsler, |
| | Rüsselkäfer |
| Limonensynthase | Maiswurzelbohrer |
| Lektin | Lepidoptera, Coleoptera, Diptera, Nematoden, |
| | z.B. Ostrinia nubilalis, Heliothis zea, |
| | Heerwürmer z.B. Spodoptera frugiperda, |
| | Maiswurzelbohrer, Sesamia sp., Aprotis ipsilon, |
| | Asiatischer Maiszünsler, Rüsselkäfer |
| Protease-Inhibitoren z.B. Cystatin, Patatin, | Rüsselkäfer, Maiswurzelbohrer |
| Virgiferin, CPTI | |
| Ribosomeninaktivierendes Protein | Lepidoptera, Coleoptera, Diptera, Nematoden, |
| | z.B. Ostrinia nubilalis, Heliothis zea, |
| | Heerwürmer z.B. Spodoptera frugiperda, |
| | Maiswurzelbohrer, Sesamia sp., Aprotis ipsilon, |
| | Asiatischer Mais zünsler, Rüsselkäfer |
| 5C9-Maispolypeptid | Lepidoptera, Coleoptera, Diptera, Nematoden, |
| | z.B. Ostrinia nubilalis, Heliothis zea, |
| | Heerwürmer z.B. Spodoptera frugiperda, |
| | Maiswurzelbohrer, Sesamia sp., Aprotis ipsilon, |
| | Asiatischer Mais zünsler, Rüsselkäfer |
| HMG-CoA-Reductase | Lepidoptera, Coleoptera, Diptera, Nematoden, |
| | z.B. Ostrinia nubilalis, Heliothis zea, |
| | Heerwürmer z.B. Spodoptera frugiperda, |
| | Maiswurzelbohrer, Sesamia sp., Aprotis ipsilon, |
| | Asiatischer Mais zünsler, Rüsselkäfer |

| Pflanze: Weizen | |
|---|---|
| Betroffene Struktur/ Exprimiertes Protein | Merkmal der Pflanze / Toleranz gegenüber |
| Acetolactatsynthase (ALS) | Sulfonylharnstoffverbindungen, Imidazolinone |
| | Triazolpyrimidine, Pyrimidyloxybenzoate, |
| | Phthalide |
| Acetyl-CoA-Carboxylase (ACCase) | Aryloxyphenoxyalkancarbonsäuren, |
| | Cyclohexandione |
| Hydroxyphenylpyruvat-Dioxygenase (HPPD) | Isoxazole wie etwa Isoxaflutol oder |
| | Isoxachlortol, Trione wie etwa Mesotrion |
| | oder Sulcotrion |
| Phosphinothricinacetyltransferase | Phosphinothricin |
| O-Methyltransferase | Geänderter Ligningehalt |
| Glutaminsynthetase | Glufosinat, Bialaphos |
| Adenylosuccinatlyase (ADSL) | Inhibitoren der IMP- und AMP-Synthese |
| Adenylosuccinatsynthase | Inhibitoren der Adenylosuccinatsynthese |
| Anthranilatsynthase | Inhibitoren der Tryptophansynthese und des |
| | -abbaus |
| Nitrilase | 3,5-Dihalogen-4-hydroxy-benzonitrile wie |
| | Bromoxynil und Loxinyl |
| 5-Enolpyruvyl-3-phosphoshikimat- | Glyphosat oder Sulfosat |
| Synthase (EPSPS) | |
| Glyphosatoxidoreductase | Glyphosat oder Sulfosat |
| Protoporphyrinogenoxidase (PROTOX) | Diphenylether, cyclische Imide, Phenylpyrazole, |
| | Pyridinderivate, Phenopylat, Oxadiazole usw. |
| Cytochrom P450 z.B. P450 SU1 | Xenobiotika und Herbizide wie etwa |
| | Sulfonylharnstoffverbindungen |
| Pilzbekämpfendes Polypeptid AlyAFP | Pflanzenpathogene, z.B. Septoria und Fusarium |
| Glucoseoxidase | Pflanzenpathogene, z.B. Fusarium, Septoria |
| Pyrrolnitrinsynthese-Gene | Pflanzenpathogene, z.B. Fusarium, Septoria |
| Serin/Threonin-Kinasen | Pflanzenpathogene, z.B. Fusarium, Septoria |
| | und andere Krankheiten |
| Polypeptid mit der Wirkung, eine Über- | Pflanzenpathogene, z.B. Fusarium, Septoria und |
| empfindlichkeitsreaktion auszulösen | andere Krankheiten |
| Gene der systemischen erworbenen | Virale, bakterielle Pilz- und Nematoden- |
| Widerstandskraft (SAR) | Pathogene |
| Chitinasen | Pflanzenpathogene |
| Glucanasen | Pflanzenpathogene |
| Doppelstrang-Ribonuclease | Viren wie etwa BYDV und MSMV |
| Hüllproteine | Viren wie etwa BYDV und MSMV |
| Toxine von Bacillus thuringiensis, VIP 3, | Lepidoptera, Coleoptera, Diptera, |
| Bacillus-cereus-Toxine, Photorabdus und | Nematoden |
| Toxine von Xenorhabdus | |
| 3- Hydroxysteroidoxidase | Lepidoptera, Coleoptera, Diptera, |
| | Nematoden |
| Peroxidase | Lepidoptera, Coleoptera, Diptera, |
| | Nematoden |
| Aminopeptidase-Inhibitoren, z.B. Leucin | Lepidoptera, Coleoptera, Diptera, |
| Aminopeptidase-Inhibitor | Nematoden |
| Lektine | Lepidoptera, Coleoptera, Diptera, |
| | Nematoden, Blattläuse |
| Protease-Inhibitoren, z.B. Cystatin, Patatin, | Lepidoptera, Coleoptera, Diptera, |
| Virgiferin, CPTI | Nematoden, Blattläuse |
| Ribosomeninaktivierendes Protein | Lepidoptera, Coleoptera, Diptera, |
| | Nematoden, Blattläuse |
| HMG-CoA-Reductase | Lepidoptera, Coleoptera, Diptera, |
| | Nematoden, z.B. Ostrinia nubilalis, |
| | Heliothis zea, Heerwürmer z.B. Spodoptera |
| | frugiperda, Maiswurzelbohrer, Sesamia sp., |
| | Aprotis ipsilon, Asiatischer Maiszünsler, |
| | Rüsselkäfer |

| Pflanze: Gerste | |
|---|---|
| Betroffene Struktur/ Exprimiertes Protein | Merkmal der Pflanze / Toleranz gegenüber |
| Acetolactatsynthase (ALS) | Sulfonylharnstoffverbindungen, Imidazolinone |
| | Triazolpyrimidine, |
| | Pyrimidyloxybenzoate, Phthalide |
| Acetyl-CoA-Carboxylase (ACCase) | Aryloxyphenoxyalkancarbonsäuren, |
| | Cyclohexandione |
| Hydroxyphenylpyruvat-Dioxygenase (HPPD) | Isoxazole wie etwa Isoxaflutol oder |
| | Isoxachlortol, Trione wie etwa Mesotrion |
| | oder Sulcotrion |
| Phosphinothricinacetyltransferase | Phosphinothricin |
| O-Methyltransferase | Geänderter Ligningehalt |
| Glutaminsynthetase | Glufosinat, Bialaphos |
| Adenylosuccinatlyase (ADSL) | Inhibitoren der IMP- und AMP-Synthese |
| Adenylosuccinatsynthase | Inhibitoren der Adenylosuccinatsynthese |
| Anthranilatsynthase | Inhibitoren der Tryptophansynthese und des |
| | -abbaus |
| Nitrilase | 3,5-Dihalogen-4-hydroxy-benzonitrile wie |
| | Bromoxynil und Loxinyl |
| 5-Enolpyruvyl-3-phosphoshikimat- | Glyphosat oder Sulfosat |
| Synthase (EPSPS) | |
| Glyphosatoxidoreductase | Glyphosat oder Sulfosat |
| Protoporphyrinogenoxidase (PROTOX) | Diphenylether, cyclische Imide, |
| | Phenylpyrazole, Pyridinderivate, |
| | Phenopylat, Oxadiazole usw. |
| Cytochrom P450 z.B. P450 SU1 | Xenobiotika und Herbizide wie etwa |
| | Sulfonylharnstoffverbindungen |
| Pilzbekämpfendes Polypeptid AlyAFP | Pflanzenpathogene, z.B. Septoria und Fusarium |
| Glucoseoxidase | Pflanzenpathogene, z.B. Fusarium, Septoria |
| Pyrrolnitrinsynthese-Gene | Pflanzenpathogene, z.B. Fusarium, Septoria |
| Serin/Threonin-Kinasen | Pflanzenpathogene, z.B. Fusarium, Septoria |
| | und andere Krankheiten |
| Polypeptid mit der Wirkung, eine | Pflanzenpathogene, z.B. Fusarium, Septoria und |
| Überempfindlichkeitsreaktion auszulösen | andere Krankheiten |
| Gene der systemischen erworbenen | Virale, bakterielle Pilz- und Nematoden- |
| Widerstandskraft (SAR) | Pathogene |
| Chitinasen | Pflanzenpathogene |
| Glucanasen | Pflanzenpathogene |
| Doppelstrang-Ribonuclease | Viren wie etwa BYDV und MSMV |
| Hüllproteine | Viren wie etwa BYDV und MSMV |
| Toxine von Bacillus thuringiensis, VIP 3, | Lepidoptera, Coleoptera, Diptera, |
| Bacillus-cereus-Toxine, Photorabdus und | Nematoden |
| Toxine von Xenorhabdus | |
| 3- Hydroxysteroidoxidase | Lepidoptera, Coleoptera, Diptera, |
| | Nematoden |
| Peroxidase | Lepidoptera, Coleoptera, Diptera, |
| | Nematoden |
| Aminopeptidase-Inhibitoren, z.B. Leucin | Lepidoptera, Coleoptera, Diptera, |
| Aminopeptidase-Inhibitor | Nematoden |
| Lektine | Lepidoptera, Coleoptera, Diptera, |
| | Nematoden, Blattläuse |
| Protease-Inhibitoren, z.B. Cystatin, Patatin, | Lepidoptera, Coleoptera, Diptera, |
| Virgiferin, CPTI | Nematoden, Blattläuse |
| Ribosomeninaktivierendes Protein | Lepidoptera, Coleoptera, Diptera, |
| | Nematoden, Blattläuse |
| HMG-CoA-Reductase | Lepidoptera, Coleoptera, Diptera, |
| | Nematoden, Blattläuse |

| Pflanze: Reis | |
|---|---|
| Betroffene Struktur/ Exprimiertes Prinzip | Merkmal der Pflanze / Toleranz gegenüber |
| Acetolactatsynthase (ALS) | Sulfonylharnstoffverbindungen, Imidazolinone |
| | Triazolpyrimidine, |
| | Pyrimidyloxybenzoate, Phthalide |
| Acetyl-CoA-Carboxylase (ACCase) | Aryloxyphenoxyalkancarbonsäuren, |
| | Cyclohexandione |
| Hydroxyphenylpyruvat-Dioxygenase (HPPD) | Isoxazole wie etwa Isoxaflutol oder |
| | Isoxachlortol, Trione wie etwa |
| | Mesotrion oder Sulcotrion |
| Phosphinothricinacetyltransferase | Phosphinothricin |
| O-Methyltransferase | Geänderter Ligningehalt |
| Glutaminsynthetase | Glufosinat, Bialaphos |
| Adenylosuccinatlyase (ADSL) | Inhibitoren der IMP- und AMP-Synthese |
| Adenylosuccinatsynthase | Inhibitoren der Adenylosuccinatsynthese |
| Anthranilatsynthase | Inhibitoren der Tryptophansynthese und des |
| | -abbaus |
| Nitrilase | 3,5-Dihalogen-4-hydroxy-benzonitrile wie |
| | Bromoxynil und Loxinyl |
| 5-Enolpyruvyl-3-phosphoshikimat | Glyphosat oder Sulfosat |
| Synthase (EPSPS) | |
| Glyphosatoxidoreductase | Glyphosat oder Sulfosat |
| Protoporphyrinogenoxidase (PROTOX) | Diphenylether, cyclische Imide, |
| | Phenylpyrazole, Pyridinderivate, |
| | Phenopylat, Oxadiazole usw. |
| Cytochrom P450 z.B. P450 SU1 | Xenobiotika und Herbizide wie etwa |
| | Sulfonylharnstoffverbindungen |
| Pilzbekämpfendes Polypeptid AlyAFP | Pflanzenpathogene |
| Glucoseoxidase | Pflanzenpathogene |
| Pyrrolnitrinsynthese-Gene | Pflanzenpathogene |
| Serin/Threonin-Kinasen | Pflanzenpathogene |
| Phenylalanin-Ammoniak-Lyase (PAL) | Pflanzenpathogene, z.B. bakterieller |
| | Blattmehltau und induzierbare Reisbräune |
| Phytoalexine | Pflanzenpathogene, z.B. bakterieller |
| | Blattmehltau und Reisbräune |
| B-1,3-Glucanase (Antisense) | Pflanzenpathogene, z.B. bakterieller |
| | Blattmehltau und Reisbräune |
| Rezeptorkinase | Pflanzenpathogene, z.B. bakterieller |
| | Blattmehltau und Reisbräune |
| Polypeptid mit der Wirkung, eine | Pflanzenpathogene |
| Überempfindlichkeitsreaktion auszulösen | |
| Gene der systemischen erworbenen | Virale, bakterielle Pilz- und Nematoden- |
| Widerstandskraft (SAR) | Pathogene |
| Chitinasen | Pflanzenpathogene, z.B. bakterieller |
| | Blattmehltau und Reisbräune |
| Glucanasen | Pflanzenpathogene |
| Doppelstrang-Ribonuclease | Viren wie etwa BYDV und MSMV |
| Hüllproteine | Viren wie etwa BYDV und MSMV |
| Toxine von Bacillus thuringiensis, VIP 3, | Lepidoptera, z.B. Stengelbohrer, Coleoptera, |
| Bacillus-cereus-Toxine, Photorabdus und | z.B. Rüsselkäfer wie Lissorhoptrus oryzophilus, |
| Toxine von Xenorhabdus | Diptera, Reiszikaden, z.B. braunrückige |
| | Reiszikade |
| 3- Hydroxysteroidoxidase | Lepidoptera, z.B. Stengelbohrer, Coleoptera, |
| | z.B. Rüsselkäfer wie Lissorhoptrus oryzophilus, |
| | Diptera, Reiszikaden, z.B. braunrückige |
| | Reiszikade |
| Peroxidase | Lepidoptera, z.B. Stengelbohrer, Coleoptera, |
| | z.B. Rüsselkäfer wie Lissorhoptrus oryzophilus, |
| | Diptera, Reiszikaden, z.B. braunrückige |
| | Reiszikade |
| Aminopeptidase-Inhibitoren, z.B. Leucin | Lepidoptera, z.B. Stengelbohrer, Coleoptera, |
| Aminopeptidase-Inhibitor | z.B. Rüsselkäfer wie Lissorhoptrus |
| | oryzophilus, Diptera, Reiszikaden, z.B. |
| | braunrückige Reiszikade |
| Lektine | Lepidoptera, z.B. Stengelbohrer, Coleoptera, |
| | z.B. Rüsselkäfer wie Lissorhoptrus |
| | oryzophilus, Diptera, Reiszikaden, z.B. |
| | braunrückige Reiszikade |
| Protease-Inhibitoren, | Lepidoptera, z.B. Stengelbohrer, Coleoptera, |
| | z.B. Rüsselkäfer wie Lissorhoptrus oryzophilus, |
| | Diptera, Reiszikaden z.B. braunrückige |
| | Reiszikade |
| Ribosomeninaktivierendes Protein | Lepidoptera, z.B. Stengelbohrer, Coleoptera, |
| | z.B. Rüsselkäfer wie Lissorhoptrus |
| | oryzophilus, Diptera, Reiszikaden, z.B. |
| | braunrückige Reiszikade |
| HMG-CoA-Reductase | Lepidoptera, z.B. Stengelbohrer, Coleoptera, |
| | z.B. Rüsselkäfer wie Lissorhoptrus |
| | oryzophilus, Diptera, Reiszikaden z.B. |
| | braunrückige Reiszikade |

| Pflanze: Sojabohne | |
|---|---|
| Betroffene Struktur/ Exprimiertes Prinzip | Merkmal der Pflanze / Toleranz gegenüber |
| Acetolactatsynthase (ALS) | Sulfonylharnstoffverbindungen, Imidazolinone |
| | Triazolpyrimidine, |
| | Pyrimidyloxybenzoate, Phthalide |
| Acetyl-CoA-Carboxylase (ACCase) | Aryloxyphenoxyalkancarbonsäuren, |
| | Cyclohexandione |
| Hydroxyphenylpyruvat-Dioxygenase (HPPD) | Isoxazole wie etwa Isoxaflutol oder |
| | Isoxachlortol, Trione wie etwa |
| | Mesotrion oder Sulcotrion |
| Phosphinothricinacetyltransferase | Phosphinothricin |
| O-Methyltransferase | Geänderter Ligningehalt |
| Glutaminsynthetase | Glufosinat, Bialaphos |
| Adenylosuccinatlyase (ADSL) | Inhibitoren der IMP- und AMP-Synthese |
| Adenylosuccinatsynthase | Inhibitoren der Adenylosuccinatsynthese |
| Anthranilatsynthase | Inhibitoren der Tryptophansynthese und des |
| | -abbaus |
| Nitrilase | 3,5-Dihalogen-4-hydroxy-benzonitrile wie |
| | Bromoxynil und Loxinyl |
| 5-Enolpyruvyl-3-phosphoshikimat- | Glyphosat oder Sulfosat |
| Synthase (EPSPS) | |
| Glyphosatoxidoreductase | Glyphosat oder Sulfosat |
| Protoporphyrinogenoxidase (PROTOX) | Diphenylether, cyclische Imide, |
| | Phenylpyrazole, Pyridinderivate, |
| | Phenopylat, Oxadiazole usw. |
| Cytochrom P450 z.B. P450 SU1 oder Auswahl | Xenobiotika und Herbizide wie etwa |
| | Sulfonylharnstoffverbindungen |
| Pilzbekämpfendes Polypeptid AlyAFP | Bakterielle und Pilz-Pathogene wie etwa |
| | Fusarium, Sklerotinia, Weißstängeligkeit |
| Oxalatoxidase | Bakterielle und Pilz-Pathogene wie etwa |
| | Fusarium, Sklerotinia, Weißstängeligkeit |
| Glucoseoxidase | Bakterielle und Pilz-Pathogene wie etwa |
| | Fusarium, Sklerotinia, Weißstängeligkeit |
| Pyrrolnitrinsynthese-Gene | Bakterielle und Pilz-Pathogene wie etwa |
| | Fusarium, Sklerotinia, Weißstängeligkeit |
| Serin/Threonin-Kinasen | Bakterielle und Pilz-Pathogene wie etwa |
| | Fusarium, Sklerotinia, Weißstängeligkeit |
| Phenylalanin-Ammoniak-Lyase (PAL) | Bakterielle und Pilz-Pathogene wie etwa |
| | Fusarium, Sklerotinia, Weißstängeligkeit |
| Phytoalexine | Pflanzenpathogene, z.B. bakterieller Blatt- |
| | mehltau und Reisbräune |
| B-1,3-glucanase (Antisense) | Pflanzenpathogene, z.B. bakterieller Blatt- |
| | mehltau und Reisbräune |
| Rezeptorkinase | Bakterielle und Pilz-Pathogene wie etwa |
| | Fusarium, Sklerotinia, Weißstängeligkeit |
| Polypeptid mit der Wirkung, eine Über- | Pflanzenpathogene |
| Empfindlichkeitsreaktion auszulösen | |
| Gene der systemischen erworbenen | Virale, bakterielle Pilz- und Nematoden- |
| Widerstandskraft (SAR) | Pathogene |
| Chitinasen | Bakterielle und Pilz-Pathogene wie etwa |
| | Fusarium, Sklerotinia, Weißstängeligkeit |
| Glucanasen | Bakterielle und Pilz-Pathogene wie etwa |
| | Fusarium, Sklerotinia, Weißstängeligkeit |
| Doppelstrang-Ribonuclease | Viren wie etwa BPMV und SbMV |
| Hüllproteine | Viren wie etwa BYDV und MSMV |
| Toxine von Bacillus thuringiensis, VIP 3, | Lepidoptera, Coleoptera, Blattläuse |
| Bacillus-cereus-Toxine, Photorabdus und | |
| Toxine von Xenorhabdus | |
| 3- Hydroxysteroidoxidase | Lepidoptera, Coleoptera, Blattläuse |
| Peroxidase | Lepidoptera, Coleoptera, Blattläuse |
| Aminopeptidase-Inhibitoren, z.B. Leucin | Lepidoptera, Coleoptera, Blattläuse |
| Aminopeptidase-Inhibitor | |
| Lektine | Lepidoptera, Coleoptera, Blattläuse |
| Protease-Inhibitoren, z.B. Virgiferin | Lepidoptera, Coleoptera, Blattläuse |
| Ribosomeninaktivierendes Protein | Lepidoptera, Coleoptera, Blattläuse |
| HMG-CoA-Reductase | Lepidoptera, Coleoptera, Blattläuse |
| Barnase | Nematoden, z.B. Wurzelknotennematoden und |
| | Zystennematoden |
| Schlüpfauslösefaktor für Zystennematoden | Zystennematoden |
| Prinzipien zur Verhinderung der Nahrungs- | Nematoden, z.B. Wurzelknoten-Nematoden und |
| aufnahme | Zystennematoden |

| Pflanze: Kartoffel | |
|---|---|
| Betroffene Struktur/ Exprimiertes Protein | Merkmal der Pflanze / Toleranz gegenüber |
| Acetolactatsynthase (ALS) | Sulfonylharnstoffverbindungen, Imidazolinone |
| | Triazolpyrimidine, |
| | Pyrimidyloxybenzoate, Phthalide |
| Acetyl-CoA-Carboxylase (ACCase) | Aryloxyphenoxyalkancarbonsäuren, |
| | Cyclohexandione |
| Hydroxyphenylpyruvat-Dioxygenase (HPPD) | Isoxazole wie etwa Isoxaflutol oder |
| | Isoxachlortol, Trione wie etwa |
| | Mesotrion oder Sulcotrion |
| Phosphinothricinacetyltransferase | Phosphinothricin |
| O-Methyltransferase | Geänderter Ligningehalt |
| Glutaminsynthetase | Glufosinat, Bialaphos |
| Adenylosuccinatlyase (ADSL) | Inhibitoren der IMP- und AMP-Synthese |
| Adenylosuccinatsynthase | Inhibitoren der Adenylosuccinatsynthese |
| Anthranilatsynthase | Inhibitoren der Tryptophansynthese und des |
| | -abbaus |
| Nitrilase | 3,5-Dihalogen-4-hydroxy-benzonitrile wie |
| | Bromoxynil und Loxinyl |
| 5-Enolpyruvyl-3-phosphoshikimat- | Glyphosat oder Sulfosat |
| Synthase (EPSPS) | |
| Glyphosat-Oxidoreductase | Glyphosat oder Sulfosat |
| Protoporphyrinogenoxidase (PROTOX) | Diphenylether, cyclische Imide, |
| | Phenylpyrazole, Pyridinderivate, |
| | Phenopylat, Oxadiazole usw. |
| Cytochrom P450 z.B. P450 SU1 oder Auswahl | Xenobiotika und Herbizide wie etwa |
| | Sulfonylharnstoffverbindungen |
| Polyphenoloxidase oder Polyphenol-Oxidase | Schwarzfleckigkeit |
| (Antisense) | |
| Metallothionein | Bakterielle und Pilz-Pathogene wie etwa |
| | Phytophtora |
| Ribonuclease | Phytophtora, Verticillium, Rhizoctonia |
| Pilzbekämpfendes Polypeptid AlyAFP | Bakterielle und Pilz-Pathogene wie etwa |
| | Phytophtora |
| Oxalatoxidase | Bakterielle und Pilz-Pathogene wie etwa |
| | Phytophtora, Verticillium, Rhizoctonia |
| Glucoseoxidase | Bakterielle und Pilz-Pathogene wie etwa |
| | Phytophtora, Verticillium, Rhizoctonia |
| Pyrrolnitrinsynthese-Gene | Bakterielle und Pilz-Pathogene wie etwa |
| | Phytophtora, Verticillium, Rhizoctonia |
| Serin/Threonin-Kinasen | Bakterielle und Pilz-Pathogene wie etwa |
| | Phytophtora, Verticillium, Rhizoctonia |
| Cecropin B | Bakterien wie etwa Corynebacterium |
| | sepedonicum, Erwinia carotovora |
| Phenylalanin-Ammoniak-Lyase (PAL) | Bakterielle und Pilz-Pathogene wie etwa |
| | Phytophtora, Verticillium, Rhizoctonia |
| Phytoalexine | Bakterielle und Pilz-Pathogene wie etwa |
| | Phytophtora, Verticillium, Rhizoctonia |
| B-1,3-glucanase (Antisense) | Bakterielle und Pilz-Pathogene wie etwa |
| | Phytophtora, Verticillium, Rhizoctonia |
| Rezeptorkinase | Bakterielle und Pilz-Pathogene wie etwa |
| | Phytophtora, Verticillium, Rhizoctonia |
| Polypeptid mit der Wirkung, eine Über- | Bakterielle und Pilz-Pathogene wie etwa |
| empfindlichkeitsreaktion auszulösen | Phytophtora, Verticillium, Rhizoctonia |
| Gene der systemischen erworbenen | Virale, bakterielle Pilz- und Nematoden- |
| Widerstandskraft (SAR) | Pathogene |
| Chitinasen | Bakterielle und Pilz-Pathogene wie etwa |
| | Phytophtora, Verticillium, Rhizoctonia |
| Barnase | Bakterielle und Pilz- Pathogene wie etwa |
| | Phytophtora, Verticillium, Rhizoctonia |
| Gen 49 zur Steuerung des Widerstandes gegen | Bakterielle und Pilz-Pathogene wie etwa |
| Krankheiten | Phytophtora, Verticillium, Rhizoctonia |
| Trans-Aldolase (Antisense) | Schwarzfleckigkeit |
| Glucanasen | Bakterielle und Pilz-Pathogene wie etwa |
| | Phytophtora, Verticillium, Rhizoctonia |
| Doppelstrang-Ribonuclease | Viren wie etwa PLRV, PVY und TRV |
| Hüllproteine | Viren wie etwa PLRV, PVY und TRV |
| 17kDa oder 60 kDa Protein | Viren wie etwa PLRV, PVY und TRV |
| Einschlussproteine des Kerns z.B. a oder b | Viren wie etwa PLRV, PVY und TRV |
| Pseudoubiquitin | Viren wie etwa PLRV, PVY und TRV |
| Replicase | Viren wie etwa PLRV, PVY und TRV |
| Toxine von Bacillus thuringiensis, VIP 3, | Coleoptera, z.B. Kartoffelkäfer, Blattläuse |
| Bacillus-cereus-Toxine, Photorabdus und | |
| Toxine von Xenorhabdus | |
| 3- Hydroxysteroidoxidase | Coleoptera, z.B. Kartoffelkäfer, Blattläuse |
| Peroxidase | Coleoptera, z.B. Kartoffelkäfer, Blattläuse |
| Aminopeptidase-Inhibitoren, z.B. Leucin | Coleoptera, z.B. Kartoffelkäfer, Blattläuse |
| Aminopeptidase-Inhibitor | |
| Stilbensynthase | Coleoptera, z.B. Kartoffelkäfer, Blattläuse |
| Lektine | Coleoptera, z.B. Kartoffelkäfer, Blattläuse |
| Protease-Inhibitoren, z.B. Cystatin, Patatin | Coleoptera, z.B. Kartoffelkäfer, Blattläuse |
| Ribosomeninaktivierendes Protein | Coleoptera, z.B. Kartoffelkäfer, Blattläuse |
| HMG-CoA-Reductase | Coleoptera, z.B. Kartoffelkäfer, Blattläuse |
| Schlüpfauslösefaktor für Zystennematoden | Zystennematoden |
| Barnase | Nematoden, z.B. Wurzelknoten-Nematoden und |
| | Zystennematoden |
| Prinzipien zur Verhinderung der Nahrungs- | Nematoden, z.B. Wurzelknoten-Nematoden und |
| aufnahme | Zystennematoden |

| Pflanze: Tomate | |
|---|---|
| Betroffene Struktur/ Exprimiertes Prinzip | Merkmal der Pflanze / Toleranz gegenüber |
| Acetolactatsynthase (ALS) | Sulfonylharnstoffverbindungen, Imidazolinone |
| | Triazolpyrimidine, |
| | Pyrimidyloxybenzoate, Phthalide |
| Acetyl-CoA-Carboxylase (ACCase) | Aryloxyphenoxyalkancarbonsäuren, |
| | Cyclohexandione |
| Hydroxyphenylpyruvat-Dioxygenase (HPPD) | Isoxazole wie etwa Isoxaflutol oder |
| | Isoxachlortol, Trione wie etwa |
| | Mesotrion oder Sulcotrion |
| Phosphinothricinacetyltransferase | Phosphinothricin |
| O-Methyltransferase | Geänderter Ligningehalt |
| Glutaminsynthetase | Glufosinat, Bialaphos |
| Adenylosuccinatlyase (ADSL) | Inhibitoren der IMP- und AMP-Synthese |
| Adenylosuccinatsynthase | Inhibitoren der Adenylosuccinatsynthese |
| Anthranilatsynthase | Inhibitoren der Tryptophansynthese und des |
| | -abbaus |
| Nitrilase | 3,5-Dihalogen-4-hydroxy-benzonitrile wie |
| | Bromoxynil und Loxinyl |
| 5-Enolpyruvyl-3-phosphoshikimat- | Glyphosat oder Sulfosat |
| Synthase (EPSPS) | |
| Glyphosatoxidoreductase | Glyphosat oder Sulfosat |
| Protoporphyrinogenoxidase (PROTOX) | Diphenylether, cyclische Imide, |
| | Phenylpyrazole, Pyridinderivate, |
| | Phenopylat, Oxadiazole usw. |
| Cytochrom P450 z.B. P450 SU1 oder Auswahl | Xenobiotika und Herbizide wie etwa |
| | Sulfonylharnstoffverbindungen |
| Polyphenoloxidase oder Polyphenol-Oxidase | Schwarzfleckigkeit |
| (Antisense) | |
| Metallothionein | Bakterielle und Pilz-Pathogene wie etwa |
| | Phytophtora |
| Ribonuclease | Phytophtora, Verticillium, Rhizoctonia |
| Pilzbekämpfendes Polypeptid AlyAFP | Bakterielle und Pilz-Pathogene wie etwa |
| | die bakterielle Fleckenkrankheit, Fusarium, |
| | Weichfäule, echter Mehltau, Krautfäule, |
| | Samtfleckenkrankheit etc. |
| Oxalatoxidase | Bakterielle und Pilz-Pathogene wie etwa |
| | die bakterielle Fleckenkrankheit, Fusarium, |
| | Weichfäule, echter Mehltau, Krautfäule, |
| | Samtfleckenkrankheit etc. |
| Glucoseoxidase | Bakterielle und Pilz-Pathogene wie etwa |
| | die bakterielle Fleckenkrankheit, Fusarium, |
| | Weichfäule, echter Mehltau, Krautfäule, |
| | Samtfleckenkrankheit etc. |
| Pyrrolnitrinsynthese-Gene | Bakterielle und Pilz-Pathogene wie etwa |
| | die bakterielle Fleckenkrankheit, Fusarium, |
| | Weichfäule, echter Mehltau, Krautfäule, |
| | Samtfleckenkrankheit etc. |
| Serin/Threonin-Kinasen | Bakterielle und Pilz-Pathogene wie etwa |
| | die bakterielle Fleckenkrankheit, Fusarium, |
| | Weichfäule, echter Mehltau, Krautfäule, |
| | Samtfleckenkrankheit etc. |
| Cecropin B | Bakterielle und Pilz-Pathogene wie etwa |
| | die bakterielle Fleckenkrankheit, Fusarium, |
| | Weichfäule, echter Mehltau, Krautfäule, |
| | Samtfleckenkrankheit etc. |
| Phenylalanin-Ammoniak-Lyase (PAL) | Bakterielle und Pilz-Pathogene wie etwa |
| | die bakterielle Fleckenkrankheit, Fusarium, |
| | Weichfäule, echter Mehltau, Krautfäule, |
| | Samtfleckenkrankheit etc. |
| Cf-Gene, z.B. Cf 9 Cf5 Cf4 Cf2 | Samtfleckenkrankheit |
| Osmotin | Dürrfleckenkrankheit |
| Alpha Hordothionin | Bakterien |
| Systemin | Bakterielle und Pilz-Pathogene wie etwa |
| | die bakterielle Fleckenkrankheit, Fusarium, |
| | Weichfäule, echter Mehltau, Krautfäule, |
| | Samtfleckenkrankheit etc. |
| Polygalacturonase-Inhibitoren | Bakterielle und Pilz-Pathogene wie etwa die |
| | bakterielle Fleckenkrankheit, Fusarium, |
| | Weichfäule, echter Mehltau, Krautfäule, |
| | Samtfleckenkrankheit etc. |
| Prf-Steuerungsgen | Bakterielle und Pilz-Pathogene wie etwa |
| | die bakterielle Fleckenkrankheit, Fusarium, |
| | Weichfäule, echter Mehltau, Krautfäule, |
| | Samtfleckenkrankheit etc. |
| 12 Fusariumresistenz-Stelle | Fusarium |
| Phytoalexine | Bakterielle und Pilz-Pathogene wie etwa |
| | die bakterielle Fleckenkrankheit, Fusarium, |
| | Weichfäule, echter Mehltau, Krautfäule, |
| | Samtfleckenkrankheit etc. |
| B-1,3-glucanase (Antisense) | Bakterielle und Pilz-Pathogene wie etwa |
| | die bakterielle Fleckenkrankheit, Fusarium, |
| | Weichfäule, echter Mehltau, Krautfäule, |
| | Samtfleckenkrankheit etc. |
| Rezeptorkinase | Bakterielle und Pilz-Pathogene wie etwa |
| | die bakterielle Fleckenkrankheit, Fusarium, |
| | Weichfäule, echter Mehltau, Krautfäule, |
| | Samtfleckenkrankheit etc. |
| Polypeptid mit der Wirkung, eine Über- | Bakterielle und Pilz-Pathogene wie etwa |
| empfindlichkeitsreaktion auszulösen | die bakterielle Fleckenkrankheit, Fusarium, |
| | Weichfäule, echter Mehltau, Krautfäule, |
| | Samtfleckenkrankheit etc. |
| Gene der systemischen erworbenen | Virale, bakterielle Pilz- und Nematoden- |
| Widerstandskraft (SAR) | Pathogene |
| Chitinasen | Bakterielle und Pilz-Pathogene wie etwa |
| | die bakterielle Fleckenkrankheit, Fusarium, |
| | Weichfäule, echter Mehltau, Krautfäule, |
| | Samtfleckenkrankheit etc. |
| Barnase | Bakterielle und Pilz-Pathogene wie etwa |
| | die bakterielle Fleckenkrankheit, Fusarium, |
| | Weichfäule, echter Mehltau, Krautfäule, |
| | Samtfleckenkrankheit etc. |
| Glucanasen | Bakterielle und Pilz-Pathogene wie etwa die |
| | bakterielle Fleckenkrankheit, Fusarium, |
| | Weichfäule, echter Mehltau, Krautfäule, |
| | Samtfleckenkrankheit etc. |
| Doppelstrang-Ribonuclease | Viren wie etwa PLRV, PVY und ToMoV |
| Hüllproteine | Viren wie etwa PLRV, PVY und ToMoV |
| 17kDa oder 60 kDa Protein | Viren wie etwa PLRV, PVY und ToMoV |
| Einschlussproteine des Kerns z.B. a oder b oder | Viren wie etwa PLRV, PVY und ToMoV |
| Nucleoprotein | TRV |
| Pseudoubiquitin | Viren wie etwa PLRV, PVY und ToMoV |
| Replicase | Viren wie etwa PLRV, PVY und ToMoV |
| Toxine von Bacillus thuringiensis, VIP 3, | Lepidoptera z.B. Heliothis, weiße Fliege |
| Bacillus-cereus-Toxine, Photorabdus und | Blattläuse |
| Toxine von Xenorhabdus | |
| 3- Hydroxysteroidoxidase | Lepidoptera z.B. Heliothis, weiße Fliege, |
| | Blattläuse |
| Peroxidase | Lepidoptera z.B. Heliothis, weiße Fliege, |
| | Blattläuse |
| Aminopeptidase-Inhibitoren, z.B. Leucin | Lepidoptera z.B. Heliothis, weiße Fliege, |
| Aminopeptidase-Inhibitor | Blattläuse |
| Lektine | Lepidoptera z.B. Heliothis, weiße Fliege, |
| | Blattläuse |
| Protease-Inhibitoren, z.B. Cystatin, Patatin | Lepidoptera z.B. Heliothis, weiße Fliege, |
| | Blattläuse |
| Ribosomeninaktivierendes Protein | Lepidoptera z.B. Heliothis, weiße Fliege, |
| | Blattläuse |
| Stilbensynthase | Lepidoptera z.B. Heliothis, weiße Fliege, |
| | Blattläuse |
| HMG-CoA-Reductase | Lepidoptera z.B. Heliothis, weiße Fliege, |
| | Blattläuse |
| Schlüpfauslösefaktor für Zystennematoden | Zystennematoden |
| Barnase | Nematoden, z.B. Wurzelknoten-Nematoden und |
| | Zystennematoden |
| Prinzipien zur Verhinderung der | Nematoden, z.B. Wurzelknoten-Nematoden und |
| Nahrungsaufnahme | Zystennematoden |

| Pflanze: Paprika | |
|---|---|
| Betroffene Struktur/ Exprimiertes Protein | Merkmal der Pflanze / Toleranz gegenüber |
| Acetolactatsynthase (ALS) | Sulfonylharnstoffverbindungen, Imidazolinone |
| | Triazolpyrimidine, |
| | Pyrimidyloxybenzoate, Phthalide |
| Acetyl-CoA-Carboxylase (ACCase) | Aryloxyphenoxyalkancarbonsäuren, |
| | Cyclohexandione |
| Hydroxyphenylpyruvat-Dioxygenase (HPPD) | Isoxazole wie etwa Isoxaflutol oder |
| | Isoxachlortol, Trione wie etwa |
| | Mesotrion oder Sulcotrion |
| Phosphinothricinacetyltransferase | Phosphinothricin |
| O-Methyltransferase | Geänderter Ligningehalt |
| Glutaminsynthetase | Glufosinat, Bialaphos |
| Adenylosuccinatlyase (ADSL) | Inhibitoren der IMP- und AMP-Synthese |
| Adenylosuccinatsynthase | Inhibitoren der Adenylosuccinatsynthese |
| Anthranilatsynthase | Inhibitoren der Tryptophansynthese und des |
| | -abbaus |
| Nitrilase | 3,5-Dihalogen-4-hydroxy-benzonitrile wie |
| | Bromoxynil und Loxinyl |
| 5-Enolpyruvyl-3-phosphoshikimat- | Glyphosat oder Sulfosat |
| Synthase (EPSPS) | |
| Glyphosatoxidoreductase | Glyphosat oder Sulfosat |
| Protoporphyrinogenoxidase (PROTOX) | Diphenylether, cyclische Imide, |
| | Phenylpyrazole, Pyridinderivate, |
| | Phenopylat, Oxadiazole usw. |
| Cytochrom P450 z.B. P450 SU1 oder Auswahl | Xenobiotika und Herbizide wie etwa |
| | Sulfonylharnstoffverbindungen |
| Polyphenoloxidase oder Polyphenol-Oxidase | Bakterielle und Pilz-Pathogene |
| (Antisense) | |
| Metallothionein | Bakterielle und Pilz-Pathogene |
| Ribonuclease | Bakterielle und Pilz-Pathogene |
| Pilzbekämpfendes Polypeptid AlyAFP | Bakterielle und Pilz-Pathogene |
| Oxalatoxidase | Bakterielle und Pilz-Pathogene |
| Glucoseoxidase | Bakterielle und Pilz-Pathogene |
| Pyrrolnitrinsynthese-Gene | Bakterielle und Pilz-Pathogene |
| Serin/Threonin-Kinasen | Bakterielle und Pilz-Pathogene |
| Cecropin B | Bakterielle und Pilz-Pathogene, Fäule, |
| | Samtfleckenkrankheit, etc |
| Phenylalanin-Ammoniak-Lyase (PAL) | Bakterielle und Pilz-Pathogene |
| Cf-Gene, z.B. Cf 9 Ct5 Cf4 Cf2 | Bakterielle und Pilz-Pathogene |
| Osmotin | Bakterielle und Pilz-Pathogene |
| Alpha Hordothionin | Bakterielle und Pilz-Pathogene |
| Systemin | Bakterielle und Pilz-Pathogene |
| Polygalacturonase-Inhibitoren | Bakterielle und Pilz-Pathogene |
| Prf-Steuerungsgen | Bakterielle und Pilz-Pathogene |
| 12 Fusariumresistenz-Stelle | Fusarium |
| Phytoalexine | Bakterielle und Pilz-Pathogene |
| B-1,3-glucanase (Antisense) | Bakterielle und Pilz-Pathogene |
| Rezeptorkinase | Bakterielle und Pilz-Pathogene |
| Polypeptid mit der Wirkung, eine Über- | Bakterielle und Pilz-Pathogene |
| empfindlichkeitsreaktion auszulösen | |
| Gene der systemischen erworbenen | Virale, bakterielle Pilz- und Nematoden- |
| Widerstandskraft (SAR) | Pathogene |
| Chitinasen | Bakterielle und Pilz-Pathogene |
| Barnase | Bakterielle und Pilz-Pathogene |
| Glucanasen | Bakterielle und Pilz-Pathogene |
| Doppelstrang-Ribonuclease | Viren wie etwa CMV, TEV |
| Hüllproteine | Viren wie etwa CMV, TEV |
| 17kDa oder 60 kDa Protein | Viren wie etwa CMV, TEV |
| Einschlussproteine des Kerns z.B. a oder b oder | Viren wie etwa CMV, TEV |
| Nucleoprotein | |
| Pseudoubiquitin | Viren wie etwa CMV, TEV |
| Replicase | Viren wie etwa CMV, TEV |
| Toxine von Bacillus thuringiensis, VIP 3, | Lepidoptera, weiße Fliege, Blattläuse |
| Bacillus-cereus-Toxine, Photorabdus und | |
| Toxine von Xenorhabdus | |
| 3- Hydroxysteroidoxidase | Lepidoptera, weiße Fliege, Blattläuse |
| Peroxidase | Lepidoptera, weiße Fliege, Blattläuse |
| Aminopeptidase-Inhibitoren, z.B. Leucin | Lepidoptera, weiße Fliege, Blattläuse |
| Aminopeptidase-Inhibitor | |
| Lektine | Lepidoptera, weiße Fliege, Blattläuse |
| Protease-Inhibitoren, z.B. Cystatin, Patatin | Lepidoptera, weiße Fliege, Blattläuse |
| Ribosomeninaktivierendes Protein | Lepidoptera, weiße Fliege, Blattläuse |
| Stilbensynthase | Lepidoptera, weiße Fliege, Blattläuse |
| HMG-CoA-Reductase | Lepidoptera, weiße Fliege, Blattläuse |
| Schlüpfauslösefaktor für Zystennematoden | Zystennematoden |
| Barnase | Nematoden, z.B. Wurzelknoten-Nematoden und |
| | Zystennematoden |
| Prinzipien zur Verhinderung der Nahrungs- | Nematoden, z.B. Wurzelknoten-Nematoden und |
| aufnahme | Zystennematoden |

| Pflanze: Wein | |
|---|---|
| Betroffene Struktur/ Exprimiertes Prinzip | Merkmal der Pflanze / Toleranz gegenüber |
| Acetolactatsynthase (ALS) | Sulfonylharnstoffverbindungen, Imidazolinone |
| | Triazolpyrimidine, |
| | Pyrimidyloxybenzoate, Phthalide |
| Acetyl-CoA-Carboxylase (ACCase) | Aryloxyphenoxyalkancarbonsäuren, |
| | Cyclohexandione |
| Hydroxyphenylpyruvat-Dioxygenase (HPPD) | Isoxazole wie etwa Isoxaflutol oder |
| | Isoxachlortol, Trione wie etwa |
| | Mesotrion oder Sulcotrion |
| Phosphinothricinacetyltransferase | Phosphinothricin |
| O-Methyltransferase | Geänderter Ligningehalt |
| Glutaminsynthetase | Glufosinat, Bialaphos |
| Adenylosuccinatlyase (ADSL) | Inhibitoren der IMP- und AMP-Synthese |
| Adenylosuccinatsynthase | Inhibitoren der Adenylosuccinatsynthese |
| Anthranilatsynthase | Inhibitoren der Tryptophansynthese und des |
| | -abbaus |
| Nitrilase | 3,5-Dihalogen-4-hydroxy-benzonitrile wie |
| | Bromoxynil und Loxinyl |
| 5-Enolpyruvyl-3-phosphoshikimat- | Glyphosat oder Sulfosat |
| Synthase (EPSPS) | |
| Glyphosatoxidoreductase | Glyphosat oder Sulfosat |
| Protoporphyrinogenoxidase (PROTOX) | Diphenylether, cyclische Imide, |
| | Phenylpyrazole, Pyridinderivate, |
| | Phenopylat, Oxadiazole usw. |
| Cytochrom P450 z.B. P450 SU1 oder Auswahl | Xenobiotika und Herbizide wie etwa |
| | Sulfonylharnstoffverbindungen |
| Polyphenoloxidase oder Polyphenol-Oxidase | Bakterielle und Pilz-Pathogene wie |
| (Antisense) | Botrytis und echter Mehltau |
| Metallothionein | Bakterielle und Pilz-Pathogene wie |
| | Botrytis und echter Mehltau |
| Ribonuclease | Bakterielle und Pilz-Pathogene wie |
| | Botrytis und echter Mehltau |
| Pilzbekämpfendes Polypeptid AlyAFP | Bakterielle und Pilz-Pathogene wie |
| | Botrytis und echter Mehltau |
| Oxalatoxidase | Bakterielle und Pilz-Pathogene wie |
| | Botrytis und echter Mehltau |
| Glucoseoxidase | Bakterielle und Pilz-Pathogene wie |
| | Botrytis und echter Mehltau |
| Pyrrolnitrinsynthese-Gene | Bakterielle und Pilz-Pathogene wie |
| | Botrytis und echter Mehltau |
| Serin/Threonin-Kinasen | Bakterielle und Pilz-Pathogene wie |
| | Botrytis und echter Mehltau |
| Cecropin B | Bakterielle und Pilz-Pathogene wie |
| | Botrytis und echter Mehltau |
| Phenylalanin-Ammoniak-Lyase (PAL) | Bakterielle und Pilz-Pathogene wie |
| | Botrytis und echter Mehltau |
| Cf-Gene, z.B. Cf 9 Cf5 Cf4 Cf2 | Bakterielle und Pilz-Pathogene wie |
| | Botrytis und echter Mehltau |
| Osmotin | Bakterielle und Pilz-Pathogene wie |
| | Botrytis und echter Mehltau |
| Alpha Hordothionin | Bakterielle und Pilz-Pathogene wie |
| | Botrytis und echter Mehltau |
| Systemin | Bakterielle und Pilz-Pathogene wie |
| | Botrytis und echter Mehltau |
| Polygalacturonase-Inhibitoren | Bakterielle und Pilz-Pathogene wie |
| | Botrytis und echter Mehltau |
| Prf-Steuerungsgen | Bakterielle und Pilz-Pathogene wie |
| | Botrytis und echter Mehltau |
| Phytoalexine | Bakterielle und Pilz-Pathogene wie |
| | Botrytis und echter Mehltau |
| B-1,3-glucanase (Antisense) | Bakterielle und Pilz-Pathogene wie |
| | Botrytis und echter Mehltau |
| Rezeptorkinase | Bakterielle und Pilz-Pathogene wie |
| | Botrytis und echter Mehltau |
| Polypeptid mit der Wirkung, eine Über- | Bakterielle und Pilz-Pathogene wie Botrytis und |
| empfindlichkeitsreaktion auszulösen | echter Mehltau |
| Gene der systemischen erworbenen | Virale, bakterielle Pilz- und Nematoden- |
| Widerstandskraft (SAR) | Pathogene |
| Chitinasen | Bakterielle und Pilz-Pathogene wie |
| | Botrytis und echter Mehltau |
| Barnase | Bakterielle und Pilz-Pathogene wie |
| | Botrytis und echter Mehltau |
| Glucanasen | Bakterielle und Pilz-Pathogene wie Botrytis |
| | und echter Mehltau |
| Doppelstrang-Ribonuclease | Viren |
| Hüllproteine | Viren |
| 17kDa oder 60 kDa Protein | Viren |
| Einschlussproteine des Kerns z.B. a oder b oder | Viren |
| Nucleoprotein | |
| Pseudoubiquitin | Viren |
| Replicase | Viren |
| Toxine von Bacillus thuringiensis, VIP 3, | Lepidoptera, Blattläuse |
| Bacillus-cereus-Toxine, Photorabdus und | |
| Toxine von Xenorhabdus | |
| 3- Hydroxysteroidoxidase | Lepidoptera, Blattläuse |
| Peroxidase | Lepidoptera, Blattläuse |
| Aminopeptidase-Inhibitoren, z.B. Leucin | Lepidoptera, Blattläuse |
| Aminopeptidase-Inhibitor | |
| Lektine | Lepidoptera, Blattläuse |
| Protease-Inhibitoren, z.B. Cystatin, Patatin | Lepidoptera, Blattläuse |
| Ribosomeninaktivierendes Protein | Lepidoptera, Blattläuse |
| Stilbensynthase | Lepidoptera, Blattläuse, Krankheiten |
| HMG-CoA-Reductase | Lepidoptera, Blattläuse |
| Schlüpfauslösefaktor für Zystennematoden | Zystennematoden |
| Barnase | Nematoden, z.B. Wurzelknoten-Nematoden und |
| | Zystennematoden oder allgemeine Krankheiten |
| CBI | Wurzelknotennematoden |
| Prinzipien zur Verhinderung der Nahrungs- | Nematoden, z.B. Wurzelknoten-Nematoden |
| aufnahme | oder Wurzelzystennematoden |

| Pflanze: Ölraps | |
|---|---|
| Betroffene Struktur/ Exprimiertes Protein | Merkmal der Pflanze / Toleranz gegenüber |
| Acetolactatsynthase (ALS) | Sulfonylharnstoffverbindungen, Imidazolinone |
| | Triazolpyrimidine, |
| | Pyrimidyloxybenzoate, Phthalide |
| Acetyl-CoA-Carboxylase (ACCase) | Aryloxyphenoxyalkancarbonsäuren, |
| | Cyclohexandione |
| Hydroxyphenylpyruvat-Dioxygenase (HPPD) | Isoxazole wie etwa Isoxaflutol oder |
| | Isoxachlortol, Trione wie etwa |
| | Mesotrion oder Sulcotrion |
| Phosphinothricinacetyltransferase | Phosphinothricin |
| O-Methyltransferase | Geänderter Ligningehalt |
| Glutaminsynthetase | Glufosinat, Bialaphos |
| Adenylosuccinatlyase (ADSL) | Inhibitoren der IMP- und AMP-Synthese |
| Adenylosuccinatsynthase | Inhibitoren der Adenylosuccinatsynthese |
| Anthranilatsynthase | Inhibitoren der Tryptophansynthese und des |
| | -abbaus |
| Nitrilase | 3,5-Dihalogen-4-hydroxy-benzonitrile wie |
| | Bromoxynil und Loxinyl |
| 5-Enolpyruvyl-3-phosphoshikimat- | Glyphosat oder Sulfosat |
| Synthase (EPSPS) | |
| Glyphosatoxidoreductase | Glyphosat oder Sulfosat |
| Protoporphyrinogenoxidase (PROTOX) | Diphenylether, cyclische Imide, |
| | Phenylpyrazole, Pyridinderivate, |
| | Phenopylat, Oxadiazole usw. |
| Cytochrom P450 z.B. P450 SU1 oder Auswahl | Xenobiotika und Herbizide wie etwa |
| | Sulfonylharnstoffverbindungen |
| Polyphenoloxidase oder Polyphenol- | Bakterielle und Pilz-Pathogene wie |
| Oxidase (Antisense) | Cylindrosporium, Phoma, Sklerotinia |
| Metallothionein | Bakterielle und Pilz-Pathogene wie |
| | Cylindrosporium, Phoma, Sklerotinia |
| Ribonuclease | Bakterielle und Pilz-Pathogene wie |
| | Cylindrosporium, Phoma, Sklerotinia |
| Pilzbekämpfendes Polypeptid AlyAFP | Bakterielle und Pilz-Pathogene wie |
| | Cylindrosporium, Phoma, Sklerotinia |
| Oxalatoxidase | Bakterielle und Pilz-Pathogene wie |
| | Cylindrosporium, Phoma, Sklerotinia |
| Glucoseoxidase | Bakterielle und Pilz-Pathogene wie |
| | Cylindrosporium, Phoma, Sklerotinia |
| Pyrrolnitrinsynthese-Gene | Bakterielle und Pilz-Pathogene wie |
| | Cylindrosporium, Phoma, Sklerotinia |
| Serin/Threonin-Kinasen | Bakterielle und Pilz-Pathogene wie |
| | Cylindrosporium, Phoma, Sklerotinia |
| Cecropin B | Bakterielle und Pilz-Pathogene wie |
| | Cylindrosporium, Phoma, Sklerotinia |
| Phenylalanin-Ammoniak-Lyase (PAL) | Bakterielle und Pilz-Pathogene wie |
| | Cylindrosporium, Phoma, Sklerotinia |
| Cf-Gene, z.B. Cf 9 Cf5 Cf4 Cf2 | Bakterielle und Pilz-Pathogene wie |
| | Cylindrosporium, Phoma, Sklerotinia |
| Osmotin | Bakterielle und Pilz-Pathogene wie |
| | Cylindrosporium, Phoma, Sklerotinia |
| Alpha Hordothionin | Bakterielle und Pilz-Pathogene wie |
| | Cylindrosporium, Phoma, Sklerotinia |
| Systemin | Bakterielle und Pilz-Pathogene wie |
| | Cylindrosporium, Phoma, Sklerotinia |
| Polygalacturonase-Inhibitoren | Bakterielle und Pilz-Pathogene wie |
| | Cylindrosporium, Phoma, Sklerotinia |
| Prf Steuerungsgen | Bakterielle und Pilz-Pathogene wie |
| | Cylindrosporium, Phoma, Sklerotinia |
| Phytoalexine | Bakterielle und Pilz-Pathogene wie |
| | Cylindrosporium, Phoma, Sklerotinia |
| B-1,3-glucanase (Antisense) | Bakterielle und Pilz-Pathogene wie |
| | Cylindrosporium, Phoma, Sklerotinia |
| Rezeptorkinase | Bakterielle und Pilz-Pathogene wie |
| | Cylindrosporium, Phoma, Sklerotinia |
| Polypeptid mit der Wirkung, eine Über- | Bakterielle und Pilz-Pathogene wie |
| empfindlichkeitsreaktion auszulösen | Cylindrosporium, Phoma, Sklerotinia |
| Gene der systemischen erworbenen | Virale, bakterielle Pilz- und Nematoden- |
| Widerstandskraft (SAR) | Pathogene |
| Chitinasen | Bakterielle und Pilz-Pathogene wie |
| | Cylindrosporium, Phoma, Sklerotinia |
| Barnase | Bakterielle und Pilz-Pathogene wie |
| | Cylindrosporium, Phoma, Sklerotinia, |
| | Nematoden |
| Glucanasen | Bakterielle und Pilz-Pathogene wie |
| | Cylindrosporium, Phoma, Sklerotinia |
| Doppelstrang-Ribonuclease | Viren |
| Hüllproteine | Viren |
| 17kDa oder 60 kDa Protein | Viren |
| Einschlussproteine des Kerns z.B. a oder b oder | Viren |
| Nucleoprotein | |
| Pseudoubiquitin | Viren |
| Replicase | Viren |
| Toxine von Bacillus thuringiensis, VIP 3, | Lepidoptera, Blattläuse |
| Bacillus-cereus-Toxine, Photorabdus und | |
| Toxine von Xenorhabdus | |
| 3- Hydroxysteroidoxidase | Lepidoptera, Blattläuse |
| Peroxidase | Lepidoptera, Blattläuse |
| Aminopeptidase-Inhibitoren, z.B. Leucin | Lepidoptera, Blattläuse |
| Aminopeptidase-Inhibitor | |
| Lektine | Lepidoptera, Blattläuse |
| Protease-Inhibitoren, z.B. Cystatin, Patatin, | Lepidoptera, Blattläuse |
| CPTI | |
| Ribosomeninaktivierendes Protein | Lepidoptera, Blattläuse |
| Stilbensynthase | Lepidoptera, Blattläuse, Krankheiten |
| HMG-CoA-Reductase | Lepidoptera, Blattläuse |
| Schlüpfauslösefaktor für Zystennematoden | Zystennematoden |
| Barnase | Nematoden, z.B. Wurzelknoten-Nematoden und |
| | Zystennematoden |
| CBI | Wurzelknotennematoden |
| Prinzipien zur Verhinderung der Nahrungsauf- | Nematoden, z.B. Wurzelknoten-Nematoden und |
| nahmen, die an Nematoden-Nährstellen | Wurzel- Zystennematoden |
| induziert werden | |

| Pflanze: Brassica-Gemüse (Kohl, Kohlsprossen etc..) | |
|---|---|
| Betroffene Struktur/ Exprimiertes Protein | Merkmal der Pflanze / Toleranz gegenüber |
| Acetolactatsynthase (ALS) | Sulfonylharnstoffverbindungen, Imidazolinone |
| | Triazolpyrimidine, |
| | Pyrimidyloxybenzoate, Phthalide |
| Acetyl-CoA-Carboxylase (ACCase) | Aryloxyphenoxyalkancarbonsäuren, |
| | Cyclohexandione |
| Hydroxyphenylpyruvat-Dioxygenase (HPPD) | Isoxazole wie etwa Isoxaflutol oder |
| | Isoxachlortol, Trione wie etwa |
| | Mesotrion oder Sulcotrion |
| Phosphinothricinacetyltransferase | Phosphinothricin |
| O-Methyltransferase | Geänderter Ligningehalt |
| Glutaminsynthetase | Glufosinat, Bialaphos |
| Adenylosuccinatlyase (ADSL) | Inhibitoren der IMP- und AMP-Synthese |
| Adenylosuccinatsynthase | Inhibitoren der Adenylosuccinatsynthese |
| Anthranilatsynthase | Inhibitoren der Tryptophansynthese und des |
| | -abbaus |
| Nitrilase | 3,5-Dihalogen-4-hydroxy-benzonitrile wie |
| | Bromoxynil und Loxinyl |
| 5-Enolpyruvyl-3-phosphoshikimat- | Glyphosat oder Sulfosat |
| Synthase (EPSPS) | |
| Glyphosatoxidoreductase | Glyphosat oder Sulfosat |
| Protoporphyrinogenoxidase (PROTOX) | Diphenylether, cyclische Imide, |
| | Phenylpyrazole, Pyridinderivate, |
| | Phenopylat, Oxadiazole usw. |
| Cytochrom P450 z.B. P450 SU1 oder Auswahl | Xenobiotika und Herbizide wie etwa |
| | Sulfonylharnstoffverbindungen |
| Polyphenoloxidase oder Polyphenol- Oxidase | Bakterielle und Pilz-Pathogene |
| (Antisense) | |
| Metallothionein | Bakterielle und Pilz-Pathogene |
| Ribonuclease | Bakterielle und Pilz-Pathogene |
| Pilzbekämpfendes Polypeptid AlyAFP | Bakterielle und Pilz-Pathogene |
| Oxalatoxidase | Bakterielle und Pilz-Pathogene |
| Glucoseoxidase | Bakterielle und Pilz-Pathogene |
| Pyrrolnitrinsynthese-Gene | Bakterielle und Pilz-Pathogene |
| Serin/Threonin-Kinasen | Bakterielle und Pilz-Pathogene |
| Cecropin B | Bakterielle und Pilz-Pathogene |
| Phenylalanin-Ammoniak-Lyase (PAL) | Bakterielle und Pilz-Pathogene |
| Cf-Gene, z.B. Cf 9 Cf5 Cf4 Cf2 | Bakterielle und Pilz-Pathogene |
| Osmotin | Bakterielle und Pilz-Pathogene |
| Alpha Hordothionin | Bakterielle und Pilz-Pathogene |
| Systemin | Bakterielle und Pilz-Pathogene |
| Polygalacturonase-Inhibitoren | Bakterielle und Pilz-Pathogene |
| Prf-Steuerungsgen | Bakterielle und Pilz-Pathogene |
| Phytoalexine | Bakterielle und Pilz-Pathogene |
| B-1,3-glucanase (Antisense) | Bakterielle und Pilz-Pathogene |
| Rezeptorkinase | Bakterielle und Pilz-Pathogene |
| Polypeptid mit der Wirkung, eine Über- | Bakterielle und Pilz-Pathogene |
| empfindlichkeitsreaktion auszulösen | |
| Gene der systemischen erworbenen | Virale, bakterielle Pilz- und Nematoden- |
| Widerstandskraft (SAR) | Pathogene |
| Chitinasen | Bakterielle und Pilz-Pathogene |
| Barnase | Bakterielle und Pilz-Pathogene |
| Glucanasen | Bakterielle und Pilz-Pathogene |
| Doppelstrang-Ribonuclease | Viren |
| Hüllproteine | Viren |
| 17kDa oder 60 kDa Protein | Viren |
| Einschlussproteine des Kerns z.B. a oder b oder | Viren |
| Nucleoprotein | |
| Pseudoubiquitin | Viren |
| Replicase | Viren |
| Toxine von Bacillus thuringiensis, VIP 3, | Lepidoptera, Blattläuse |
| Bacillus-cereus-Toxine, Photorabdus und | |
| Toxine von Xenorhabdus | |
| 3- Hydroxysteroidoxidase | Lepidoptera, Blattläuse |
| Peroxidase | Lepidoptera, Blattläuse |
| Aminopeptidase-Inhibitoren, z.B. Leucin | Lepidoptera, Blattläuse |
| Aminopeptidase-Inhibitor | |
| Lektine | Lepidoptera, Blattläuse |
| Protease-Inhibitoren, z.B. Cystatin, Patatin, | Lepidoptera, Blattläuse |
| CPTI | |
| Ribosomeninaktivierendes Protein | Lepidoptera, Blattläuse |
| Stilbensynthase | Lepidoptera, Blattläuse, Krankheiten |
| HMG-CoA-Reductase | Lepidoptera, Blattläuse |
| Schlüpfauslösefaktor für Zystennematoden | Zystennematoden |
| Barnase | Nematoden, z.B. Wurzelknoten-Nematoden und |
| | Zystennematoden |
| CBI | Wurzelknotennematoden |
| Prinzipien zur Verhinderung der Nahrungs- | Nematoden, z.B. Wurzelknoten-Nematoden und |
| aufnahmen, die an Nematoden-Nährstellen | Wurzel-Zystennematoden |
| induziert werden | Zystennematoden |

| Pflanzen: Kernobst z.B. Äpfel, Birnen | |
|---|---|
| Betroffene Struktur/ Exprimiertes Protein | Merkmal der Pflanze / Toleranz gegenüber |
| Acetolactatsynthase (ALS) | Sulfonylharnstoffverbindungen, Imidazolinone |
| | Triazolpyrimidine, |
| | Pyrimidyloxybenzoate, Phthalide |
| Acetyl-CoA-Carboxylase (ACCase) | Aryloxyphenoxyalkancarbonsäuren, |
| | Cyclohexandione |
| Hydroxyphenylpyruvat-Dioxygenase (HPPD) | Isoxazole wie etwa Isoxaflutol oder |
| | Isoxachlortol, Trione wie etwa |
| | Mesotrion oder Sulcotrion |
| Phosphinothricinacetyltransferase | Phosphinothricin |
| O-Methyltransferase | Geänderter Ligningehalt |
| Glutaminsynthetase | Glufosinat, Bialaphos |
| Adenylosuccinatlyase (ADSL) | Inhibitoren der IMP- und AMP-Synthese |
| Adenylosuccinatsynthase | Inhibitoren der Adenylosuccinatsynthese |
| Anthranilatsynthase | Inhibitoren der Tryptophansynthese und des |
| | -abbaus |
| Nitrilase | 3,5-Dihalogen-4-hydroxy-benzonitrile wie |
| | Bromoxynil und Loxinyl |
| 5-Enolpyruvyl-3-phosphoshikimat- | Glyphosat oder Sulfosat |
| Synthase (EPSPS) | |
| Glyphosatoxidoreductase | Glyphosat oder Sulfosat |
| Protoporphyrinogenoxidase (PROTOX) | Diphenylether, cyclische Imide, |
| | Phenylpyrazole, Pyridinderivate, |
| | Phenopylat, Oxadiazole usw. |
| Cytochrom P450 z.B. P450 SU1 oder Auswahl | Xenobiotika und Herbizide wie etwa |
| | Sulfonylharnstoffverbindungen |
| Polyphenoloxidase oder Polyphenol-Oxidase | Bakterielle und Pilz-Pathogene wie |
| (Antisense) | Lagerschorf an Äpfeln oder Feuerbrand |
| Metallothionein | Bakterielle und Pilz-Pathogene wie |
| | Lagerschorf an Äpfeln oder Feuerbrand |
| Ribonuclease | Bakterielle und Pilz-Pathogene wie |
| | Lagerschorf an Äpfeln oder Feuerbrand |
| Pilzbekämpfendes Polypeptid AlyAFP | Bakterielle und Pilz-Pathogene wie |
| | Lagerschorf an Äpfeln oder Feuerbrand |
| Oxalatoxidase | Bakterielle und Pilz-Pathogene wie |
| | Lagerschorf an Äpfeln oder Feuerbrand |
| Glucoseoxidase | Bakterielle und Pilz-Pathogene wie |
| | Lagerschorf an Äpfeln oder Feuerbrand |
| Pyrrolnitrinsynthese-Gene | Bakterielle und Pilz-Pathogene wie |
| | Lagerschorf an Äpfeln oder Feuerbrand |
| Serin/Threonin-Kinasen | Bakterielle und Pilz-Pathogene wie |
| | Lagerschorf an Äpfeln oder Feuerbrand |
| Cecropin B | Bakterielle und Pilz-Pathogene wie |
| | Lagerschorf an Äpfeln oder Feuerbrand |
| Phenylalanin-Ammoniak-Lyase (PAL) | Bakterielle und Pilz-Pathogene wie |
| | Lagerschorf an Äpfeln oder Feuerbrand |
| Cf-Gene, z.B. Cf 9 Cf5 Cf4 Cf2 | Bakterielle und Pilz-Pathogene wie |
| | Lagerschorf an Äpfeln oder Feuerbrand |
| Osmotin | Bakterielle und Pilz-Pathogene wie |
| | Lagerschorf an Äpfeln oder Feuerbrand |
| Alpha Hordothionin | Bakterielle und Pilz-Pathogene wie |
| | Lagerschorf an Äpfeln oder Feuerbrand |
| Systemin | Bakterielle und Pilz-Pathogene wie |
| | Lagerschorf an Äpfeln oder Feuerbrand |
| Polygalacturonase-Inhibitoren | Bakterielle und Pilz-Pathogene wie |
| | Lagerschorf an Äpfeln oder Feuerbrand |
| Prf-Steuerungsgen | Bakterielle und Pilz-Pathogene wie |
| | Lagerschorf an Äpfeln oder Feuerbrand |
| Phytoalexine | Bakterielle und Pilz-Pathogene wie |
| | Lagerschorf an Äpfeln oder Feuerbrand |
| B-1,3-glucanase (Antisense) | Bakterielle und Pilz-Pathogene wie |
| | Lagerschorf an Äpfeln oder Feuerbrand |
| Rezeptorkinase | Bakterielle und Pilz-Pathogene wie |
| | Lagerschorf an Äpfeln oder Feuerbrand |
| Polypeptid mit der Wirkung, eine Über- | Bakterielle und Pilz-Pathogene wie Lager- |
| empfindlichkeitsreaktion auszulösen | schorf an Äpfeln oder Feuerbrand |
| Gene der systemischen erworbenen | Virale, bakterielle Pilz- und Nematoden- |
| Widerstandskraft (SAR) | Pathogene |
| lytisch wirkendes Protein | Bakterielle und Pilz-Pathogene wie |
| | Lagerschorf an Äpfeln oder Feuerbrand |
| Lysozym | Bakterielle und Pilz-Pathogene wie |
| | Lagerschorf an Äpfeln oder Feuerbrand |
| Chitinasen | Bakterielle und Pilz-Pathogene wie |
| | Lagerschorf an Äpfeln oder Feuerbrand |
| Barnase | Bakterielle und Pilz-Pathogene wie |
| | Lagerschorf an Äpfeln oder Feuerbrand |
| Glucanasen | Bakterielle und Pilz-Pathogene wie |
| | Lagerschorf an Äpfeln oder Feuerbrand |
| Doppelstrang-Ribonuclease | Viren |
| Hüllproteine | Viren |
| 17kDa oder 60 kDa Protein | Viren |
| Einschlussproteine des Kerns z.B. a oder b oder | Viren |
| Nucleoprotein | |
| Pseudoubiquitin | Viren |
| Replicase | Viren |
| Toxine von Bacillus thuringiensis, VIP 3, | Lepidoptera, Blattläuse, Milben |
| Bacillus-cereus-Toxine, Photorabdus und | |
| Toxine von Xenorhabdus | |
| 3- Hydroxysteroidoxidase | Lepidoptera, Blattläuse, Milben |
| Peroxidase | Lepidoptera, Blattläuse, Milben |
| Aminopeptidase-Inhibitoren, z.B. Leucin | Lepidoptera, Blattläuse, Milben |
| Aminopeptidase-Inhibitor | |
| Lektine | Lepidoptera, Blattläuse, Milben |
| Protease-Inhibitoren, z.B. Cystatin, Patatin, | Lepidoptera, Blattläuse, Milben |
| CPTI | |
| Ribosomeninaktivierendes Protein | Lepidoptera, Blattläuse, Milben |
| Stilbensynthase | Lepidoptera, Blattläuse, Krankheiten, Milben |
| HMG-CoA-Reductase | Lepidoptera, Blattläuse, Milben |
| Schlüpfauslösefaktor für Zystennematoden | Zystennematoden |
| Barnase | Nematoden, z.B. Wurzelknoten-Nematoden und |
| | Zystennematoden |
| CBI | Wurzelknotennematoden |
| Prinzipien zur Verhinderung der Nahrungs- | Nematoden, z.B. Wurzelknoten-Nematoden und |
| aufnahmen, die an Nematoden-Nährstellen | Wurzel-Zystennematoden |
| induziert werden | |

| Pflanze: Melone | |
|---|---|
| Betroffene Struktur/ Exprimiertes Protein | Merkmal der Pflanze / Toleranz gegenüber |
| Acetolactatsynthase (ALS) | Sulfonylharnstoffverbindungen, Imidazolinone |
| | Triazolpyrimidine, |
| | Pyrimidyloxybenzoate, Phthalide |
| Acetyl-CoA-Carboxylase (ACCase) | Aryloxyphenoxyalkancarbonsäuren, |
| | Cyclohexandione |
| Hydroxyphenylpyruvat-Dioxygenase (HPPD) | Isoxazole wie etwa Isoxaflutol oder |
| | Isoxachlortol, Trione wie etwa |
| | Mesotrion oder Sulcotrion |
| Phosphinothricinacetyltransferase | Phosphinothricin |
| O-Methyltransferase | Geänderter Ligningehalt |
| Glutaminsynthetase | Glufosinat, Bialaphos |
| Adenylosuccinatlyase (ADSL) | Inhibitoren der IMP- und AMP-Synthese |
| Adenylosuccinatsynthase | Inhibitoren der Adenylosuccinatsynthese |
| Anthranilatsynthase | Inhibitoren der Tryptophansynthese und des |
| | -abbaus |
| Nitrilase | 3,5-Dihalogen-4-hydroxy-benzonitrile wie |
| | Bromoxynil und Loxinyl |
| 5-Enolpyruvyl-3-phosphoshikimat- | Glyphosat oder Sulfosat |
| Synthase (EPSPS) | |
| Glyphosatoxidoreductase | Glyphosat oder Sulfosat |
| Protoporphyrinogenoxidase (PROTOX) | Diphenylether, cyclische Imide, |
| | Phenylpyrazole, Pyridinderivate, |
| | Phenopylat, Oxadiazole usw. |
| Cytochrom P450 z.B. P450 SU1 oder Auswahl | Xenobiotika und Herbizide wie etwa |
| | Sulfonylharnstoffverbindungen |
| Polyphenoloxidase oder Polyphenol-Oxidase | Bakterielle oder Pilz-Pathogene wie |
| (Antisense) | Phytophtora |
| Metallothionein | Bakterielle oder Pilz-Pathogene wie |
| | Phytophtora |
| Ribonuclease | Bakterielle oder Pilz-Pathogene wie |
| | Phytophtora |
| Pilzbekämpfendes Polypeptid AlyAFP | Bakterielle oder Pilz-Pathogene wie |
| | Phytophtora |
| Oxalatoxidase | Bakterielle oder Pilz-Pathogene wie |
| | Phytophtora |
| Glucoseoxidase | Bakterielle oder Pilz-Pathogene wie |
| | Phytophtora |
| Pyrrolnitrinsynthesegene | Bakterielle oder Pilz-Pathogene wie |
| | Phytophtora |
| Serin/Threonin-Kinasen | Bakterielle oder Pilz-Pathogene wie |
| | Phytophtora |
| Cecropin B | Bakterielle oder Pilz-Pathogene wie |
| | Phytophtora |
| Phenylalanin-Ammoniak-Lyase (PAL) | Bakterielle oder Pilz-Pathogene wie |
| | Phytophtora |
| Cf-Gene, z.B. Cf 9 Cf5 Cf4 Cf2 | Bakterielle oder Pilz-Pathogene wie |
| | Phytophtora |
| Osmotin | Bakterielle oder Pilz-Pathogene wie |
| | Phytophtora |
| Alpha Hordothionin | Bakterielle oder Pilz-Pathogene wie |
| | Phytophtora |
| Systemin | Bakterielle oder Pilz-Pathogene wie |
| | Phytophtora |
| Polygalacturonase-Inhibitoren | Bakterielle oder Pilz-Pathogene wie |
| | Phytophtora |
| Prf-Steuerungsgen | Bakterielle oder Pilz-Pathogene wie |
| | Phytophtora |
| Phytoalexine | Bakterielle oder Pilz-Pathogene wie |
| | Phytophtora |
| B-1,3-glucanase (Antisense) | Bakterielle oder Pilz-Pathogene wie |
| | Phytophtora |
| Rezeptorkinase | Bakterielle oder Pilz-Pathogene wie |
| | Phytophtora |
| Polypeptid mit der Wirkung, eine Über- | Bakterielle oder Pilz-Pathogene wie |
| empfindlichkeitsreaktion auszulösen | Phytophtora |
| Gene der systemischen erworbenen | Virale, bakterielle Pilz- und Nematoden- |
| Widerstandskraft (SAR) | Pathogene |
| Lytisch wirkendes Protein | Bakterielle oder Pilz-Pathogene wie |
| | Phytophtora |
| Lysozym | Bakterielle oder Pilz-Pathogene wie |
| | Phytophtora |
| Chitinasen | Bakterielle oder Pilz-Pathogene wie |
| | Phytophtora |
| Barnase | Bakterielle oder Pilz-Pathogene wie |
| | Phytophtora |
| Glucanasen | Bakterielle oder Pilz-Pathogene wie |
| | Phytophtora |
| Doppelstrang-Ribonuclease | Viren wie CMV, PRSV, WMV2, SMV, |
| | ZYMV |
| Hüllproteine | Viren wie CMV, PRSV, WMV2, SMV, |
| | ZYMV |
| 17kDa oder 60 kDa Protein | Viren wie CMV, PRSV, WMV2, SMV, |
| | ZYMV |
| Einschlussproteine des Kerns z.B. a oder b oder | Viren wie CMV, PRSV, WMV2, SMV, |
| Nucleoprotein | ZYMV |
| Pseudoubiquitin | Viren wie CMV, PRSV, WMV2, SMV, |
| | ZYMV |
| Replicase | Viren wie CMV, PRSV, WMV2, SMV, |
| | ZYMV |
| Toxine von Bacillus thuringiensis, VIP 3, | Lepidoptera, Blattläuse, Milben |
| Bacillus-cereus-Toxine, Photorabdus und | |
| Toxine von Xenorhabdus | |
| 3- Hydroxysteroidoxidase | Lepidoptera, Blattläuse, Milben, weiße Fliege |
| Peroxidase | Lepidoptera, Blattläuse, Milben, weiße Fliege |
| Aminopeptidase-Inhibitoren, z.B. Leucin | Lepidoptera, Blattläuse, Milben, weiße Fliege |
| Aminopeptidase-Inhibitor | |
| Lektine | Lepidoptera, Blattläuse, Milben, weiße Fliege |
| Protease-Inhibitoren, z.B. Cystatin, Patatin, | Lepidoptera, Blattläuse, Milben, weiße Fliege |
| CPTI, Virgiferin | |
| Ribosomeninaktivierendes Protein | Lepidoptera, Blattläuse, Milben, weiße Fliege |
| Stilbensynthase | Lepidoptera, Blattläuse, Milben, weiße Fliege |
| HMG-CoA-Reductase | Lepidoptera, Blattläuse, Milben, weiße Fliege |
| Schlüpfauslösefaktor für Zystennematoden | Zystennematoden |
| Barnase | Nematoden, z.B. Wurzelknoten-Nematoden und |
| | Zystennematoden |
| CBI | Wurzelknotennematoden |
| Prinzipien zur Verhinderung der Nahrungs- | Nematoden, z.B. Wurzelknoten-Nematoden und |
| aufnahmen, die an Nematoden-Nährstellen | Wurzel-Zystennematoden |
| induziert werden | |

| Pflanze: Banane | |
|---|---|
| Betroffene Struktur/ Exprimiertes Protein | Merkmal der Pflanze / Toleranz gegenüber |
| Acetolactatsynthase (ALS) | Sulfonylharnstoffverbindungen, Imidazolinone |
| | Triazolpyrimidine, |
| | Pyrimidyloxybenzoate, Phthalide |
| Acetyl-CoA-Carboxylase (ACCase) | Aryloxyphenoxyalkancarbonsäuren, |
| | Cyclohexandione |
| Hydroxyphenylpyruvat-Dioxygenase (HPPD) | Isoxazole wie etwa Isoxaflutol oder |
| | Isoxachlortol, Trione wie etwa |
| | Mesotrion oder Sulcotrion |
| Phosphinothricinacetyltransferase | Phosphinothricin |
| O-Methyltransferase | Geänderter Ligningehalt |
| Glutaminsynthetase | Glufosinat, Bialaphos |
| Adenylosuccinatlyase (ADSL) | Inhibitoren der IMP- und AMP-Synthese |
| Adenylosuccinatsynthase | Inhibitoren der Adenylosuccinatsynthese |
| Anthranilatsynthase | Inhibitoren der Tryptophansynthese und des |
| | -abbaus |
| Nitrilase | 3,5-Dihalogen-4-hydroxy-benzonitrile wie |
| | Bromoxynil und Loxinyl |
| 5-Enolpyruvyl-3-phosphoshikimat- | Glyphosat oder Sulfosat |
| Synthase (EPSPS) | |
| Glyphosatoxidoreductase | Glyphosat oder Sulfosat |
| Protoporphyrinogenoxidase (PROTOX) | Diphenylether, cyclische Imide, |
| | Phenylpyrazole, Pyridinderivate, |
| | Phenopylat, Oxadiazole usw. |
| Cytochrom P450 z.B. P450 SU1 oder Auswahl | Xenobiotika und Herbizide wie etwa |
| | Sulfonylharnstoffverbindungen |
| Polyphenoloxidase oder Polyphenol-Oxidase | Bakterielle oder Pilz-Pathogene |
| (Antisense) | |
| Metallothionein | Bakterielle oder Pilz-Pathogene |
| Ribonuclease | Bakterielle oder Pilz-Pathogene |
| Pilzbekämpfendes Polypeptid AlyAFP | Bakterielle oder Pilz-Pathogene |
| Oxalatoxidase | Bakterielle oder Pilz-Pathogene |
| Glucoseoxidase | Bakterielle oder Pilz-Pathogene |
| Pyrrolnitrinsynthese-Gene | Bakterielle oder Pilz-Pathogene |
| Serin/Threonin-Kinasen | Bakterielle oder Pilz-Pathogene |
| Cecropin B | Bakterielle oder Pilz-Pathogene |
| Phenylalanin-Ammoniak-Lyase (PAL) | Bakterielle oder Pilz-Pathogene |
| Cf-Gene, z.B. Cf 9 Cf5 Cf4 Cf2 | Bakterielle oder Pilz-Pathogene |
| Osmotin | Bakterielle oder Pilz-Pathogene |
| Alpha Hordothionin | Bakterielle oder Pilz-Pathogene |
| Systemin | Bakterielle oder Pilz-Pathogene |
| Polygalacturonase-Inhibitoren | Bakterielle oder Pilz-Pathogene |
| Prf-Steuerungsgen | Bakterielle oder Pilz-Pathogene |
| Phytoalexine | Bakterielle oder Pilz-Pathogene |
| B-1,3-glucanase (Antisense) | Bakterielle oder Pilz-Pathogene |
| Rezeptorkinase | Bakterielle oder Pilz-Pathogene |
| Polypeptid mit der Wirkung, eine Über- | Bakterielle oder Pilz-Pathogene |
| empfindlichkeitsreaktion auszulösen | |
| Gene der systemischen erworbenen | Virale, bakterielle Pilz- und Nematoden- |
| Widerstandskraft (SAR) | Pathogene |
| Lytisch wirkendes Protein | Bakterielle oder Pilz-Pathogene |
| Lysozym | Bakterielle oder Pilz-Pathogene |
| Chitinasen | Bakterielle oder Pilz-Pathogene |
| Barnase | Bakterielle oder Pilz-Pathogene |
| Glucanasen | Bakterielle oder Pilz-Pathogene |
| Doppelstrang-Ribonuclease | Viren wie das Banana Bunchy Top Virus |
| | (BBTV) |
| Hüllproteine | Viren wie das Banana Bunchy Top Virus |
| | (BBTV) |
| 17kDa oder 60 kDa Protein | Viren wie das Banana Bunchy Top Virus |
| | (BBTV) |
| Einschlussproteine des Kerns z.B. a oder b oder | Viren wie das Banana Bunchy Top Virus |
| Nucleoprotein | (BBTV) |
| Pseudoubiquitin | Viren wie das Banana Bunchy Top Virus |
| | (BBTV) |
| Replicase | Viren wie das Banana Bunchy Top Virus |
| | (BBTV) |
| Toxine von Bacillus thuringiensis, VIP 3, | Lepidoptera, Blattläuse, Milben, Nematoden |
| Bacillus-cereus-Toxine, Photorabdus und | |
| Toxine von Xenorhabdus | |
| 3- Hydroxysteroidoxidase | Lepidoptera, Blattläuse, Milben, Nematoden |
| Peroxidase | Lepidoptera, Blattläuse, Milben, Nematoden |
| Aminopeptidase-Inhibitoren, z.B. Leucin | Lepidoptera, Blattläuse, Milben, Nematoden |
| Aminopeptidase-Inhibitor | |
| Lektine | Lepidoptera, Blattläuse, Milben, Nematoden |
| Protease-Inhibitoren, z.B. Cystatin, Patatin, | Lepidoptera, Blattläuse, Milben, Nematoden |
| CPTI, Virgiferin | |
| Ribosomeninaktivierendes Protein | Lepidoptera, Blattläuse, Milben, Nematoden |
| Stilbensynthase | Lepidoptera, Blattläuse, Milben, Nematoden |
| HMG-CoA-Reductase | Lepidoptera, Blattläuse, Milben, Nematoden |
| Schlüpfauslösefaktor für Zystennematoden | Zystennematoden |
| Barnase | Nematoden, z.B. Wurzelknoten-Nematoden und |
| | Zystennematoden |
| CBI | Wurzelknotennematoden |
| Prinzipien zur Verhinderung der Nahrungs- | Nematoden, z.B. Wurzelknoten-Nematoden und |
| aufnahmen, die an Nematoden-Nährstellen | Wurzel-Zystennematoden |
| induziert werden | |

| Pflanze: Baumwolle | |
|---|---|
| Betroffene Struktur/ Exprimiertes Protein | Merkmal der Pflanze / Toleranz gegenüber |
| Acetolactatsynthase (ALS) | Sulfonylharnstoffverbindungen, Imidazolinone |
| | Triazolpyrimidine, |
| | Pyrimidyloxybenzoate, Phthalide |
| Acetyl-CoA-Carboxylase (ACCase) | Arykoxyphenoxyalkancarbonsäuren, |
| | Cyclohexandione |
| Hydroxyphenylpyruvat-Dioxygenase (HPPD) | Isoxazole wie etwa Isoxaflutol oder |
| | Isoxachlortol, Trione wie etwa |
| | Mesotrion oder Sulcotrion |
| Phosphinothricinacetyltransferase | Phosphinothricin |
| O-Methyltransferase | Geänderter Ligningehalt |
| Glutaminsynthetase | Glufosinat, Bialaphos |
| Adenylosuccinatlyase (ADSL) | Inhibitoren der IMP- und AMP-Synthese |
| Adenylosuccinatsynthase | Inhibitoren der Adenylosuccinatsynthese |
| Anthranilatsynthase | Inhibitoren der Tryptophansynthese und des |
| | -abbaus |
| Nitrilase | 3,5-Dihalogen-4-hydroxy-benzonitrile wie |
| | Bromoxynil und Loxinyl |
| 5-Enolpyruvyl-3-phosphoshikimat- | Glyphosat oder Sulfosat |
| Synthase (EPSPS) | |
| Glyphosatoxidoreductase | Glyphosat oder Sulfosat |
| Protoporphyrinogenoxidase (PROTOX) | Diphenylether, cyclische Imide, |
| | Phenylpyrazole, Pyridinderivate, |
| | Phenopylat, Oxadiazole usw. |
| Cytochrom P450 z.B. P450 SU1 oder Auswahl | Xenobiotika und Herbizide wie etwa |
| | Sulfonylharnstoffverbindungen |
| Polyphenoloxidase oder Polyphenol-Oxidase | Bakterielle oder Pilz-Pathogene |
| (Antisense) | |
| Metallothionein | Bakterielle oder Pilz-Pathogene |
| Ribonuclease | Bakterielle oder Pilz-Pathogene |
| Pilzbekämpfendes Polypeptid AlyAFP | Bakterielle oder Pilz-Pathogene |
| Oxalatoxidase | Bakterielle oder Pilz-Pathogene |
| Glucoseoxidase | Bakterielle oder Pilz-Pathogene |
| Pyrrolnitrinsynthese-Gene | Bakterielle oder Pilz-Pathogene |
| Serin/Threonin-Kinasen | Bakterielle oder Pilz-Pathogene |
| Cecropin B | Bakterielle oder Pilz-Pathogene |
| Phenylalanin-Ammoniak-Lyase (PAL) | Bakterielle oder Pilz-Pathogene |
| Cf-Gene, z.B. Cf 9 Cf5 Cf4 Cf2 | Bakterielle oder Pilz-Pathogene |
| Osmotin | Bakterielle oder Pilz-Pathogene |
| Alpha Hordothionin | Bakterielle oder Pilz-Pathogene |
| Systemin | Bakterielle oder Pilz-Pathogene |
| Polygalacturonase-Inhibitoren | Bakterielle oder Pilz-Pathogene |
| Prf-Steuerungsgen | Bakterielle oder Pilz-Pathogene |
| Phytoalexine | Bakterielle oder Pilz-Pathogene |
| B-1,3-glucanase (Antisense) | Bakterielle oder Pilz-Pathogene |
| Rezeptorkinase | Bakterielle oder Pilz-Pathogene |
| Polypeptid mit der Wirkung, eine Über- | Bakterielle oder Pilz-Pathogene |
| empfindlichkeitsreaktion auszulösen | |
| Gene der systemischen erworbenen | Virale, bakterielle Pilz- und Nematoden- |
| Widerstandskraft (SAR) | Pathogene |
| Lytisch wirkendes Protein | Bakterielle oder Pilz-Pathogene |
| Lysozym | Bakterielle oder Pilz-Pathogene |
| Chitinasen | Bakterielle oder Pilz-Pathogene |
| Barnase | Bakterielle oder Pilz-Pathogene |
| Glucanasen | Bakterielle oder Pilz-Pathogene |
| Doppelstrang-Ribonuclease | Viren wie das Wundtumorvirus (WTV) |
| Hüllproteine | Viren wie das Wundtumorvirus (WTV) |
| 17kDa oder 60 kDa Protein | Viren wie das Wundtumorvirus (WTV) |
| Einschlussproteine des Kerns z.B. a oder b oder | Viren wie das Wundtumorvirus (WTV) |
| Nucleoprotein | |
| Pseudoubiquitin | Viren wie das Wundtumorvirus (WTV) |
| Replicase | Viren wie das Wundtumorvirus (WTV) |
| Toxine von Bacillus thuringiensis, VIP 3, | Lepidoptera, Blattläuse, Milben, Nematoden, |
| Bacillus-cereus-Toxine, Photorabdus und | weiße Fliege |
| Toxine von Xenorhabdus | |
| 3- Hydroxysteroidoxidase | Lepidoptera, Blattläuse, Milben, Nematoden, |
| | weiße Fliege |
| Peroxidase | Lepidoptera, Blattläuse, Milben, Nematoden, |
| | weiße Fliege |
| Aminopeptidase-Inhibitoren, z.B. Leucin | Lepidoptera, Blattläuse, Milben, Nematoden, |
| Aminopeptidase-Inhibitor | weiße Fliege |
| Lektine | Lepidoptera, Blattläuse, Milben, Nematoden, |
| | weiße Fliege |
| Protease-Inhibitoren, z.B. Cystatin, Patatin, | Lepidoptera, Blattläuse, Milben, Nematoden, |
| CPTI, Virgiferin | weiße Fliege |
| Ribosomeninaktivierendes Protein | Lepidoptera, Blattläuse, Milben, Nematoden, |
| | weiße Fliege |
| Stilbensynthase | Lepidoptera, Blattläuse, Milben, Nematoden, |
| | weiße Fliege |
| HMG-CoA-Reductase | Lepidoptera, Blattläuse, Milben, Nematoden, |
| | weiße Fliege |
| Schlüpfauslösefaktor für Zystennematoden | Zystennematoden |
| Barnase | Nematoden, z.B. Wurzelknoten-Nematoden und |
| | Zystennematoden |
| CBI | Wurzelknotennematoden |
| Prinzipien zur Verhinderung der Nahrungs- | Nematoden, z.B. Wurzelknoten-Nematoden und |
| aufnahmen, die an Nematoden-Nährstellen | Wurzel-Zystennematoden |
| induziert werden | |

| Pflanze: Zuckerrohr | |
|---|---|
| Betroffene Merkmal/ Exprimiertes Protein | Merkmal der Pflanze / Toleranz gegenüber |
| Acetolactatsynthase (ALS) | Sulfonylharnstoffverbindungen, Imidazolinone |
| | Triazolpyrimidine, |
| | Pyrimidyloxybenzoate, Phthalide |
| Acetyl-CoA-Carboxylase (ACCase) | Aryloxyphenoxyalkancarbonsäuren, |
| | Cyclohexandione |
| Hydroxyphenylpyruvat-Dioxygenase (HPPD) | Isoxazole wie etwa Isoxaflutol oder |
| | Isoxachlortol, Trione wie etwa |
| | Mesotrion oder Sulcotrion |
| Phosphinothricinacetyltransferase | Phosphinothricin |
| O-Methyltransferase | Geänderter Ligningehalt |
| Glutaminsynthetase | Glufosinat, Bialaphos |
| Adenylosuccinatlyase (ADSL) | Inhibitoren der IMP- und AMP-Synthese |
| Adenylosuccinatsynthase | Inhibitoren der Adenylosuccinatsynthese |
| Anthranilatsynthase | Inhibitoren der Tryptophansynthese und des |
| | -abbaus |
| Nitrilase | 3,5-Dihalogen-4-hydroxy-benzonitrile wie |
| | Bromoxynil und Loxinyl |
| 5-Enolpyruvyl-3-phosphoshikimat- | Glyphosat oder Sulfosat |
| Synthase (EPSPS) | |
| Glyphosatoxidoreductase | Glyphosat oder Sulfosat |
| Protoporphyrinogenoxidase (PROTOX) | Diphenylether, cyclische Imide, |
| | Phenylpyrazole, Pyridinderivate, |
| | Phenopylat, Oxadiazole usw. |
| Cytochrom P450 z.B. P450 SU1 oder Auswahl | Xenobiotika und Herbizide wie etwa |
| | Sulfonylharnstoffverbindungen |
| Polyphenoloxidase oder Polyphenol-Oxidase | Bakterielle oder Pilz-Pathogene |
| (Antisense) | |
| Metallothionein | Bakterielle oder Pilz-Pathogene |
| Ribonuclease | Bakterielle oder Pilz-Pathogene |
| Pilzbekämpfendes Polypeptid AlyAFP | Bakterielle oder Pilz-Pathogene |
| Oxalatoxidase | Bakterielle oder Pilz-Pathogene |
| Glucoseoxidase | Bakterielle oder Pilz-Pathogene |
| Pyrrolnitrinsynthese-Gene | Bakterielle oder Pilz-Pathogene |
| Serin/Threonin-Kinasen | Bakterielle oder Pilz-Pathogene |
| Cecropin B | Bakterielle oder Pilz-Pathogene |
| Phenylalanin-Ammoniak-Lyase (PAL) | Bakterielle oder Pilz-Pathogene |
| Cf-Gene, z.B. Cf 9 Cf5 Cf4 Cf2 | Bakterielle oder Pilz-Pathogene |
| Osmotin | Bakterielle oder Pilz-Pathogene |
| Alpha Hordothionin | Bakterielle oder Pilz-Pathogene |
| Systemin | Bakterielle oder Pilz-Pathogene |
| Polygalacturonase-Inhibitoren | Bakterielle oder Pilz-Pathogene |
| Prf-Steuerungsgen | Bakterielle oder Pilz-Pathogene |
| Phytoalexine | Bakterielle oder Pilz-Pathogene |
| B-1,3-glucanase (Antisense) | Bakterielle oder Pilz-Pathogene |
| Rezeptorkinase | Bakterielle oder Pilz-Pathogene |
| Polypeptid mit der Wirkung, eine Über- | Bakterielle oder Pilz-Pathogene |
| empfindlichkeitsreaktion auszulösen | |
| Gene der systemischen erworbenen | Virale, bakterielle Pilz- und Nematoden- |
| Widerstandskraft (SAR) | Pathogene |
| Lytisch wirkendes Protein | Bakterielle oder Pilz-Pathogene |
| Lysozym | Bakterielle oder Pilz-Pathogene, z.B. |
| | Clavibacter |
| Chitinasen | Bakterielle oder Pilz-Pathogene |
| Barnase | Bakterielle oder Pilz-Pathogene |
| Glucanasen | Bakterielle oder Pilz-Pathogene |
| Doppelstrang-Ribonuclease | Viren wie SCMV, SrMV |
| Hüllproteine | Viren wie SCMV, SrMV |
| 17kDa oder 60 kDa Protein | Viren wie SCMV, SrMV |
| Einschlussproteine des Kerns z.B. a oder b oder | Viren wie SCMV, SrMV |
| Nucleoprotein | |
| Pseudoubiquitin | Viren wie SCMV, SrMV |
| Replicase | Viren wie SCMV, SrMV |
| Toxine von Bacillus thuringiensis, VIP 3, | Lepidoptera, Blattläuse, Milben, Nematoden, |
| Bacillus-cereus-Toxine, Photorabdus und | weiße Fliege, Käfer wie z.B. der mexikanische |
| Toxine von Xenorhabdus | Reisbohrer |
| 3- Hydroxysteroidoxidase | Lepidoptera, Blattläuse, Milben, Nematoden, |
| | weiße Fliege, Käfer wie z.B. der mexikanische |
| | Reisbohrer |
| Peroxidase | Lepidoptera, Blattläuse, Milben, Nematoden, |
| | weiße Fliege, Käfer wie z.B. der mexikanische |
| | Reisbohrer |
| Aminopeptidase-Inhibitoren, z.B. Leucin | Lepidoptera, Blattläuse, Milben, Nematoden, |
| Aminopeptidase-Inhibitor | weiße Fliege, Käfer wie z.B. der mexikanische |
| | Reisbohrer |
| Lektine | Lepidoptera, Blattläuse, Milben, Nematoden, |
| | weiße Fliege, Käfer wie z.B. der mexikanische |
| | Reisbohrer |
| Protease-Inhibitoren, z.B. Cystatin, Patatin, | Lepidoptera, Blattläuse, Milben, Nematoden, |
| CPTI, Virgiferin | weiße Fliege, Käfer wie z.B. der mexikanische |
| | Reisbohrer |
| Ribosomeninaktivierendes Protein | Lepidoptera, Blattläuse, Milben, Nematoden, |
| | weiße Fliege, Käfer wie z.B. der mexikanische |
| | Reisbohrer |
| Stilbensynthase | Lepidoptera, Blattläuse, Milben, Nematoden, |
| | weiße Fliege, Käfer wie z.B. der mexikanische |
| | Reisbohrer |
| HMG-CoA-Reductase | Lepidoptera, Blattläuse, Milben, Nematoden, |
| | weiße Fliege, Käfer wie z.B. der mexikanische |
| | Reisbohrer |
| Schlüpfauslösefaktor für Zystennematoden | Zystennematoden |
| Barnase | Nematoden, z.B. Wurzelknoten-Nematoden und |
| | Zystennematoden |
| CBI | Wurzelknotennematoden |
| Prinzipien zur Verhinderung der Nahrungs- | Nematoden, z.B. Wurzelknoten-Nematoden und |
| aufnahmen, die an Nematoden-Nährstellen | Wurzel-Zystennematoden |
| induziert werden | |

| Pflanze: Sonnenblume | |
|---|---|
| Betroffene Struktur/ Exprimiertes Protein | Merkmal der Pflanze / Toleranz gegenüber |
| Acetolactatsynthase (ALS) | Sulfonylharnstoffverbindungen, Imidazolinone |
| | Triazolpyrimidine, |
| | Pyrimidyloxybenzoate, Phthalide |
| Acetyl-CoA-Carboxylase (ACCase) | Aryloxyphenoxyalkancarbonsäuren, |
| | Cyclohexandione |
| Hydroxyphenylpyruvat-Dioxygenase (HPPD) | Isoxazole wie etwa Isoxaflutol oder |
| | Isoxachlortol, Trione wie etwa |
| | Mesotrion oder Sulcotrion |
| Phosphinothricinacetyltransferase | Phosphinothricin |
| O-Methyltransferase | Geänderter Ligningehalt |
| Glutaminsynthetase | Glufosinat, Bialaphos |
| Adenylosuccinatlyase (ADSL) | Inhibitoren der IMP- und AMP-Synthese |
| Adenylosuccinatsynthase | Inhibitoren der Adenylosuccinatsynthese |
| Anthranilatsynthase | Inhibitoren der Tryptophansynthese und des |
| | -abbaus |
| Nitrilase | 3,5-Dihalogen-4-hydroxy-benzonitrile wie |
| | Bromoxynil und Loxinyl |
| 5-Enolpyruvyl-3-phosphoshikimat- | Glyphosat oder Sulfosat |
| Synthase (EPSPS) | |
| Glyphosatoxidoreductase | Glyphosat oder Sulfosat |
| Protoporphyrinogenoxidase (PROTOX) | Diphenylether, cyclische Imide, |
| | Phenylpyrazole, Pyridinderivate, |
| | Phenopylat, Oxadiazole usw. |
| Cytochrom P450 z.B. P450 SU1 oder Auswahl | Xenobiotika und Herbizide wie etwa |
| | Sulfonylharnstoffverbindungen |
| Polyphenoloxidase oder Polyphenol-Oxidase | Bakterielle oder Pilz-Pathogene |
| (Antisense) | |
| Metallothionein | Bakterielle oder Pilz-Pathogene |
| Ribonuclease | Bakterielle oder Pilz-Pathogene |
| Pilzbekämpfendes Polypeptid AlyAFP | Bakterielle oder Pilz-Pathogene |
| Oxalatoxidase | Bakterielle oder Pilz-Pathogene, z.B. |
| | Sklerotinia |
| Glucoseoxidase | Bakterielle oder Pilz-Pathogene |
| Pyrrolnitrinsynthese-Gene | Bakterielle oder Pilz-Pathogene |
| Serin/Threonin-Kinasen | Bakterielle oder Pilz-Pathogene |
| Cecropin B | Bakterielle oder Pilz-Pathogene |
| Phenylalanin-Ammoniak-Lyase (PAL) | Bakterielle oder Pilz-Pathogene |
| Cf-Gene, z.B. Cf 9 Cf5 Cf4 Cf2 | Bakterielle oder Pilz-Pathogene |
| Osmotin | Bakterielle oder Pilz-Pathogene |
| Alpha Hordothionin | Bakterielle oder Pilz-Pathogene |
| Systemin | Bakterielle oder Pilz-Pathogene |
| Polygalacturonase-Inhibitoren | Bakterielle oder Pilz-Pathogene |
| Prf-Steuerungsgen | Bakterielle oder Pilz-Pathogene |
| Phytoalexine | Bakterielle oder Pilz-Pathogene |
| B-1,3-glucanase (Antisense) | Bakterielle oder Pilz-Pathogene |
| Rezeptorkinase | Bakterielle oder Pilz-Pathogene |
| Polypeptid mit der Wirkung, eine Über- | Bakterielle oder Pilz-Pathogene |
| empfindlichkeitsreaktion auszulösen | |
| Gene der systemischen erworbenen | Virale, bakterielle Pilz- und Nematoden- |
| Widerstandskraft (SAR) | Pathogene |
| Lytisch wirkendes Protein | Bakterielle oder Pilz-Pathogene |
| Lysozym | Bakterielle oder Pilz-Pathogene |
| Chitinasen | Bakterielle oder Pilz-Pathogene |
| Barnase | Bakterielle oder Pilz-Pathogene |
| Glucanasen | Bakterielle oder Pilz-Pathogene |
| Doppelstrang-Ribonuclease | Viren wie CMV, TMV |
| Hüllproteine | Viren wie CMV, TMV |
| 17kDa oder 60 kDa Protein | Viren wie CMV, TMV |
| Einschlussproteine des Kerns z.B. a oder b oder | Viren wie CMV, TMV |
| Nucleoprotein | |
| Pseudoubiquitin | Viren wie CMV, TMV |
| Replicase | Viren wie CMV, TMV |
| Toxine von Bacillus thuringiensis, VIP 3, | Lepidoptera, Blattläuse, Milben, Nematoden, |
| Bacillus-cereus-Toxine, Photorabdus und | weiße Fliege, Käfer |
| Toxine von Xenorhabdus | |
| 3- Hydroxysteroidoxidase | Lepidoptera, Blattläuse, Milben, Nematoden, |
| | weiße Fliege, Käfer |
| Peroxidase | Lepidoptera, Blattläuse, Milben, Nematoden, |
| | weiße Fliege, Käfer |
| Aminopeptidase-Inhibitoren, z.B. Leucin | Lepidoptera, Blattläuse, Milben, Nematoden, |
| Aminopeptidase-Inhibitor | weiße Fliege, Käfer |
| Lektine | Lepidoptera, Blattläuse, Milben, Nematoden, |
| | weiße Fliege, Käfer |
| Protease-Inhibitoren, z.B. Cystatin, Patatin, | Lepidoptera, Blattläuse, Milben, Nematoden, |
| CPTI, Virgiferin | weiße Fliege, Käfer |
| Ribosomeninaktivierendes Protein | Lepidoptera, Blattläuse, Milben, Nematoden, |
| | weiße Fliege, Käfer |
| Stilbensynthase | Lepidoptera, Blattläuse, Milben, Nematoden, |
| | weiße Fliege, Käfer |
| HMG-CoA-Reductase | Lepidoptera, Blattläuse, Milben, Nematoden, |
| | weiße Fliege, Käfer |
| Schlüpfauslösefaktor für Zystennematoden | Zystennematoden |
| Barnase | Nematoden, z.B. Wurzelknoten-Nematoden und |
| | Zystennematoden |
| CBI | Wurzelknotennematoden |
| Prinzipien zur Verhinderung der Nahrungs- | Nematoden, z.B. Wurzelknoten-Nematoden und |
| aufnahmen, die an Nematoden-Nährstellen | Wurzel-Zystennematoden |
| induziert werden | |

| Pflanzen: Zuckerrübe, Rüben | |
|---|---|
| Betroffene Struktur/ Exprimiertes Protein | Merkmal der Pflanze / Toleranz gegenüber |
| Acetolactatsynthase (ALS) | Sulfonylharnstoffverbindungen, Imidazolinone |
| | Triazolpyrimidine, |
| | Pyrimidyloxybenzoate, Phthalide |
| Acetyl-CoA-Carboxylase (ACCase) | Aryloxyphenoxyalkancarbonsäuren, |
| | Cyclohexandione |
| Hydroxyphenylpyruvat-Dioxygenase (HPPD) | Isoxazole wie etwa Isoxaflutol oder |
| | Isoxachlortol, Trione wie etwa |
| | Mesotrion oder Sulcotrion |
| Phosphinothricinacetyltransferase | Phosphinothricin |
| O-Methyltransferase | Geänderter Ligningehalt |
| Glutaminsynthetase | Glufosinat, Bialaphos |
| Adenylosuccinatlyase (ADSL) | Inhibitoren der IMP- und AMP-Synthese |
| Adenylosuccinatsynthase | Inhibitoren der Adenylosuccinatsynthese |
| Anthranilatsynthase | Inhibitoren der Tryptophansynthese und des |
| | -abbaus |
| Nitrilase | 3,5-Dihalogen-4-hydroxy-benzonitrile wie |
| | Bromoxynil und Loxinyl |
| 5-Enolpyruvyl-3-phosphoshikimat- | Glyphosat oder Sulfosat |
| Synthase (EPSPS) | |
| Glyphosatoxidoreductase | Glyphosat oder Sulfosat |
| Protoporphyrinogenoxidase (PROTOX) | Diphenylether, cyclische Imide, |
| | Phenylpyrazole, Pyridinderivate, |
| | Phenopylat, Oxadiazole usw. |
| Cytochrom P450 z.B. P450 SU1 oder Auswahl | Xenobiotika und Herbizide wie etwa |
| | Sulfonylharnstoffverbindungen |
| Polyphenoloxidase oder Polyphenol-Oxidase | Bakterielle oder Pilz-Pathogene |
| (Antisense) | |
| Metallothionein | Bakterielle oder Pilz-Pathogene |
| Ribonuclease | Bakterielle oder Pilz-Pathogene |
| Pilzbekämpfendes Polypeptid AlyAFP | Bakterielle oder Pilz-Pathogene |
| Oxalatoxidase | Bakterielle oder Pilz-Pathogene, z.B. |
| | Sklerotinia |
| Glucoseoxidase | Bakterielle oder Pilz-Pathogene |
| Pyrrolnitrinsynthese-Gene | Bakterielle oder Pilz-Pathogene |
| Serin/Threonin-Kinasen | Bakterielle oder Pilz-Pathogene |
| Cecropin B | Bakterielle oder Pilz-Pathogene |
| Betroffene Struktur / exprimiertes Prinzip | Merkmal der Pflanze / Toleranz gegenüber |
| Phenylalanin-Ammoniak-Lyase (PAL) | Bakterielle oder Pilz-Pathogene |
| Cf-Gene, z.B. Cf 9 Cf5 Cf4 Cf2 | Bakterielle oder Pilz-Pathogene |
| Osmotin | Bakterielle oder Pilz-Pathogene |
| Alpha Hordothionin | Bakterielle oder Pilz-Pathogene |
| Systemin | Bakterielle oder Pilz_Pathogene |
| Polygalacturonase-Inhibitoren | Bakterielle oder Pilz-Pathogene |
| Prf-Steuerungsgen | Bakterielle oder Pilz-Pathogene |
| Phytoalexine | Bakterielle oder Pilz-Pathogene |
| B-1,3-glucanase (Antisense) | Bakterielle oder Pilz-Pathogene |
| AX + WIN-Proteine | Bakterielle und Pilz-Pathogene wie |
| | Cercospora beticola |
| Rezeptorkinase | Bakterielle oder Pilz-Pathogene |
| Polypeptid mit der Wirkung, eine Über- | Bakterielle oder Pilz-Pathogene |
| empfindlichkeitsreaktion auszulösen | |
| Gene der systemischen erworbenen | Virale, bakterielle Pilz- und Nematoden- |
| Widerstandskraft (SAR) | Pathogene |
| Lytisch wirkendes Protein | Bakterielle oder Pilz-Pathogene |
| Lysozym | Bakterielle oder Pilz-Pathogene |
| Chitinasen | Bakterielle oder Pilz-Pathogene |
| Barnase | Bakterielle oder Pilz-Pathogene |
| Glucanasen | Bakterielle oder Pilz-Pathogene |
| Doppelstrang-Ribonuclease | Viren wie etwa BNYVV |
| Hüllproteine | Viren wie etwa BNYVV |
| 17kDa oder 60 kDa Protein | Viren wie etwa BNYVV |
| Einschlussproteine des Kerns z.B. a oder b oder | Viren wie etwa BNYVV |
| Nucleoprotein | |
| Pseudoubiquitin | Viren wie etwa BNYVV |
| Replicase | Viren wie etwa BNYVV |
| Toxine von Bacillus thuringiensis, VIP 3, | Lepidoptera, Blattläuse, Milben, Nematoden, |
| Bacillus-cereus-Toxine, Photorabdus und | weiße Fliege, Käfer, Wurzelfliegen |
| Toxine von Xenorhabdus | |
| 3- Hydroxysteroidoxidase | Lepidoptera, Blattläuse, Milben, Nematoden, |
| | weiße Fliege, Käfer, Wurzelfliegen |
| Peroxidase | Lepidoptera, Blattläuse, Milben, Nematoden, |
| | weiße Fliege, Käfer, Wurzelfliegen |
| Aminopeptidase-Inhibitoren, z.B. Leucin | Lepidoptera, Blattläuse, Milben, Nematoden, |
| Aminopeptidase-Inhibitor | weiße Fliege, Käfer, Wurzelfliegen |
| Lektine | Lepidoptera, Blattläuse, Milben, Nematoden, |
| | weiße Fliege, Käfer, Wurzelfliegen |
| Protease-Inhibitoren, z.B. Cystatin, Patatin, | Lepidoptera, Blattläuse, Milben, Nematoden, |
| CPTI, Virgiferin | weiße Fliege, Käfer, Wurzelfliegen |
| Ribosomeninaktivierendes Protein | Lepidoptera, Blattläuse, Milben, Nematoden, |
| | weiße Fliege, Käfer, Wurzelfliegen |
| Stilbensynthase | Lepidoptera, Blattläuse, Milben, Nematoden, |
| | weiße Fliege, Käfer, Wurzelfliegen |
| HMG-CoA-Reductase | Lepidoptera, Blattläuse, Milben, Nematoden, |
| | weiße Fliege, Käfer, Wurzelfliegen |
| Schlüpfauslösefaktor für Zystennematoden | Zystennematoden |
| Barnase | Nematoden, z.B. Wurzelknoten-Nematoden und |
| | Zystennematoden |
| Resistenz-Stelle für Zystennematoden der | Zystennematoden |
| Rüben | |
| CBI | Wurzelknotennematoden |
| Prinzipien zur Verhinderung der Nahrungs- | Nematoden, z.B. Wurzelknoten-Nematoden und |
| aufnahmen, die Nahrungsaufnahmen, die | Wurzel-Zystennematoden |
| induziert werden | |

**Tabelle 2**

| | |
|---|---|
| Die folgenden Abkürzungen wurden in der Tabelle benutzt: | |
| Aktives Prinzip der transgenen Pflanze: AP | |
| Photorhabdus luminescens: PL | |
| Xenorhabdus nematophilus: XN | |
| Proteinase-Inhibitoren: Plnh. | |
| Pflanzenlektine PLec. | |
| Agglutinine: Aggl. | |
| 3-Hydroxysteroidoxidase: HO | |
| Cholesterinoxidase: CO | |
| Chitinase: CH | |
| Glucanase: GL | |
| Stilbensynthase SS | |
| AP | Kontrolle von |
| CrylA(a) | Adoxophyes spp. |
| CrylA(a) | Agrotis spp. |
| CrylA(a) | Alabama argiliaceae |
| CrylA(a) | Anticarsia gemmatalis |
| CrylA(a) | Chilo spp. |
| CrylA(a) | Clysia ambiguella |
| CrylA(a) | Crocidolomia binotalis |
| CrylA(a) | Cydia spp. |
| CrylA(a) | Diparopsis castanea |
| CrylA(a) | Earias spp. |
| CrylA(a) | Ephestia spp. |
| CrylA(a) | Heliothis spp. |
| CrylA(a) | Heliula undalis |
| CrylA(a) | Keiferia lycopersicella |
| CrylA(a) | Leucoptera scitella |
| CrylA(a) | Lithocollethis spp. |
| CrylA(a) | Lobesia botrana |
| CrylA(a) | Ostrinia nubilalis |
| CrylA(a) | Pandemis spp. |
| CrylA(a) | Pectinophora gossyp. |
| CrylA(a) | Phyllocnistis citrella |
| CrylA(a) | Pieris spp. |
| CrylA(a) | Plutella xylostella |
| CrylA(a) | Scirpophaga spp. |
| CrylA(a) | Sesamia spp. |
| CrylA(a) | Sparganothis spp. |
| CrylA(a) | Spodoptera spp. |
| CrylA(a) | Tortrix spp. |
| CrylA(a) | Trichoplusia ni |
| CrylA(a) | Agriotes spp. |
| CrylA(a) | Anthonomus grandis |
| CrylA(a) | Curculio spp. |
| CrylA(a) | Diabrotica balteata |
| CrylA(a) | Leptinotarsa spp. |
| CrylA(a) | Lissorhoptrus spp. |
| CrylA(a) | Otiorhynchus spp. |
| CrylA(a) | Aleurothrixus spp. |
| CrylA(a) | Aleyrodes spp. |
| CrylA(a) | Aonidiella spp. |
| CrylA(a) | Aphididea spp. |
| CrylA(a) | Aphis spp. |
| CrylA(a) | Bemisia tabaci |
| CrylA(a) | Empoasca spp. |
| CrylA(a) | Mycus spp. |
| CrylA(a) | Nephotettix spp. |
| CrylA(a) | Nilaparvata spp. |
| CrylA(a) | Pseudococcus spp. |
| CrylA(a) | Psylla spp. |
| CrylA(a) | Quadraspidiotus spp. |
| CrylA(a) | Schizaphis spp. |
| CrylA(a) | Trialeurodes spp. |
| CrylA(a) | Lyriomyza spp. |
| CrylA(a) | Oscinella spp. |
| CrylA(a) | Phorbia spp. |
| CrylA(a) | Frankliniella spp. |
| CrylA(a) | Thrips spp. |
| CrylA(a) | Scirtothrips aurantii |
| CrylA(a) | Aceria spp. |
| CrylA(a) | Aculus spp. |
| CrylA(a) | Brevipaipus spp. |
| CrylA(a) | Panonychus spp. |
| CrylA(a) | Phyllocoptruta spp. |
| CrylA(a) | Tetranychus spp. |
| CrylA(a) | Heterodera spp. |
| CrylA(a) | Meloidogyne spp. |
| CrylA(b) | Adoxophyes spp |
| CrylA(b) | Agrotis spp |
| CrylA(b) | Alabama argillaceae |
| CrylA(b) | Anticarsia gemmatalis |
| CrylA(b) | Chilo spp. |
| CrylA(b) | Ciysia ambiguella |
| CrylA(b) | Crocidolomia binotaiis |
| CrylA(b) | Cydia spp. |
| CrylA(b) | Diparopsis castanea |
| CrylA(b) | Earias spp. |
| CrylA(b) | Ephestia spp. |
| CrylA(b) | Heliothis spp. |
| CrylA(b) | Hellula undalis |
| CrylA(b) | Keiferia lycopersicella |
| CrylA(b) | Leucoptera scitella |
| CrylA(b) | Lithocollethis spp. |
| CrylA(b) | Lobesia botrana |
| CrylA(b) | Ostrinia nubilalis |
| CrylA(b) | Pandemis spp. |
| CrylA(b) | Pectinophora gossyp. |
| CrylA(b) | Phyllocnistis citrella |
| CrylA(b) | Pieris spp. |
| CrylA(b) | Plutelia xyiostella |
| CrylA(b) | Scirpophaga spp. |
| CrylA(b) | Sesamia spp. |
| CrylA(b) | Sparganothis spp. |
| CrylA(b) | Spodoptera spp. |
| CrylA(b) | Tortrix spp. |
| CrylA(b) | Trichoplusia ni |
| CrylA(b) | Agriotes spp. |
| CrylA(b) | Anthonomus grandis |
| CrylA(b) | Curculio spp. |
| CrylA(b) | Diabrotica balteata |
| CrylA(b) | Leptinotarsa spp. |
| CrylA(b) | Lissorhoptrus spp. |
| CrylA(b) | Otiorhynchus spp. |
| CrylA(b) | Aleurothrixus spp. |
| CrylA(b) | Aleyrodes spp. |
| CrylA(b) | Aonidiella spp. |
| CrylA(b) | Aphididae spp. |
| CrylA(b) | Aphis spp. |
| CrylA(b) | Bemisia tabaci |
| CrylA(b) | Empoasca spp. |
| CrylA(b) | Mycus spp. |
| CrylA(b) | Nephotettix spp. |
| CrylA(b) | Nilaparvata spp. |
| CrylA(b) | Pseudococcus spp. |
| CrylA(b) | Psylla spp. |
| CrylA(b) | Quadraspidiotus spp. |
| CrylA(b) | Schizaphis spp. |
| CrylA(b) | Trialeurodes spp. |
| CrylA(b) | Lyriomyza spp. |
| CrylA(b) | Oscinella spp. |
| CrylA(b) | Phorbia spp. |
| CrylA(b) | Frankliniella spp. |
| CrylA(b) | Thrips spp. |
| CrylA(b) | Scirtothrips aurantii |
| CrylA(b) | Aceria spp. |
| CrylA(b) | Aculus spp. |
| CrylA(b) | Brevipalpus spp. |
| CrylA(b) | Panonychus spp. |
| CrylA(b) | Phyllocoptruta spp. |
| CrylA(b) | Tetranychus spp. |
| CrylA(b) | Heterodera spp. |
| CrylA(b) | Meloidogyne spp. |
| CrylA(c) | Adoxophyes spp. |
| CrylA(c) | Agrotis spp. |
| CrylA(c) | Alabama argillaceae |
| CrylA(c) | Anticarsia gemmatalis |
| CrylA(c) | Chilo spp. |
| CrylA(c) | Ciysia ambiguella |
| CrylA(c) | Crocidolomia binotalis |
| CrylA(c) | Cydia spp. |
| CrylA(c) | Diparopsis castanea |
| CrylA(c) | Earias spp. |
| CrylA(c) | Ephestia spp. |
| CrylA(c) | Heliothis spp. |
| CrylA(c) | Hellula undalis |
| CrylA(c) | Keiferia lycopersicella |
| CrylA(c) | Leucoptera scitella |
| CrylA(c) | Lithocollethis spp. |
| CrylA(c) | Lobesia botrana |
| CrylA(c) | Ostrinia nubilalis |
| CrylA(c) | Pandemis spp. |
| CrylA(c) | Pectinophora gossypielia. |
| CrylA(c) | Phyllocnistis citrella |
| CrylA(c) | Pieris spp. |
| CrylA(c) | Plutella xyiostella |
| CrylA(c) | Scirpophaga spp. |
| CrylA(c) | Sesamia spp. |
| CrylA(c) | Sparganothis spp. |
| CrylA(c) | Spodoptera spp. |
| CrylA(c) | Tortrix spp. |
| CrylA(c) | Trichoplusia ni |
| CrylA(c) | Agriotes spp. |
| CrylA(c) | Anthonomus grandis |
| CrylA(c) | Curculio spp. |
| CrylA(c) | Diabrotica baiteata |
| CrylA(c) | Leptinotarsa spp. |
| CrylA(c) | Lissorhoptrus spp. |
| CrylA(c) | Otiorhynchus spp. |
| CrylA(c) | Aleurothrixus spp. |
| CrylA(c) | Aleyrodes spp. |
| CrylA(c) | Aonidiella spp. |
| CrylA(c) | Aphididae spp. |
| CrylA(c) | Aphis spp. |
| CrylA(c) | Bemisia tabaci |
| CrylA(c) | Empoasca spp. |
| CrylA(c) | Mycus spp. |
| CrylA(c) | Nephotettix spp. |
| CrylA(c) | Nilaparvata spp. |
| CrylA(c) | Pseudococcus spp. |
| CrylA(c) | Psylla spp. |
| CrylA(c) | Quadraspidiotus spp. |
| CrylA(c) | Schizaphis spp. |
| CrylA(c) | Trialeurodes spp. |
| CrylA(c) | Lyriomyza spp. |
| CrylA(c) | Oscinelia spp. |
| CrylA(c) | Phorbia spp. |
| CrylA(c) | Frankiiniella spp. |
| CrylA(c) | Thrips spp. |
| CrylA(c) | Scirtothrips aurantii |
| CrylA(c) | Aceria spp. |
| CrylA(c) | Aculus spp. |
| CrylA(c) | Brevipalpus spp. |
| CrylA(c) | Panonychus spp. |
| CrylA(c) | Phyllocoptruta spp. |
| CrylA(c) | Tetranychus spp. |
| CrylA(c) | Heterodera spp. |
| CrylA(c) | Meloidogyne spp. |
| CryllA | Adoxophyes spp. |
| CryllA | Agrotis spp. |
| CryllA | Alabama argillaceae |
| CryllA | Anticarsia gemmatalis |
| CryllA | Chilo spp. |
| CryllA | Clysia ambiguella |
| CryllA | Crocidolomia binotalis |
| CryllA | Cydia spp. |
| CryllA | Diparopsis castanea |
| CryllA | Earias spp. |
| CryllA | Ephestia spp. |
| CryllA | Heliothis spp. |
| CryllA | Hellula undalis |
| CryllA | Keiferia lycopersicella |
| CryllA | Leucoptera scitella |
| CryllA | Lithocoliethis spp. |
| CryllA | Lobesia botrana |
| CryllA | Ostrinia nubilalis |
| CryllA | Pandemis spp. |
| CryllA | Pectinophora gossyp. |
| CryllA | Phyllocnistis citrella |
| CryllA | Pieris spp. |
| CryllA | Plutella xylostella |
| CryllA | Scirpophaga spp. |
| CryllA | Sesamia spp. |
| CryllA | Sparganothis spp. |
| CryllA | Spodoptera spp. |
| CryllA | Tortrix spp. |
| CryllA | Trichoplusia ni |
| CryllA | Agriotes spp. |
| CryllA | Anthonomus grandis |
| CryllA | Curculio spp. |
| CryllA | Diabrotica balteata |
| CryllA | Leptinotarsa spp. |
| CryllA | Lissorhoptrus spp. |
| CryllA | Otiorhynchus spp. |
| CryllA | Aleurothrixus spp. |
| CryllA | Aleyrodes spp. |
| CryllA | Aonidiella spp. |
| CryllA | Aphididae spp. |
| CryllA | Aphis spp. |
| CryllA | Bemisia tabaci |
| CryllA | Empoasca spp. |
| CryllA | Mycus spp. |
| CryllA | Nephotettix spp. |
| CryllA | Nilaparvata spp. |
| CryllA | Pseudococcus spp. |
| CryllA | Psyila spp. |
| CryllA | Quadraspidiotus spp. |
| CryllA | Schizaphis spp. |
| CryllA | Trialeurodes spp. |
| CryllA | Lyriomyza spp. |
| CryllA | Oscinella spp. |
| CryllA | Phorbia spp. |
| CryllA | Frankliniella spp. |
| CryllA | Thrips spp. |
| CryllA | Scirtothrips aurantii |
| CryllA | Aceria spp. |
| CryllA | Acutus spp. |
| CryllA | Brevipalpus spp. |
| CryllA | Panonychus spp. |
| CryllA | Phyllocoptruta spp. |
| CryllA | Tetranychus spp. |
| CryllA | Heterodera spp. |
| CryllA | Meloidogyne spp. |
| CrylllA | Adoxophyes spp. |
| CrylllA | Agrotis spp. |
| CrylllA | Alabama argiiiaceae |
| CrylllA | Anticarsia gemmataiis |
| CrylllA | Chilo spp. |
| CrylllA | Ciysia ambiguelia |
| CrylllA | Crocodolomia binotalis |
| CrylllA | Cydia spp. |
| CrylllA | Diparopsis castanea |
| CrylllA | Earias spp. |
| CrylllA | Ephestia spp. |
| CrylllA | Heliothis spp. |
| CrylllA | Hellula undalis |
| CrylllA | Keiferia lycopersicella |
| CrylllA | Leucoptera scitella |
| CrylllA | Lithocollethis spp. |
| CrylllA | Lobesia botrana |
| CrylllA | Ostrinia nubilalis |
| CrylllA | Pandemis spp. |
| CrylllA | Pectinophora gossyp. |
| CrylllA | Phyllocnistis citrella |
| CrylllA | Pieris spp. |
| CrylllA | Plutella xylostella |
| CrylllA | Scirpophaga spp. |
| CrylllA | Sesamia spp. |
| CrylllA | Sparganothis spp. |
| CrylllA | Spodoptera spp. |
| CrylllA | Tortrix spp. |
| CrylllA | Trichoplusia ni |
| CrylllA | Agriotes spp. |
| CrylllA | Anthonomus grandis |
| CrylllA | Curculio spp. |
| CrylllA | Diabrotica balteata |
| CrylllA | Leptinotarsa spp. |
| CrylllA | Lissorhoptrus spp. |
| CrylllA | Otiorhynchus spp. |
| CrylllA | Aleurothrixus spp. |
| CrylllA | Aleyrodes spp. |
| CrylllA | Aonidiella spp. |
| CrylllA | Aphididae spp. |
| CrylllA | Aphis spp. |
| CrylllA | Bemisia tabaci |
| CrylllA | Empoasca spp. |
| CrylllA | Mycus spp. |
| CrylllA | Nephotettix spp. |
| CrylllA | Nilaparvata spp. |
| CrylllA | Pseudococcus spp. |
| CrylllA | Psylla spp. |
| CrylllA | Quadraspidiotus spp. |
| CrylllA | Schizaphis spp. |
| CrylllA | Trialeurodes spp. |
| CrylllA | Lyriomyza spp. |
| CrylllA | Oscinella spp. |
| CrylllA | Phorbia spp. |
| CrylllA | Frankliniella spp. |
| CrylllA | Thrips spp. |
| CrylllA | Scirtothrips aurantii |
| CrylllA | Aceria spp. |
| CrylllA | Aculus spp. |
| CrylllA | Brevipalpus spp. |
| CrylllA | Panonychus spp. |
| CrylllA | Phyllocoptruta spp. |
| CrylllA | Tetranychus spp. |
| CrylllA | Heterodera spp. |
| CrylllA | Meloidogyne spp. |
| CrylllB2 | Adoxophyes spp. |
| CrylllB2 | Agrotis spp. |
| CrylllB2 | Alabama argiilaceae |
| CrylllB2 | Anticarsia gemmatalis |
| CrylllB2 | Chilo spp. |
| CrylllB2 | Clysia ambiguella |
| CrylllB2 | Crocidolomia binotaiis |
| CrylllB2 | Cydia spp. |
| CrylllB2 | Diparopsis castanea |
| CrylllB2 | Earias spp. |
| CrylllB2 | Ephestia spp. |
| CrylllB2 | Heliothis spp. |
| CrylllB2 | Hellula undalis |
| CrylllB2 | Keiferia lycopersicella |
| CrylllB2 | Leucoptera sectelia |
| CrylllB2 | Lithocollethis spp. |
| CrylllB2 | Lobesia botrana |
| CrylllB2 | Ostrinia nubilalis |
| CrylllB2 | Pandemis spp. |
| CryllIB2 | Pectinophora gossyp. |
| CrylIIB2 | Phyllocnistis citrella |
| CrylllB2 | Pieris spp. |
| CrylllB2 | Plutella xylostella |
| CrylllB2 | Scirpophaga spp. |
| CrylllB2 | Sesamia spp. |
| CrylllB2 | Sparganothis spp. |
| CrylllB2 | Spodoptera spp. |
| CrylllB2 | Tortrix spp. |
| CrylllB2 | Trichoplusia ni |
| CrylllB2 | Agriotes spp. |
| CrylllB2 | Anthonomus grandis |
| CrylllB2 | Curculio spp. |
| CrylllB2 | Diabrotica balteata |
| CrylllB2 | Leptinotarsa spp. |
| CrylllB2 | Lissorhoptrus spp. |
| CrylllB2 | Otiorhynchus spp. |
| CrylllB2 | Aleurothrixus spp. |
| CrylllB2 | Aleyrodes spp. |
| CrylllB2 | Aonidiella spp. |
| CrylllB2 | Aphididae spp. |
| CrylllB2 | Aphis spp. |
| CrylllB2 | Bemisia tabaci |
| CrylllB2 | Empoasca spp. |
| CrylIIB2 | Mycus spp. |
| CrylllB2 | Nephotettix spp. |
| CrylllB2 | Nilaparvata spp. |
| CrylllB2 | Pseudococcus spp. |
| CrylllB2 | Psylla spp. |
| CrylllB2 | Quadraspidiotus spp. |
| CrylllB2 | Schizaphis spp. |
| CrylllB2 | Trialeurodes spp. |
| CrylllB2 | Lyriornyza spp. |
| CrylllB2 | Oscinella spp. |
| CrylllB2 | Phorbia spp. |
| CrylllB2 | Frankliniella spp. |
| CrylllB2 | Thrips spp. |
| CrylllB2 | Scirtothrips aurantii |
| CrylllB2 | Aceria spp. |
| CrylllB2 | Acutus spp. |
| CrylllB2 | Brevipalpus spp. |
| CrylllB2 | Panonychus spp. |
| CrylllB2 | Phyllocoptruta spp. |
| CrylllB2 | Tetranychus spp. |
| CrylllB2 | Heterodera spp. |
| CrylllB2 | Meloidogyne spp. |
| CytA | Adoxophyes spp. |
| CytA | Agrotis spp. |
| CytA | Alabama argiilaceae |
| CytA | Anticarsia gemmatalis |
| CytA | Chilo spp. |
| CytA | Clysia ambiguella |
| CytA | Crocidolomia binotaiis |
| CytA | Cydia spp. |
| CytA | Diparopsis castanea |
| CytA | Earias spp. |
| CytA | Ephestia spp. |
| CytA | Heliothis spp. |
| CytA | Hellula undalis |
| CytA | Keiferia lycopersicella |
| CytA | Leucoptera scitelia |
| CytA | Lithocollethis spp. |
| CytA | Lobesia botrana |
| CytA | Ostrinia nubilalis |
| CytA | Pandemis spp. |
| CytA | Pectinophora gossyp. |
| CytA | Phyllocnistis citrella |
| CytA | Pieris spp. |
| CytA | Plutella xylostella |
| CytA | Scirpophaga spp. |
| CytA | Sesamia spp. |
| CytA | Sparganothis spp. |
| CytA | Spodoptera spp. |
| CytA | Tortrix spp. |
| CytA | Trichoplusia ni |
| CytA | Agriotes spp. |
| CytA | Anthonomus grandis |
| CytA | Curculio spp. |
| CytA | Diabrotica balteata |
| CytA | Leptinotarsa spp. |
| CytA | Lissorhoptrus spp. |
| CytA | Otiorhynchus spp. |
| CytA | Aleurothrixus spp. |
| CytA | Aleyrodes spp. |
| CytA | Aonidielia spp. |
| CytA | Aphididae spp. |
| CytA | Aphis spp. |
| CytA | Bemisia tabaci |
| CytA | Empoasca spp. |
| CytA | Mycus spp. |
| CytA | Nephotettix spp. |
| CytA | Nilaparvata spp. |
| CytA | Pseudococcus spp. |
| CytA | Psylla spp. |
| CytA | Quadraspidiotus spp. |
| CytA | Schizaphis spp. |
| CytA | Trialeurodes spp. |
| CytA | Lyriomyza spp. |
| CytA | Oscinella spp. |
| CytA | Phorbia spp. |
| CytA | Frankliniella spp. |
| CytA | Thrips spp. |
| CytA | Scirtothrips aurantii |
| CytA | Aceria spp. |
| CytA | Acutus spp. |
| CytA | Brevipalpus spp. |
| CytA | Panonychus spp. |
| CytA | Phyllocoptruta spp. |
| CytA | Tetranychus spp. |
| CytA | Heterodera spp. |
| CytA | Meloidogyne spp. |
| VIP3 | Adoxophyes spp. |
| VIP3 | Agrotis spp. |
| VIP3 | Alabama argillaceae |
| VIP3 | Anticarsia gemmatalis |
| VIP3 | Chilo spp. |
| VIP3 | Clysia ambiguella |
| VIP3 | Crocidolomia binotalis |
| VIP3 | Cydia spp. |
| VIP3 | Diparopsis castanea |
| VIP3 | Earias spp. |
| VIP3 | Ephestia spp. |
| VIP3 | Heliothis spp. |
| VIP3 | Hellula undalis |
| VIP3 | Keiferia |
| | lycopersicella |
| VIP3 | Leucoptera scitella |
| VIP3 | Lithocollethis spp. |
| VIP3 | Lobesia botrana |
| VIP3 | Ostrinia nubilalis |
| VIP3 | Pandemis spp. |
| VIP3 | Pectinophora gossyp. |
| VIP3 | Phyllocnistis citrella |
| VIP3 | Pieris spp. |
| VIP3 | Piutella xylostella |
| VIP3 | Scirpophaga spp. |
| VIP3 | Sesamia spp. |
| VIP3 | Sparganothis spp. |
| VIP3 | Spodoptera spp. |
| VIP3 | Tortrix spp. |
| VIP3 | Trichoplusia ni |
| VIP3 | Agriotes spp. |
| VIP3 | Anthonomus grandis |
| VIP3 | Curculio spp. |
| VIP3 | Diabrotica balteata |
| VIP3 | Leptinotarsa spp. |
| VIP3 | Lissorhoptrus spp. |
| VIP3 | Otiorhynchus spp. |
| VIP3 | Aleurothrixus spp. |
| VIP3 | Aleyrodes spp. |
| VIP3 | Aonidiella spp. |
| VIP3 | Aphididae spp. |
| VIP3 | Aphis spp. |
| VIP3 | Bemisia tabaci |
| VIP3 | Empoasca spp. |
| VIP3 | Mycus spp. |
| VIP3 | Nephotettix spp. |
| VIP3 | Niiaparvata spp. |
| VIP3 | Pseudococcus spp. |
| VIP3 | Psylla spp. |
| VIP3 | Quadraspidiotus spp. |
| VIP3 | Schizaphis spp. |
| VIP3 | Trialeurodes spp. |
| VIP3 | Lyriomyza spp. |
| VIP3 | Oscinella spp. |
| VIP3 | Phorbia spp. |
| VIP3 | Frankliniella spp. |
| VIP3 | Thrips spp. |
| VIP3 | Scirtothrips aurantii |
| VIP3 | Aceria spp. |
| VIP3 | Acutus spp. |
| VIP3 | Brevipalpus spp. |
| VIP3 | Panonychus spp. |
| VIP3 | Phyllocoptruta spp. |
| VIP3 | Tetranychus spp. |
| VIP3 | Heterodera spp. |
| VIP3 | Meloidogyne spp. |
| GL | Adoxophyes spp. |
| GL | Agrotis spp. |
| GL | Alabama argillaceae |
| GL | Anticarsia gemmatalis |
| GL | Chilo spp. |
| GL | Clysia ambiguella |
| GL | Crocidolomia binotaiis |
| GL | Cydia spp. |
| GL | Diparopsis castanea |
| GL | Earias spp. |
| GL | Ephestia spp. |
| GL | Heliothis spp. |
| GL | Hellula undalis |
| GL | Keiferia lycopersicella |
| GL | Leucoptera scitella |
| GL | Lithocollethis spp. |
| GL | Lobesia botrana |
| GL | Ostrinia nubilalis |
| GL | Pandemis spp. |
| GL | Pectinophora gossyp. |
| GL | Phyliocnistis citrella |
| GL | Pieris spp. |
| GL | Plutella xylostella |
| GL | Scirpophaga spp. |
| GL | Sesamia spp. |
| GL | Sparganothis spp. |
| GL | Spodoptera spp. |
| GL | Tortrix spp. |
| GL | Trichoplusia ni |
| GL | Agriotes spp. |
| GL | Anthonomus grandis |
| GL | Curculio spp. |
| GL | Diabrotica balteata |
| GL | Leptinotarsa spp. |
| GL | Lissorhoptrus spp. |
| GL | Otiorhynchus spp. |
| GL | Aleurothrixus spp. |
| GL | Aleyrodes spp. |
| GL | Aonidiella spp. |
| GL | Aphididae spp. |
| GL | Aphis spp. |
| GL | Bemisia tabaci |
| GL | Empoasca spp. |
| GL | Mycus spp. |
| GL | Nephotettix spp. |
| GL | Nilaparvata spp. |
| GL | Pseudococcus spp. |
| GL | Psylia spp. |
| GL | Quadraspidiotus spp. |
| GL | Schizaphis spp. |
| GL | Trialeurodes spp. |
| GL | Lyriomyza spp. |
| GL | Oscinella spp. |
| GL | Phorbia spp. |
| GL | Frankliniella spp. |
| GL | Thrips spp. |
| GL | Scirtothrips aurantii |
| GL | Aceria spp. |
| GL | Aculus spp. |
| GL | Brevipalpus spp. |
| GL | Panonychus spp. |
| GL | Phyliocoptruta spp. |
| GL | Tetranychus spp. |
| GL | Heterodera spp. |
| GL | Meioidogyne spp. |
| PL | Adoxophyesspp. |
| PL | Agrotis spp. |
| PL | Alabama argillaceae |
| PL | Anticarsia gemmatalis |
| PL | Chilo spp. |
| PL | Clysia ambiguella |
| PL | Crocidolomia binotalis |
| PL | Cydia spp. |
| PL | Diparopsis castanea |
| PL | Earias spp. |
| PL | Ephestia spp. |
| PL | Heliothis spp. |
| PL | Hellula undaiis |
| PL | Keiferia lycopersicella |
| PL | Leucoptera scitella |
| PL | Lithocollethis spp. |
| PL | Lobesia botrana |
| PL | Ostrinia nubilalis |
| PL | Pandemis spp. |
| PL | Pectinophora gossyp. |
| PL | Phyllocnistis citrella |
| PL | Pieris spp. |
| PL | Plutella xylostella |
| PL | Scirpophaga spp. |
| PL | Sesamia spp. |
| PL | Sparganothis spp. |
| PL | Spodoptera spp. |
| PL | Tortrix spp. |
| PL | Trichoplusia ni |
| PL | Agriotes spp. |
| PL | Anthonomus grandis |
| PL | Curculio spp. |
| PL | Diabrotica balteata |
| PL | Leptinotarsa spp. |
| PL | Lissorhoptrus spp. |
| PL | Otiorhynchus spp. |
| PL | Aleurothrixus spp. |
| PL | Aleyrodes spp. |
| PL | Aonidiella spp. |
| PL | Aphididae spp. |
| PL | Aphis spp. |
| PL | Bemisia tabaci |
| PL | Empoasca spp. |
| PL | Mycus spp. |
| PL | Nephotettix spp. |
| PL | Nilaparvata spp. |
| PL | Pseudococcus spp. |
| PL | Psylla spp. |
| PL | Quadraspidiotus spp. |
| PL | Schizaphis spp. |
| PL | Trialeurodes spp. |
| PL | Lyriomyza spp. |
| PL | Oscinella spp. |
| PL | Phorbia spp. |
| PL | Franklinieila spp. |
| PL | Thrips spp. |
| PL | Scirtothrips auranii |
| PL | Aceria spp. |
| PL | Aculus spp. |
| PL | Brevipalpus spp. |
| PL | Panonychus spp. |
| PL | Phyllocoptruta spp. |
| PL | Tetranychus spp. |
| PL | Heterodera spp. |
| PL | Meloidogyne spp. |
| XN | Adoxophyes spp. |
| XN | Agrotis spp. |
| XN | Alabama argiliaceae |
| XN | Anticarsia gemmatalis |
| XN | Chilo spp. |
| XN | Clysia ambiguella |
| XN | Crocidolomia binotalis |
| XN | Cydia spp. |
| XN | Diparopsis castanea |
| XN | Earias spp. |
| XN | Ephestia spp. |
| XN | Heliothis spp. |
| XN | Helluia undaiis |
| XN | Keiferia lycopersicella |
| XN | Leucoptera scitella |
| XN | Lithocollethis spp. |
| XN | Lobesia botrana |
| XN | Ostrinia nubilalis |
| XN | Pandemis spp. |
| XN | Pectinophora gossyp. |
| XN | Phyllocnistis citrella |
| XN | Pieris spp. |
| XN | Plutella xylostella |
| XN | Scirpophaga spp. |
| XN | Sesamia spp. |
| XN | Sparganothis spp. |
| XN | Spodoptera spp. |
| XN | Tortrix spp. |
| XN | Trichoplusia ni |
| XN | Agriotes spp. |
| XN | Anthonomus grandis |
| XN | Curculio spp. |
| XN | Diabrotica balteata |
| XN | Leptinotarsa spp. |
| XN | Lissorhoptrus spp. |
| XN | Otiorhynchus spp. |
| XN | Aleurothrixus spp. |
| XN | Aleyrodes spp. |
| XN | Aonidiella spp. |
| XN | Aphididae spp. |
| XN | Aphis spp. |
| XN | Bemisia tabaci |
| XN | Empoasca spp. |
| XN | Mycus spp. |
| XN | Nephotettix spp. |
| XN | Nilaparvata spp. |
| XN | Pseudococcus spp. |
| XN | Psylla spp. |
| XN | Quadraspidiotus spp. |
| XN | Schizaphis spp. |
| XN | Trialeurodes spp. |
| XN | Lyriomyza spp. |
| XN | Oscinella spp. |
| XN | Phorbia spp. |
| XN | Frankliniella spp. |
| XN | Thrips spp. |
| XN | Scirtothrips aurantii |
| XN | Aceria spp. |
| XN | Aculus spp. |
| XN | Brevipalpus spp. |
| XN | Panonychus spp. |
| XN | Phyllocoptruta spp. |
| XN | Tetranychus spp. |
| XN | Heterodera spp. |
| XN | Meloidogyne spp. |
| Plnh. | Adoxophyes spp. |
| Plnh. | Agrotis spp. |
| Plnh. | Alabama argiliaceae |
| Plnh. | Anticarsia gemmatalis |
| Plnh. | Chilo spp. |
| Plnh. | Clysia ambiguella |
| Plnh. | Crocidolomia |
| | binotalis |
| Plnh. | Cydia spp. |
| Plnh. | Diparopsis castanea |
| Plnh. | Earias spp. |
| Plnh. | Ephestia spp. |
| Plnh. | Heliothis spp. |
| Plnh. | Heliuia undalis |
| Plnh. | Keiferia lycopersicella |
| Plnh. | Leucoptera scitella |
| Plnh. | Lithocollethis spp. |
| Plnh. | Lobesia botrana |
| Plnh. | Ostrinia nubilalis |
| Plnh. | Pandemis spp. |
| Plnh. | Pectinophora gossyp. |
| Plnh. | Phyllocnistis citrelia |
| Plnh. | Pieris spp. |
| Plnh. | Plutella xylostella |
| Plnh. | Scirpophaga spp. |
| Plnh. | Sesamia spp. |
| Plnh. | Sparganothis spp. |
| Plnh. | Spodoptera spp. |
| Plnh. | Tortrix spp. |
| Plnh. | Trichoplusia ni |
| Plnh. | Agriotes spp. |
| Plnh. | Anthonomus grandis |
| Plnh. | Curculio spp. |
| Plnh. | Diabrotica balteata |
| Plnh. | Leptinotarsa spp. |
| Plnh. | Lissorhoptrus spp. |
| Plnh. | Otiorhynchus spp. |
| Plnh. | Aleurothrixus spp. |
| Plnh. | Aleyrodes spp. |
| Plnh. | Aonidiella spp. |
| Plnh. | Aphididae spp. |
| Plnh. | Aphis spp. |
| Plnh. | Bemisia tabaci |
| Plnh. | Empoasca spp. |
| Plnh. | Mycus spp. |
| Plnh. | Nephotettix spp. |
| Plnh. | Nilaparvata spp. |
| Plnh. | Pseudococcus spp. |
| Plnh. | Psylla spp. |
| Plnh. | Quadraspidiotus spp. |
| Plnh. | Schizaphis spp. |
| Plnh. | Trialeurodes spp. |
| Plnh. | Lyriomyza spp. |
| Plnh. | Oscinella spp. |
| Plnh. | Phorbia spp. |
| Plnh. | Frankliniella spp. |
| Plnh. | Thrips spp. |
| Plnh. | Scirtothrips aurantii |
| Plnh. | Aceria spp. |
| Plnh. | Acutus spp. |
| Plnh. | Brevipalpus spp. |
| Plnh. | Panonychus spp. |
| Plnh. | Phyllocoptruta spp. |
| Plnh. | Tetranychus spp. |
| Plnh. | Heterodera spp. |
| Plnh. | Meloidogyne spp. |
| PLec. | Adoxophyes spp. |
| PLec. | Agrotis spp. |
| PLec. | Alabama argillaceae |
| PLec. | Anticarsia gemmatalis |
| PLec. | Chilo spp. |
| PLec. | Clysia ambiguella |
| PLec. | Crocidolomia binotalis |
| PLec. | Cydia spp. |
| PLec. | Diparopsis castanea |
| PLec. | Earias spp. |
| PLec. | Ephestia spp. |
| PLec. | Heliothis spp. |
| PLec. | Hellula undalis |
| PLec. | Keiferia lycopersicella |
| PLec. | Leucoptera scitella |
| PLec. | Lithocollethis spp. |
| PLec. | Lobesia botrana |
| PLec. | Ostrinia nubilalis |
| PLec. | Pandemis spp. |
| PLec. | Pectinophora gossyp. |
| PLec. | Phyllocnistis citrella |
| PLec. | Pieris spp. |
| PLec. | Plutella xylostella |
| PLec. | Scirpophaga spp. |
| PLec. | Sesamia spp. |
| PLec. | Sparganothis spp. |
| PLec. | Spodoptera spp. |
| PLec. | Tortrix spp. |
| PLec. | Trichoplusia ni |
| PLec. | Agriotes spp. |
| PLec. | Anthonomus grandis |
| PLec. | Curculio spp. |
| PLec. | Diabrotica balteata |
| PLec. | Leptinotarsa spp. |
| PLec. | Lissorhoptrus spp. |
| PLec. | Otiorhynchus spp. |
| PLec. | Aleurothrixus spp. |
| PLec. | Aleyrodes spp. |
| PLec. | Aonidiella spp. |
| PLec. | Aphididae spp. |
| PLec. | Aphis spp. |
| PLec. | Bemisia tabaci |
| PLec. | Empoasca spp. |
| PLec. | Mycus spp. |
| PLec. | Nephotettix spp. |
| PLec. | Nilaparvata spp. |
| PLec. | Pseudococcus spp. |
| PLec. | Psylia spp. |
| PLec. | Quadraspidiotus spp. |
| PLec. | Schizaphis spp. |
| PLec. | Trialeurodes spp. |
| PLec. | Lyriomyza spp. |
| PLec. | Oscinella spp. |
| PLec. | Phorbia spp. |
| PLec. | Frankliniella spp. |
| PLec. | Thrips spp. |
| PLec. | Scirtothnps aurantii |
| PLec. | Aceria spp. |
| PLec. | Aculus spp. |
| PLec. | Brevipalpus spp. |
| PLec. | Panonychus spp. |
| PLec. | Phyllocoptruta spp. |
| PLec. | Tetranychus spp. |
| PLec. | Heterodera spp. |
| PLec. | Meloidogyne spp. |
| Aggl. | Adoxophyes spp. |
| Aggl. | Agrotis spp. |
| Aggl. | Alabama |
| | argillaceae |
| Aggl. | Anticarsia gemmatalis |
| Aggl. | Chilo spp. |
| Aggl. | Clysia ambiguella |
| Aggl. | Crocidolomia |
| | binotalis |
| Aggl. | Cydia spp. |
| Aggl. | Diparopsis |
| | castanea |
| Aggl. | Earias spp. |
| Aggl. | Ephestia spp. |
| Aggl. | Heliothis spp. |
| Aggl. | Hellula undalis |
| Aggl. | Keiferia |
| | lycopersicella |
| Aggl. | Leucoptera scitella |
| Aggl. | Lithocollethis spp. |
| Aggl. | Lobesia botrana |
| Aggl. | Ostrinia nubilalis |
| Aggl. | Pandemis spp. |
| Aggl. | Pectinophora |
| | gossyp. |
| Aggl. | Phyllocnistis citrella |
| Aggl. | Pieris spp. |
| Aggl. | Plutiia xylostella |
| Aggl. | Scirpophaga spp. |
| Aggl. | Sesamia spp. |
| Aggl. | Sparganothis spp. |
| Aggl. | Spodoptera spp. |
| Aggl. | Tortrix spp. |
| Aggl. | Trichoplusia ni |
| Aggl. | Agriotes spp. |
| Aggl. | Anthonomus grandis |
| Aggl. | Curculio spp. |
| Aggl. | Diabrotica balteata |
| Aggl. | Leptinotarsa spp. |
| Aggl. | Lissorhoptrus spp. |
| Aggl. | Otiorhynchus spp. |
| Aggl. | Aleurothrixus spp. |
| Aggl. | Aleyrodes spp. |
| Aggl. | Aonidiella spp. |
| Aggl. | Aphididae spp. |
| Aggl. | Aphis spp. |
| Aggl. | Bemisia tabaci |
| Aggl. | Empoasca spp. |
| Aggl. | Mycus spp. |
| Aggl. | Nephotettix spp. |
| Aggl. | Nilaparvata spp. |
| Aggl. | Pseudococcus spp. |
| Aggl. | Psylla spp. |
| Aggl. | Quadraspidiotus spp. |
| Aggl. | Schizaphis spp. |
| Aggl. | Trialeurodes spp. |
| Aggl. | Lyriomyza spp. |
| Aggl. | Oscinella spp. |
| Aggl. | Phorbia spp. |
| Aggl. | Frankliniella spp. |
| Aggl. | Thrips spp. |
| Aggl. | Scirtothrips auranti |
| Aggl. | Aceria spp. |
| Aggl. | Aculus spp. |
| Aggl. | Brevipalpus spp. |
| Aggl. | Panonychus spp. |
| Aggl. | Phyllocoptruta spp |
| Aggl. | Tetranychus spp. |
| Aggl. | Heterodera spp. |
| Aggl. | Meloidogyne spp. |
| CO | Adoxophyes spp. |
| CO | Agrotis spp. |
| CO | Alabama argiliaceae |
| CO | Anticarsia gemmatalis |
| CO | Chilo spp. |
| CO | Ciysia ambiguella |
| CO | Crocidolomia binotalis |
| CO | Cydia spp. |
| CO | Diparopsis castanea |
| CO | Earias spp. |
| CO | Ephestia spp. |
| CO | Heliothis spp. |
| CO | Hellula undalis |
| CO | Keiferia lycopersicella |
| CO | Leucoptera scitella |
| CO | Lithocollethis spp. |
| CO | Lobesia botrana |
| CO | Ostrinia nubilalis |
| CO | Pandemis spp. |
| CO | Pectinophora gossyp. |
| CO | Phyllocnistis citrella |
| CO | Pieris spp. |
| CO | Plutella xylostella |
| CO | Scirpophaga spp. |
| CO | Sesamia spp. |
| CO | Sparganothis spp. |
| CO | Spodoptera spp. |
| CO | Tortrix spp. |
| CO | Trichoplusia ni |
| CO | Agriotes spp. |
| CO | Anthonomus grandis |
| CO | Curculio spp. |
| CO | Diabrotica balteata |
| CO | Leptinotarsa spp. |
| CO | Lissorhoptrus spp. |
| CO | Otiorhynchus spp. |
| CO | Aleurothrixus spp. |
| CO | Aleyrodes spp. |
| CO | Aonidielia spp. |
| CO | Aphididae spp. |
| CO | Aphis spp. |
| CO | Bemisia tabaci |
| CO | Empoasca spp. |
| CO | Mycus spp. |
| CO | Nephotettix spp. |
| CO | Nilaparvata spp. |
| CO | Pseudococcus spp. |
| CO | Psylla spp. |
| CO | Quadraspidiotus spp. |
| CO | Schizaphis spp. |
| CO | Trialeurodes spp. |
| CO | Lyriomyza spp. |
| CO | Oscinella spp. |
| CO | Phorbia spp. |
| CO | Frankliniella spp. |
| CO | Thrips spp. |
| CO | Scirtothrips aurantii |
| CO | Aceria spp. |
| CO | Acutus spp. |
| CO | Brevipalpus spp. |
| CO | Panonychus spp. |
| CO | Phyllocoptruta spp. |
| CO | Tetranychus spp. |
| CO | Heterodera spp. |
| CO | Meloidogyne spp. |
| CH | Adoxophyes spp. |
| CH | Agrotis spp. |
| CH | Alabama argillaceae |
| CH | Anticarsia |
| | gemmatalis |
| CH | Chilo spp. |
| CH | Clysia ambiguella |
| CH | Crocidolomia binotalis |
| CH | Cydia spp. |
| CH | Diparopsis castanea |
| CH | Earias spp. |
| CH | Ephestia spp. |
| CH | Heliothis spp. |
| CH | Hellula undalis |
| CH | Keiferia lycopersicella |
| CH | Leucoptera scitella |
| CH | Lithocollethis spp. |
| CH | Lobesia botrana |
| CH | Ostrinia nubilalis |
| CH | Pandemis spp. |
| CH | Pectinophora gossyp. |
| CH | Phyllocnistis citrella |
| CH | Pieris spp. |
| CH | Plutella xylostella |
| CH | Scirpophaga spp. |
| CH | Sesamia spp. |
| CH | Sparganothis spp. |
| CH | Spodoptera spp. |
| CH | Tortrix spp. |
| CH | Trichoplusia ni |
| CH | Agriotes spp. |
| CH | Anthonomus |
| | grandis |
| CH | Curculio spp. |
| CH | Diabrotica balteata |
| CH | Leptinotarsa spp. |
| CH | Lissorhoptrus spp. |
| CH | Otiorhynohus spp. |
| CH | Aleurothrixus spp. |
| CH | Aleyrodes spp. |
| CH | Aonidiella spp. |
| CH | Aphididae spp. |
| CH | Aphis spp. |
| CH | Bemisia tabaci |
| CH | Empoasca spp. |
| CH | Mycus spp. |
| CH | Nephotettix spp. |
| CH | Nilaparvata spp. |
| CH | Pseudococcus spp. |
| CH | Psylla spp. |
| CH | Quadraspidiotus spp. |
| CH | Schizaphis spp. |
| CH | Trialeurodes spp. |
| CH | Lyriomyza spp. |
| CH | Oscinella spp. |
| CH | Phorbia spp. |
| CH | Frankliniella spp. |
| CH | Thrips spp. |
| CH | Scirtothrips aurantii |
| CH | Aceria spp. |
| CH | Aculus spp. |
| CH | Brevipalpus spp. |
| CH | Panonychus spp. |
| CH | Phyllocoptruta spp. |
| CH | Tetranychus spp. |
| CH | Heterodera spp. |
| CH | Meloidogyne spp. |
| SS | Adoxophyes spp. |
| SS | Agrotis spp. |
| SS | Alabama argillaceae |
| SS | Anticarsia gemmatalis |
| SS | Chilo spp. |
| SS | Clysia ambiguella |
| SS | Crocidolomia binotalis |
| SS | Cydia spp. |
| SS | Diparopsis castanea |
| SS | Earias spp. |
| SS | Ephestia spp. |
| SS | Heliothis spp. |
| SS | Hellula undalis |
| SS | Keiferia lycopersicella |
| SS | Leucoptera scitella |
| SS | Lithocollethis spp. |
| SS | Lobesia botrana |
| SS | Ostrinia nubilalis |
| SS | Pandemis spp. |
| SS | Pectinophora gossyp. |
| SS | Phyllocnistis citrella |
| SS | Pieris spp. |
| SS | Plutella xylostella |
| SS | Scirpophaga spp. |
| SS | Sesamia spp. |
| SS | Sparganothis spp. |
| SS | Spodoptera spp. |
| SS | Tortrix spp. |
| SS | Trichopiusia ni |
| SS | Agriotes spp. |
| SS | Anthonomus grandis |
| SS | Curculio spp. |
| SS | Diabrotica balteata |
| SS | Leptinotarsa spp. |
| SS | Lissorhoptrus spp. |
| SS | Otiorhynchus spp. |
| SS | Aleurothrixus spp. |
| SS | Aleyrodes spp. |
| SS | Aonidielia spp. |
| SS | Aphididae spp. |
| SS | Aphis spp. |
| SS | Bemisia tabaci |
| SS | Empoasca spp. |
| SS | Mycus spp. |
| SS | Nephotettix spp. |
| SS | Nilaparvata spp. |
| SS | Pseudococcus spp. |
| SS | Psylla spp. |
| SS | Quadraspidiotus spp. |
| SS | Schizaphis spp. |
| SS | Trialeurodes spp. |
| SS | Lyriomyza spp. |
| SS | Oscinella spp. |
| SS | Phorbia spp. |
| SS | Frankliniella spp. |
| SS | Thrips spp. |
| SS | Scirtothrips aurantii |
| SS | Aceria spp. |
| SS | Aculus spp. |
| SS | Brevipalpus spp. |
| SS | Panonychus spp. |
| SS | Phyllocoptruta spp. |
| SS | Tetranychus spp. |
| SS | Heterodera spp. |
| SS | Meloidogyne spp. |
| HO | Adoxophyes spp. |
| HO | Agrotis spp. |
| HO | Alabama argillaceae |
| HO | Anticarsia gemmatalis |
| HO | Chilo spp. |
| HO | Clysia ambiguella |
| HO | Crocidolomia binotalis |
| HO | Cydia spp. |
| HO | Diparopsis castanea |
| HO | Earias spp. |
| HO | Ephestia spp. |
| HO | Heliothis spp. |
| HO | Hellula undalis |
| HO | Keiferia lycopersicella |
| HO | Leucoptera scitella |
| HO | Lithocollethis spp. |
| HO | Lobesia botrana |
| HO | Ostrinia nubilalis |
| HO | Pandemis spp. |
| HO | Pectinophora gossypiella |
| HO | Phyllocnistis citrella |
| HO | Pieris spp. |
| HO | Plutella xylostella |
| HO | Scirpophaga spp. |
| HO | Sesamia spp. |
| HO | Sparganothis spp. |
| HO | Spodoptera spp. |
| HO | Tortrix spp. |
| HO | Trichoplusia ni |
| HO | Agriotes spp. |
| HO | Anthonomus grandis |
| HO | Curculio spp. |
| HO | Diabrotica balteata |
| HO | Leptinotarsa spp. |
| HO | Lissorhoptrus spp. |
| HO | Otiorhynchus spp. |
| HO | Aleurothrixus spp. |
| HO | Aleyrodes spp. |
| HO | Aonidiella spp. |
| HO | Aphididae spp. |
| HO | Aphis spp. |
| HO | Bemisia tabaci |
| HO | Empoasca spp. |
| HO | Mycus spp. |
| HO | Nephotettix spp. |
| HO | Nilaparvata spp. |
| HO | Pseudococcus spp. |
| HO | Psylla spp. |
| HO | Quadraspidiotus spp. |
| HO | Schizaphis spp. |
| HO | Trialeurodes spp. |
| HO | Lyriomyza spp. |
| HO | Oscinella spp. |
| HO | Phorbia spp. |
| HO | Frankliniella spp. |
| HO | Thrips spp. |
| HO | Scirtothrips aurantii |
| HO | Aceria spp. |
| HO | Acutus spp. |
| HO | Brevipalpus spp. |
| HO | Panonychus spp. |
| HO | Phyllocoptruta spp. |
| HO | Tetranychus spp. |
| HO | Heterodera spp. |
| HO | Meloidogyne spp. |

**Tabelle 3:**

| | | |
|---|---|---|
| Abkürzungen: | | |
| Acetyl-CoA-Carboxylase: ACCase | | |
| Acetolactatsynthase: ALS | | |
| Hydroxyphenylpyruvat-Dioxygenase: HPPD | | |
| Hemmung der Proteinsynthese: IPS | | |
| Hormonimitation: HO | | |
| Glutaminsynthetase: GS | | |
| Protoporphyrinogenoxidase: PROTOX | | |
| 5-Enolpyruvyl-3-Phosphoshikimat-Synthase: EPSPS | | |

| Prinzip | Toleranz gegenüber | Pflanze |
|---|---|---|
| ALS | Sulfonylharnstoffverbin-dungen usw.*** | Baumwolle |
| ALS | Sulfonylharnstoffverbin-dungen usw. *** | Reis |
| ALS | Sulfonylharnstoffverbin-dungen usw. *** | Brassica |
| ALS | Sulfonylharnstoffverbin-dungen usw. *** | Kartoffeln |
| ALS | Sulfonylharnstoffverbin-dungen usw. *** | Tomaten |
| ALS | Sulfonylharnstoffverbin-dungen usw. *** | Kürbis |
| ALS | Sulfonylharnstoffverbin-dungen usw. *** | Sojabohnen |
| ALS | Sulfonylharnstoffverbin-dungen usw. *** | Mais |
| ALS | Sulfonylharnstoffverbin-dungen usw. *** | Weizen |
| ALS | Sulfonylharnstoffver-bindungen usw. *** | Kernobst |
| ALS | Sulfonylharnstoffver-bindungen usw. *** * | Steinobst |
| ALS | Sulfonylharnstoffver-bindungen usw. *** * | Zitrus |
| ACCase | +++ | Baumwolle |
| ACCase | +++ | Reis |
| ACCase | +++ | Brassica |
| ACCase | +++ | Kartoffel |
| ACCase | +++ | Tomaten |
| ACCase | +++ | Kürbis |
| ACCase | +++ | Sojabohnen |
| ACCase | +++ | Mais |
| ACCase | +++ | Weizen |
| ACCase | +++ | Kernobst |
| ACCase | +++ | Steinobst |
| ACCase | +++ | Zitrus |
| HPPD | Isoxaflutol, Isoxachlotol, Sulcotrion, Mesotrion | Baumwolle |
| HPPD | Isoxaflutol, Isoxachlotol, Sulcotrion, Mesotrion | Reis |
| HPPD | Isoxaflutol, Isoxachlotol, Sulcotrion, Mesotrion | Brassica |
| HPPD | Isoxaflutol, Isoxachlotol, Sulcotrion, Mesotrion | Kartoffeln |
| HPPD | Isoxaflutol, Isoxachlotol, Sulcotrion, Mesotrion | Tomaten |
| HPPD | Isoxaflutol, Isoxachlotol, Sulcotrion, Mesotrion | Kürbis |
| HPPD | Isoxaflutol, Isoxachlotol, Sulcotrion, Mesotrion | Sojabohnen |
| HPPD | Isoxaflutol, Isoxachlotol, Sulcotrion, Mesotrion | Mais |
| HPPD | Isoxaflutol, Isoxachlotol, Sulcotrion, Mesotrion | Weizen |
| HPPD | Isoxaflutol, Isoxachlotol, Sulcotrion, Mesotrion | Kernobst |
| HPPD | Isoxaflutol, Isoxachlotol, Sulcotrion, Mesotrion | Steinobst |
| HPPD | Isoxaflutol, Isoxachlotol, Sulcotrion, Mesotrion | Zitrus |
| Nitrilase | Bromoxynil, Loxynil | Baumwolle |
| Nitrilase | Bromoxynil, Loxynil | Reis |
| Nitrilase | Bromoxynil, Loxynil | Brassica |
| Nitrilase | Bromoxynil, Loxynil | Kartoffeln |
| Nitrilase | Bromoxynil, Loxynil | Tomaten |
| Nitrilase | Bromoxynil, Loxynil | Kürbis |
| Nitrilase | Bromoxynil, Loxynil | Sojabohnen |
| Nitrilase | Bromoxynil, Loxynil | Mais |
| Nitrilase | Bromoxynil, Loxynil | Weizen |
| Nitrilase | Bromoxynil, Loxynil | Kernobst |
| Nitrilase | Bromoxynil, Loxynil | Steinobst |
| Nitrilase | Bromoxynil, Loxynil | Zitrus |
| IPS | Chloroactanilide &&& | Baumwolle |
| IPS | Chloroactanilide &&& | Reis |
| IPS | Chloroactanilid &&& | Brassica |
| IPS | Chloroactanilide &&& | Kartoffeln |
| IPS | Chloroactanilide &&& | Tomaten |
| IPS | Chloroactanilide &&& | Kürbis |
| IPS | Chloroactanilide &&& | Sojabohnen |
| IPS | Chloroactanilide &&& | Mais |
| IPS | Chloroactanilide &&& | Weizen |
| IPS | Chloroactanilide &&& | Kernobst |
| IPS | Chloroactanilide &&& | Steinobst |
| IPS | Chloroactanilide &&& | Zitrus |
| HOM | 2,4-D, Mecoprop-P | Baumwolle |
| HOM | 2,4-D, Mecoprop-P | Reis |
| HOM | 2,4-D, Mecoprop-P | Brassica |
| HOM | 2,4-D, Mecoprop-P | Kartoffeln |
| HOM | 2,4-D, Mecoprop-P | Tomaten |
| HOM | 2,4-D, Mecoprop-P | Kürbis |
| HOM | 2,4-D, Mecoprop-P | Sojabohnen |
| HOM | 2,4-D, Mecoprop-P | Mais |
| HOM | 2,4-D, Mecoprop-P | Weizen |
| HOM | 2,4-D, Mecoprop-P | Kernobst |
| HOM | 2,4-D, Mecoprop-P | Steinobst |
| HOM | 2,4-D, Mecoprop-P | Zitrus |
| PROTOX | Protox-Inhibitoren /// | Baumwolle |
| PROTOX | Protox-Inhibitoren /// | Reis |
| PROTOX | Protox-Inhibitoren /// | Brassica |
| PROTOX | Protox-Inhibitoren /// | Kartoffeln |
| PROTOX | Protox-Inhibitoren /// | Tomaten |
| PROTOX | Protox-Inhibitoren /// | Kürbis |
| PROTOX | Protox-Inhibitoren /// | Sojabohnen |
| PROTOX | Protox-Inhibitoren /// | Mais |
| PROTOX | Protox-Inhibitoren /// | Weizen |
| PROTOX | Protox-Inhibitoren /// | Kernobst |
| PROTOX | Protox-Inhibitoren /// | Steinobst |
| PROTOX | Protox-Inhibitoren /// | Zitrus |
| EPSPS | Glyphosat und/oder Sulphosat | Baumwolle |
| EPSPS | Glyphosat und/oder Sulphosat | Reis |
| EPSPS | Glyphosat und/oder Sulphosat | Brassica |
| EPSPS | Glyphosat und/oder Sulphosat | Kartoffeln |
| EPSPS | Glyphosat und/oder Sulphosat | Tomaten |
| EPSPS | Glyphosat und/oder Sulphosat | Kürbis |
| EPSPS | Glyphosat und/oder Sulphosat | Sojabohnen |
| EPSPS | Glyphosat und/oder Sulphosat | Mais |
| EPSPS | Glyphosat und/oder Sulphosat | Weizen |
| EPSPS | Glyphosat und/oder Sulphosat | Kernobst |
| EPSPS | Glyphosat und/oder Sulphosat | Steinobst |
| EPSPS | Glyphosat und/oder Sulphosat | Zitrus |
| GS | Gluphosinat und/oder Bialaphos | Baumwolle |
| GS | Gluphosinat und/oder Bialaphos | Reis |
| GS | Gluphosinat und/oder Bialaphos | Brassica |
| GS | Gluphosinat und/oder Bialaphos | Kartoffeln |
| GS | Gluphosinat und/oder Bialaphos | Tomaten |
| GS | Gluphosinat und/oder Bialaphos | Kürbis |
| GS | Gluphosinat und/oder Bialaphos | Sojabohnen |
| GS | Gluphosinat und/oder Bialaphos | Mais |
| GS | Gluphosinat und/oder Bialaphos | Weizen |
| GS | Gluphosinat und/oder Bialaphos | Kernobst |
| GS | Gluphosinat und/oder Bialaphos | Stein-obst |
| GS | Gluphosinat und/oder Bialaphos | Zitrus |

| | | |
|---|---|---|
| *** einbezogen sind Sulfonylharnstoffverbindungen, Imidazolinone, Triazolpyrimidine, Dimethoxypyrimidine und N-Acylsulfonamide: Sulfonylharnstoffverbindungen wie Chlorsulfuron, Chlorimuron, Ethamethsulfuron, Metsulfuron, Primisulfuron, Prosulfuron, Triasulfuron, Cinosulfuron, Trifusulfuron, Oxasulfuron, Bensulfuron, Tribenuron, ACC 322140, Fluzasulfuron, Ethoxysulfuron, Fluzasdulfuron, Nicosulfuron, Rimsulfuron, Thifensulfuron, Pyrazosulfuron, Clopyrasulfuron, NC 330, Azimsulfuron, Imazosulfuron, Sulfosulfuron, Amidosulfuron, Flupyrsulfuron, CGA 362622 Imidazolinone wie Imazamethabenz, Imazaquin, Imazamethypyr, Imazethapyr, Imazapyr und Imazamox; Triazolpyrimidine wie DE 511, Flumetsulam und Chloransulam; Dimethoxypyrimidine wie etwa Pyrithiobac, Pyriminobac, Bispyribac und Pyribenzoxim. +++ Tolerant gegenüber Diclofop-methyl, Fluazifop-P-butyl, Haloxyfop-P-methyl, Haloxyfop-P-ethyl, Quizalafop-P-ethyl, Clodinafop-propargyl, Fenoxaprop-ethyl, Tepraloxydim, Alloxydim, Sethoxydim, Cycloxydim, Cloproxydim, Tralkoxydim, Butoxydim, Caloxydim, Clefoxydim, Clethodim. &&& Chloroacetanilide wie etwa Alachlor, Acetochlor, Dimethenamid /// Protox-Inhibitoren: Zum Beispiel Diphenyether wie etwa Acifluorfen, Aclonifen, Bifenox, Chlornitrofen, Ethoxyfen, Fluoroglycofen, Fomesafen, Lactofen, Oxyfluorfen; Imide wie etwa Azafenidin, Carfentrazone-ethyl, Cinidon-ethyl, Flumiclorac-pentyl, Flumioxazin, Fluthiacet-methyl, Oxadiargyl, Oxadiazon, Pentoxazone, Sulfentrazone, Imide und andere Verbindungen wie etwa Flumipropyn, Flupropacil, Nipyraclofen and Thidiazimin; sowie Fluazola und Pyraflufen-ethyl. | | |

**Tabelle 4**

| Liste von Beispielen transgener Pflanzen mit modifizierten Eigenschaften: | |
|---|---|
| Transgene Pflanzen | Transgen modifizierte Eigenschaften |
| Dianthus caryophyllus (Nelke) | Verlängerte Haltbarkeit durch reduzierte Ethylen-Akkumulation aufgrund der Expression der ACC Synthase; Sulfonylharnstoff-Herbizid Toleranz |
| Linie 66 | |
| [Florigene Pty. Ltd.] | |
| | |
| Dianthus caryophyllus (Nelke) | Modifizierte Blütenfarbe; Sulfonylharnstoff-Herbizid Toleranz |
| Linien 4, 11, 15, 16 | |
| [Florigene Pty. Ltd.] | |
| | |
| Dianthus caryophyllus (Nelke) | Modifizierte Blütenfarbe; Sulfonylharnstoff-Herbizid Toleranz |
| Linien 959A, 988A, 1226A, 1351A, 1363A, 1400A | |
| [Florigene Pty. Ltd.] | |
| | |
| Brassica napus (Argentinischer Raps) | Modifizierter Fettsäuregehalt im Samen |
| Linien 23-18-17, 23-198 | |
| [Monsanto Company] | |
| | |
| Zea mays L. (Mais) | Erhöhter Lysingehalt |
| Linien REN-ØØØ38-3 (LY038) | |
| [Monsanto Company] | |
| | |
| Zea mays L. (Mais) | Erhöhter Lysingehalt, Resistenz gegen den Maiszünsler; |
| Linien REN-ØØØ38-3, MON-ØØ81Ø-6 (MON-ØØ81Ø-6 x LY038) | |
| [Monsanto Company] | |
| | |
| Cucumis melo (Melone) | Verzögerte Reife durch Expression der S-Adenosylmethionin Hydrolase |
| Linien A, B | |
| [Agritope Inc.] | |
| | |
| Carica papaya (Papaya) | Resistenz gegen den Papaya Ringspot Virus (PRSV) |
| Linien 55-1/63-1 | |
| [Cornell University] | |
| | |
| Solanum tuberosum L. (Kartoffel) | Resistenz gegen den Colorado Kartoffelkäfer und den Kartoffelblattrollvirus (PLRV) |
| Linien RBMT21-129, RBMT21-350, RBMT22-082 | |
| [Monsanto Company] | |
| | |
| Solanum tuberosum L. (Kartoffel) | Resistenz gegen den Colorado Kartoffelkäfer und den Kartoffelvirus Y (PVY) |
| Linien RBMT15-101, SEMT15-02, SEMT15-15 | |
| [Monsanto Company] | |
| | |
| Glycine max L. (Sojabohne) | Modifizierter Fettsäuregehalt im Samen, insbesondere erhöhter Ölsäuregehalt |
| Linien DD-Ø26ØØ5-3 (G94-1, G94-19, G168 | |
| [DuPont Canada Agricultural Products] | |
| | |
| Glycine max L. (Sojabohne) | Modifizierter Fettsäuregehalt im Samen, insbesondere reduzierter Linolensäuregehalt |
| Linien OT96-15 | |
| [Agriculture & Agri-Food Canada] | |
| Cucurbita pepo (Kürbis) | Resistenz gegen virale Infektionen, Wassermelonen Mosaik Virus (WMV) 2 und Zucchini Gelbmosaik Virus (ZYMV) |
| Linie ZW20 | |
| [Upjohn (USA); Seminis Vegetable Inc. (Canada)] | |
| | |
| Cucurbita pepo (Kürbis) | Resistenz gegen virale Infektionen, Gurken Mosaik Virus (CMV), Wassermelonen Mosaik Virus (WMV) 2 und Zucchini Gelbmosaik Virus (ZYMV) |
| Linie CZW-3 | |
| [Asgrow (USA); Seminis Vegetable Inc. (Canada)] | |
| | |
| Nicotiana tabacum L. (Tabak) | Reduzierter Nikotingehalt |
| Linie Vector 21-41 | |
| [Vector Tobacco] | |
| | |
| Lycopersicon esculentum (Tomate) | Verlängerte Haltbarkeit durch reduzierte Ethylen-Akkumulation aufgrund der Expression der ACC Synthase |
| Linie 1345-4 | |
| [DNA Plant Technology] | |
| | |
| Lycopersicon esculentum (Tomate) | Verzögerte Reife durch Expression der S-Adenosylmethionin Hydrolase |
| Linie 35 1 N | |
| [Agritope Inc.] | |
| | |
| Lycopersicon esculentum (Tomate) | Verzögerte Reife durch Expression von ACCd |
| Linie CGN-89322-3 (8338) | |
| [Monsanto Company] | |
| | |
| Lycopersicon esculentum (Tomate) | Verzögertes Weichwerden ("anti-Matsch") durch eine verminderte Expression der |
| Linien B, Da, F | Polygalacturonase |
| [Zeneca Seeds] | |
| | |
| | |
| Lycopersicon esculentum (Tomate) | Verzögertes Weichwerden ("anti-Matsch") durch eine verminderte Expression der Polygalacturonase |
| Linie CGN-89564-2 (FLAVR SAVR) | |
| [Calgene Inc.] | |

### Beispiele:

Die Erfindung wird durch die folgenden Beispiele näher erläutert, ohne sie dadurch einzuschränken.

### Beispiel 1

Einzeln getopfte transgene Baumwollpflanzen mit einer Lepidoptera Resistenz und einer Herbizidresistenz (Linie DP444 BG/RR) werden in 2 Replikationen gegen Larven des Baumwollkapselwurms *(Heliothis armigera)* behandelt. Die Applikation erfolgt durch Sprühapplikation mit dem jeweiligen Wirkstoff in der gewünschten Aufwandmenge.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

Es ist dabei eine deutliche Verbesserung der Bekämpfung der Schädlinge im Vergleich zu den nicht erfindungsgemäß behandelten Kontrollpflanzen zu erkennen.

### Beispiel 2

Töpfe mit je 5 transgenem Maispflanzen mit einer Lepidoptera Resistenz und einer Herbizidresistenz (Linie SGI1890 Hx X SGI1847) werden in 2 Replikationen gegen den Heerwurm (*Spodoptera frugiperda*) behandelt. Die Applikation erfolgt durch Sprühapplikation mit dem jeweiligen Wirkstoff in der gewünschten Aufwandmenge.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

Es ist dabei eine deutliche Verbesserung der Bekämpfung der Schädlinge im Vergleich zu den nicht erfindungsgemäß behandelten Kontrollpflanzen zu erkennen.

### Beispiel 3

Töpfe mit je 5 transgenem Maispflanzen mit einer Herbizid Resistenz (Linie FR1064LL X FR2108) werden in 2 Replikationen gegen den Heerwurm (*Spodoptera frugiperda*) behandelt. Die Applikation erfolgt durch Sprühapplikation mit dem jeweiligen Wirkstoff in der gewünschten Aufwandmenge.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

Es ist dabei eine deutliche Verbesserung der Bekämpfung der Schädlinge im Vergleich zu den nicht erfindungsgemäß behandelten Kontrollpflanzen zu erkennen.

## Patentansprüche

1. Verfahren zur Verbesserung der Nutzung des Produktionspotentials einer transgenen Pflanze, **dadurch gekennzeichnet, dass** die Pflanze mit einer wirksamen Menge mindestens einer optisch aktiven Phthalsäurediamidverbindung behandelt wird.

2. Verfahren nach Anspruch 1, **gekennzeichnet durch** eine optisch aktive Phthalsäurediamidverbindung der Formel (I) 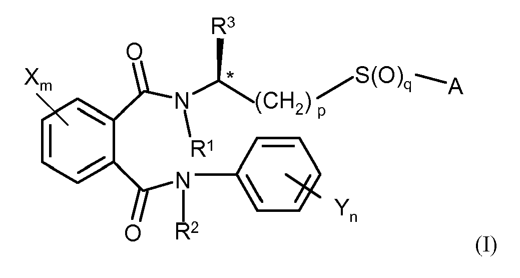 wobei
R¹ und R² unabhängig voneinander für Wasserstoff, (C₁-C₆)-Alkyl, substituiertes (C₁-C₆)- Alkyl, das mit mindestens einem Substituenten, ausgewählt unter Halogen, Cyano, (C₁-C₆)-Alkoxy, (C₁-C₆)-Alkylthio, Mono-(C₁-C₆)-alkylamino und Di-(C₁-C₆)- alkylamino in welcher die Alkylgruppen gleich oder verschieden sind, substituiert ist oder (C₁-C₆)-Alkoxycarbonyl stehen;
R³ für (C₁-C₆)-Alkyl steht;
A für Wasserstoff, (C₁-C₆)-Alkyl, Halo(C₁-C₆)-alkyl, (C₃-C₆)-Alkenyl, Halo(C₃-C₆)- alkenyl, (C₃-C₆)-Alkinyl, Halo(C₃-C₆)-alkinyl, (C₁-C₆)-Alkoxy(C₁-C₆)-alkyl, Halo(C₁-C₆)-alkoxy(C₁-C₆)-alkyl, (C₁-C₆)-Alkylthio(C₁-C₆)-alkyl, Halo(C₁-C₆)- alkylthio(C₁-C₆)-alkyl, (C₁-C₆)-Alkylsulfinyl(C₁-C₆)-alkyl, Halo(C₁- C₆)alkylsulfinyl(C₁-C₆)-alkyl, (C₁-C₆)-Alkylsulfonyl(C₁-C₆)-alkyl, Halo(C₁- C₆)alkylsulfonyl(C₁-C₆)-alkyl, Tri-(C₁-C₆)-alkylsilyl, (C₁-C₆)-Alkylcarbonyl, Mono- (C₁-C₆)-alkylcarbamid, Di-(C₁-C₆)-alkylcarbamid in welcher die Alkylgruppen gleich oder verschieden sind, Phenoxy(C₁-C₆)-alkyl oder substituiertes Phenoxy(C₁- C₆)-alkyl steht, das mit einem oder mehreren, gleichen oder verschiedenen Substituenten substituiert ist, welche ausgewählt sind unter Halogen, (C₁-C₆)-Alkyl, Halo(C₁-C₆)-alkyl, (C₁-C₆)-alkoxy, Halo(C₁-C₆)-alkoxy, (C₁-C₆)-Alkylthio, Halo(C₁-C₆)-alkylthio, (C₁-C₆)-Alkylsulfinyl, Halo(C₁-C₆)-alkylsulfmyl, (C₁-C₆)- Alkylsulfonyl, Halo(C₁-C₆)-alkylsulfonyl, Mono-(C₁-C₆)-alkylamino, Di-(C₁-C₆)- alkylamino, in welcher die Alkylgruppen gleich oder verschieden sind, Mono- (halo(C₁-C₆)-alkyl)amino und Di-(halo(C₁-C₆)-alkyl)amino, in welcher die Alkylgruppen gleich oder verschieden sind, für Phenyl(C₁-C₆)-alkyl oder substituiertes Phenyl(C₁-C₆)-alkyl steht, dessen Phenylring mit einem oder mehreren, gleichen oder verschiedenen Substituenten substituiert ist, welche ausgewählt sind unter Halogen, (C₁-C₆)-Alkyl, Halo(C₁-C₆)-alkyl, (C₁-C₆)-Alkoxy, Halo (C₁-C₆)- alkoxy, (C₁-C₆)-Alkylthio, Halo(C₁-C₆)-alkylthio, (C₁-C₆)-Alkylsulfinyl, Halo(C₁- C₆)-alkylsulfinyl, (C₁-C₆)-Alkylsulfonyl, Halo(C₁-C₆)-alkylsulfonyl, Mono-(C₁-C₆)- alkylamino, Di-(C₁-C₆)-alkylamino in welcher die Alkylgruppen gleich oder verschieden sind, Mono-(halo(C₁-C₆)-alkyl)amino und Di-(halo(C₁-C₆)-alkyl)amino in welcher die Alkylgruppen gleich oder verschieden sind;
p für 0, 1, 2, 3 oder 4 steht;
q für 0, 1 oder 2 steht;
X unabhängig voneinander für Halogen, Cyano, Amino, Nitro, (C₁-C₆)-Alkyl, Halo(C₁-C₆)-alkyl, (C₂-C₆)-Alkenyl, Halo(C₂-C₆)-alkenyl, (C₂-C₆)-Allcinyl, Halo(C₂- C₆)-alkinyl, (C₁-C₆)-Alkoxy, Halo(C₁-C₆)-alkoxy, (C₁-C₆)-Alkylthio, Halo(C₁-C₆)- alkylthio, (C₁-C₆)-Alkylsulfinyl, Halo(C₁-C₆)-alkylsulfmyl, (C₁-C₆)-Alkylsulfonyl, (C₁-C₆)-Alkylsulfonyloxy, Halo(C₁-C₆)-alkylsulfonyl, Mono-(C₁-C₆)-alkylamino oder Di-(C₁-C₆)-alkylamino in welcher die Alkylgruppen gleich oder verschieden sind, steht;
m für 0, 1, 2, 3 oder 4 steht;
Y unabhängig voneinander für Wasserstoff, Halogen, Cyano, Amino, Nitro, (C₁-C₆)- Alkyl, Halo(C₁-C₆)-alkyl, (C₂-C₆)-Alkenyl, Halo(C₂-C₆)-alkenyl, (C₂-C₆)-Alkinyl, Halo(C₂-C₆)-alkinyl, (C₃-C₆)-Cycloalkyl, HalO(C₃-C₆)-cycloalkyl, (C₁-C₆)-Alkoxy, Halo(C₁-C₆)-alkoxy, (C₁-C₆)-Alkylthio, Halo(C₁-C₆)-alkylthio, (C₁-C₆)- Alkylsulfinyl, Halo(C₁-C₆)-alkylsulfinyl, (C₁-C₆)-Alkylsulfonyl, Halo(C₁-C₆)- alkylsulfonyl, Mono-(C₁-C₆)-alkylamino, Di-(C₁-C₆)-alkylamino oder Tri-(C₁-C₆)- alkylsilyl in welchen die Alkylgruppen gleich oder verschieden sind, Phenyl oder substituiertes Phenyl steht, das mit einem oder mehreren, gleichen oder verschiedenen Substituenten, ausgewählt unter Halogen, (C₁-C₆)-Alkyl, Halo(C₁- C₆)-alkyl, (C₁-C₆)-Alkoxy group, Halo(C₁-C₆)-alkoxy, (C₁-C₆)-Alkylthio, Halo(C₁- C₆)-alkylthio, (C₁-C₆)-Alkylsulfinyl, Halo(C₁-C₆)-alkylsulfinyl, (C₁-C₆)- Alkylsulfonyl, Halo(C₁-C₆)-alkylsulfonyl, Mono-(C₁-C₆)-alkylamino, Di-(C₁-C₆)- alkylamino in welcher die Alkylgruppen gleich oder verschieden sind, Mono- (halo(C₁-C₆)-alkyl)amino, und Di(halo (C₁-C₆)-alkyl)amino in welcher die Alkylgruppen gleich oder verschieden sind, substitutiert ist, für heterocyclische Verbindungen oder substituierte heterocyclische Verbindungen steht, die mit einem oder mehreren, gleichen oder verschiedenen Substituenten, welche ausgewählt sind unter Halogen, (C₁-C₆)-Alkyl, Halo(C₁-C₆)-alkyl, (C₁-C₆)-Alkoxy, Halo(C₁-C₆)- alkoxy, (C₁-C₆)-Alkylthio, Halo(C₁-C₆)-alkylthio, (C₁-C₆)-Alkylsulfinyl, Halo(C₁- C₆)-alkylsulfinyl, (C₁-C₆)-Alkylsulfonyl, Halo(C₁-C₆)-alkylsulfonyl, Mono-(C₁-C₆)- alkylamino, Di-(C₁-C₆)-alkylamino in welcher die Alkylgruppen gleich oder verschieden sind, Mono-(halo(C₁-C₆)-alkyl)amino, und Di-(halo(C₁-C₆)-alkyl)amino in welcher die Alkylgruppen gleich oder verschieden sind, substituiert sind; und
n für 1, 2, 3, 4 oder 5 steht.

3. Verfahren nach Anspruch 1 oder 2, **gekennzeichnet durch** eine optisch aktive Phthalsäurediamidverbindung der Formel (I) wobei
R¹ und R² unabhängig voneinander für Wasserstoff oder (C₁-C₆)-Alkyl stehen;
R³ für Methyl oder Ethyl steht;
A für (C₁-C₆)-Alkyl, (C₃-C₆)-Alkenyl, (C₃-C₆)-Alkinyl, (C₁-C₆)-Alkoxy(C₁-C₆)-alkyl, (C₁-C₆)-Alkylthio(C₁-C₆)-alkyl, (C₁-C₆)-Alkylsulfinyl(C₁-C₆)-alkyl, (C₁-C₆)- Alkylsulfonyl(C₁-C₆)-alkyl oder Mono-(C₁-C₆)-alkylcarbamid steht;
p für 0, 1, 2, 3 oder 4 steht;
q für 0, 1 oder 2 steht;
X unabhängig voneinander für Halogen, Nitro, Halo(C₁-C₆)-alkyl, Halo(C₁-C₆)-alkoxy, (C₁-C₆)-Alkylthio, (C₁-C₆)-Alkylsulfinyl, (C₁-C₆)-Alkylsulfonyl, (C₁-C₆)- Alkylsulfonyloxy, steht;
m für 0, 1 oder 2 steht;
Y unabhängig voneinander für Halogen, Cyano, (C₁-C₆)-Alkyl, Halo(C₁-C₆)-alkyl, (C₂- C₆)-Alkenyl, Halo(C₂-C₆)-alkenyl, (C₂-C₆)-Alkinyl, Halo(C₂-C₆)-alkinyl, (C₁-C₆)- Alkoxy, Halo(C₁-C₆)-alkoxy, (C₁-C₆)-Alkylthio, Halo(C₁-C₆)-alkylthio, (C₁-C₆)- Alkylsulfinyl, Halo(C₁-C₆)-alkylsulfinyl, (C₁-C₆)-Alkylsulfonyl, Halo(C₁-C₆)- alkylsulfonyl, Mono-(halo(C₁-C₆)-alkyl)amino oder Di-(halo(C₁-C₆)-alkyl)amino in welcher die Alkylgruppen gleich oder verschieden sind steht; und
n für 0, 1, 2, 3 oder 4 steht.

4. Verfahren nach Anspruch 3, **gekennzeichnet durch** eine optisch aktive Phthalsäurediamidverbindung der Formel (I)
wobei
R¹ und R² unabhängig voneinander für Wasserstoff oder (C₁-C₆)-Alkyl stehen;
R³ für Methyl steht;
A unabhängig voneinander für (C₁-C₆)-Alkyl, (C₃-C₆)-Alkenyl, (C₃-C₆)-Alkinyl, (C₁- C₆)-Alkoxy(C₁-C₆)-alkyl, (C₁-C₆)-Alkylthio(C₁-C₆)-alkyl, (C₁-C₆)-Alkylsulfinyl(C₁- C₆)-alkyl oder (C₁-C₆)-Alkylsulfonyl(C₁-C₆)-alkyl steht;
p für 1 oder 2 steht;
q für 0, 1 oder 2 steht;
X unabhängig voneinander für Halogen, Nitro, Halo(C₁-C₆)-alkyl, Halo(C₁-C₆)- alkoxy, (C₁-C₆)-Alkylthio, (C₁-C₆)-Alkylsulfinyl, (C₁-C₆)-Alkylsulfonyl oder (C₁- C₆)-Alkylsulfonyloxy steht;
m für 1 oder 2 steht;
Y unabhängig voneinander für Halogen, Cyano, (C₁-C₆)-Alkyl, Halo(C₁-C₆)-alkyl, (C₂- C₆)-Alkenyl, Halo(C₂-C₆)-alkenyl, (C₂-C₆)-Alkinyl, Halo(C₂-C₆)-alkinyl, (C₁-C₆)- Alkoxy, Halo(C₁-C₆)-alkoxy, (C₁-C₆)-Alkylthio, Halo(C₁-C₆)-alkylthio, (C₁-C₆)- Alkylsulfinyl, Halo(C₁-C₆)-alkylsulfinyl, (C₁-C₆)-Alkylsulfonyl oder Halo(C₁-C₆)- alkylsulfonyl steht; und
n für 1, 2 oder 3 steht.

5. Verfahren nach einem der Ansprüche 1 bis 4, **dadurch gekennzeichnet, dass** die Pflanze mindestens eine genetisch veränderte Struktur oder eine Toleranz gemäß Tabelle 1 aufweist.

6. Verfahren nach einem der Ansprüche 1 bis 4, **dadurch gekennzeichnet, dass** es sich um eine Pflanze mit mindestens einem geänderten Wirkprinzip gemäß Tabelle 3 handelt.

7. Verfahren nach einem der Ansprüche 1 bis 4, **dadurch gekennzeichnet, dass** es sich um eine transgene Pflanze gemäß der Tabelle 4 handelt.

8. Verfahren nach einem der Ansprüche 1 bis 4, **dadurch gekennzeichnet, dass** die Pflanze mindestens eine genetische Modifikation gemäß der Tabelle 2 enthält.

9. Verfahren nach einem der Ansprüche 1 bis 4, **dadurch gekennzeichnet, dass** die transgene Pflanze mindestens ein Gen oder ein Genfragment codierend ein Bt-Toxin enthält.

10. Verfahren nach Anspruch einem der Ansprüche 1 bis 2, **dadurch gekennzeichnet, daß** es sich bei der transgenen Pflanze um eine Gemüse-, Mais-, Soja-, Baumwoll-, Tabak-, Reis-, Zuckerrüben-, oder Kartoffelpflanze handelt.

11. Verfahren nach einem der Ansprüche 1 bis 10, **dadurch gekennzeichnet, dass** die optisch aktive Phthalsäurediamidverbindung zur Kontrolle von Lepidopteren eingesetzt wird.

12. Verfahren nach einem der obigen Ansprüche, **dadurch gekennzeichnet, dass** Aufwandmengen der optisch aktiven Phthalsäurediamidverbindung zwischen 0,1 g/ha und 5,0 kg/ha verwendet werden.

13. Verfahren nach einem der vorherigen Ansprüche, **dadurch gekennzeichnet, daß** die Anwendungsform der optisch aktiven Phthalsäurediamidverbindung in einer Mischung mit mindestens einem Mischpartner vorliegt.
